(19) Europäisches Patentamt
European Patent Office
Office européen des brevets

(11) **EP 4 141 980 A1**

(12) **EUROPEAN PATENT APPLICATION**

(43) Date of publication:
**01.03.2023 Bulletin 2023/09**

(21) Application number: **21192830.4**

(22) Date of filing: **24.08.2021**

(51) International Patent Classification (IPC):
***H01L 51/54*** *(2006.01)*

(52) Cooperative Patent Classification (CPC):
H01L 51/508; **H01L 51/0052; H01L 51/0058; H01L 51/0067; H01L 51/0072;** H01L 51/5278; H01L 2251/55

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **GARNIER, Jerome**
**01099 Dresden (DE)**
- **PAVICIC, Domagoj**
**01099 Dresden (DE)**
- **WERNER, Ansgar**
**01099 Dresden (DE)**
- **GALÁN GARCÍA, Elena**
**01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

# (54) ORGANIC LIGHT EMITTING DIODE AND A COMPOUND FOR USE THEREIN

(57) The present invention relates to an organic light emitting diode comprising an anode, a cathode, a first emission layer, a second emission layer, a first charge generation layer and a first electron transport layer stack; and to a display device or lighting device comprising the same.

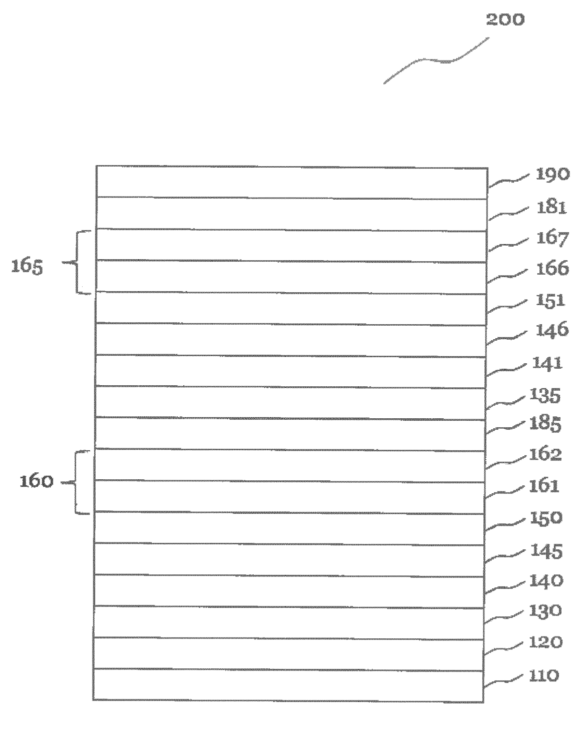


Fig. 1

EP 4 141 980 A1

**Description**

TECHNICAL FIELD

The present invention relates to an organic light emitting diode and to a compound, which may be used in the organic light emitting diode.

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer (HTL), an emission layer (EML), an electron transport layer (ETL), and a cathode, which are sequentially stacked on a substrate. In this regard, the HTL, the EML, and the ETL are thin films formed from organic and / or organometallic compounds.

When a voltage is applied to the anode and the cathode, holes injected from the anode electrode move to the EML, via the HTL, and electrons injected from the cathode electrode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency.

A variety of organic electronic diodes comprising different electron transport materials are well known in the art. However, there is still a need to improve the performance of such devices, in particular to improve the performance of multi-emission-layer OLEDs, specifically with regard to efficiency and voltage.

It is, therefore, the object of the present invention to provide an organic light emitting diode overcoming drawbacks of the prior art, in particular a multi-emission-layer top emission OLED with improved performance, in particular with improved efficiency, more particularly with improved efficiency while maintain good driving voltage and/or stable vacuum processability.

DISCLOSURE

The object is achieved by an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$$(Ar^1\text{-}A_c)_a\text{-}X_b \qquad (I);$$

- a and b are independently 1 or 2;
- c is independently o or 1;
- $Ar^1$ is independently selected from $C_6$ to $C_{60}$ aryl or $C_2$ to $C_{42}$ heteroaryl,
  - wherein each $Ar^1$ may be substituted with one to three substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $FY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^1$ and each $C_3$ to $C_{11}$ heteroaryl substituent on $Ar^1$ may be substituted with $C_1$ to $C_4$ alkyl or halogen;
- A is independently selected from $C_6$ to $C_{30}$ aryl,

- wherein each A may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on A may be substituted with $C_1$ to $C_4$ alkyl or halogen;

- X is independently selected from the group consisting of $C_2$ to $C_{42}$ heteroaryl,

  - wherein each X may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - wherein each $C_6$ to $C_{12}$ aryl substituent on X and each $C_3$ to $C_{11}$ heteroaryl substituent on X may be substituted with $C_1$ to $C_4$ alkyl or halogen;

- the molecular dipole moment of the compound of formula (I) is $\geq 0$ D and $\leq 4$ D;

- the second electron transport layer comprises a compound of Formula (II)

$$(Ar^2)_m\text{-}(Z_k\text{-}G)_n \qquad (II);$$

- m and n are independently 1 or 2;

- k is independently 0, 1 or 2;

- $Ar^2$ is independently selected from the group consisting of $C_2$ to $C_{42}$ heteroaryl and $C_6$ to $C_{60}$ aryl,

  - wherein each $Ar^2$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;

- Z is independently selected from $C_6$ to $C_{36}$ aryl,

  - wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$

alkoxy;

- wherein each $C_6$ to $C_{12}$ aryl substituent on Z may be substituted with $C_1$ to $C_4$ alkyl or halogen;

- G is chosen so that the dipole moment of a compound G-phenyl is ≥ 1 D and ≤ 7 D;
- the first electron transport layer and the second electron transport layer are free of an electrical dopant; and
- the compound of Formula (I) is free of 1,3,5-triazine;

wherein it is provided that the following compounds are excluded from Formula (I)

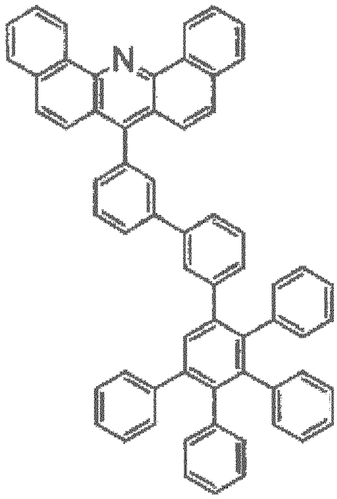

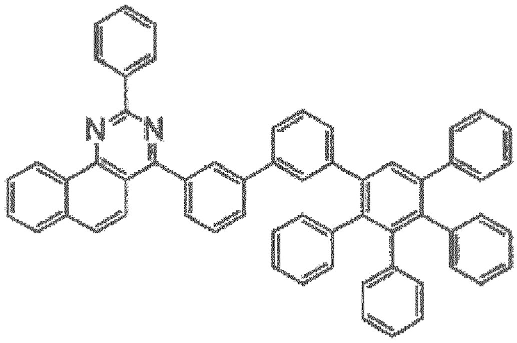

The object is further achieved by a compound of the following formula (IV)

$$(Ar'^1\text{-}A'_d)_e\text{-}X'_f \qquad (IV)$$

wherein

- e and f are independently 1 or 2;
- d is independently 0 or 1;
- Ar'[1] is independently selected from the group consisting of a group having the formula (IVa) and a group having the formula (IVb)

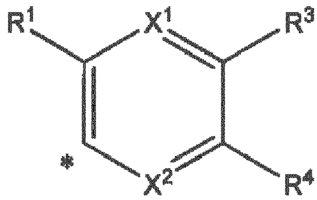

(IVa)

wherein in (IVa)

- the asterisk symbol "*" represents the binding position for binding the group of formula (IVa) to A' or, in case that d = 0 to X';
- $X^1$ and $X^2$ are both N; or $X^1$ is $CR^2$ and $X^2$ is $CR^5$; and

- at least one of $R^1$ and $R^5$ is phenyl; and/or $R^1$ and $R^2$ are both phenyl; and/or $R^2$ and $R^3$ are both phenyl; and/or $R^3$ and $R^4$ are both phenyl; and/or $R^4$ and $R^5$ are both phenyl;

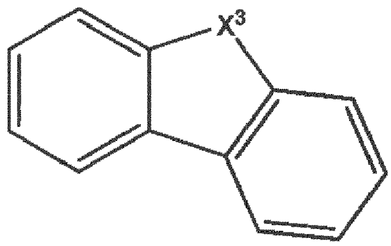

(IVb)

wherein in (IVb)

- the asterisk symbol "*" represents the binding position for binding the group of formula (IVb) to A' or, in case that d = 0 to X'; and

- $X^3$ is selected from $C(CH_3)_2$, O or S;

- wherein each $Ar^1$ may be substituted with one or two phenyl, preferably one phenyl

- A' is independently selected from phenylene or biphenylene;

- X' is independently selected from pyrimidinyl, pyrazinyl, quinazolinyl, benzoquinazolinyl, benzoacridinyl and dibenzoacridinyl;

  wherein each pyrimidinyl, pyrazinyl, quinazolinyl, and benzoquinazolinyl, may be independently be substituted with one or more phenyl, preferably one or two phenyl;

  wherein it is provided that the following compounds are excluded from Formula (IV)

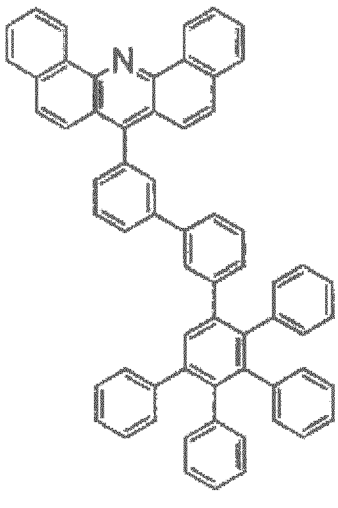

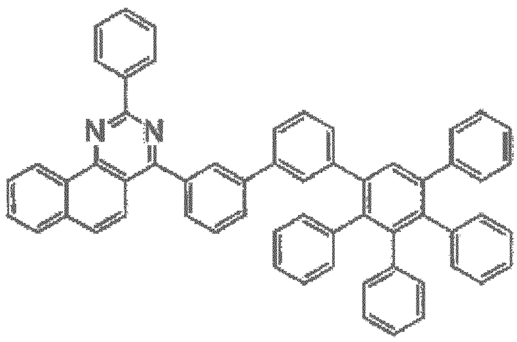

Finally, object is achieved by a device comprising the inventive organic light emitting diode and/or the compound, wherein the device is a display device or a lighting device.

First electron transport layer

The first electron transport layer comprises a compound of Formula (I)

$(Ar^1\text{-}A_c)_a\text{-}X_b$, (I).

It is an essential feature of the present invention that the compound of Formula (I) is free of 1,3,5-triazine. This means that the compound of Formula (I) does not contain (neither at an terminal end of the compound nor as a central portion thereof) a structural moiety having the following structure

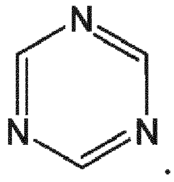

The first electron transport layer may consist of the compound of Formula (I). Alternatively, the first electron transport layer may consist of a mixture of the compound of Formula (I) and one or more further compounds, provided that none of the further compounds is an electrical dopant. The first electron transport layer may comprise more than one compound of Formula (I). In particular, the first electron transport layer may consist of a mixture of the compound of Formula (I) and further compounds known in the art as electron transport matrix compounds. Exemplary further electron transport matrix compounds which may be contained are disclosed below.

In the compound of Formula (I), the group "A" is a spacer moiety connecting (if present, that is in case that c > 1) the group $Ar^1$ and X. In case that the compound of Formula (I) comprises more than one groups ($Ar^1$-$A_c$), the groups may or may not independently comprise the spacer A.

In the compound of Formula (I), a and b are independently 1 or 2. Alternatively, a and b may both be 1.

In the compound of Formula (I), c is independently 0 or 1.

$Ar^1$ is independently selected from $C_6$ to $C_{60}$ aryl or $C_2$ to $C_{42}$ heteroaryl (wherein 1,3,5-triazine is excluded), alternatively $C_6$ to $C_{54}$ aryl or $C_2$ to $C_{39}$ heteroaryl (wherein 1,3,5-triazine is excluded), alternatively $C_6$ to $C_{48}$ aryl or $C_2$ to $C_{36}$ heteroaryl (wherein 1,3,5-triazine is excluded), alternatively $C_6$ to $C_{42}$ aryl or $C_2$ to $C_{36}$ heteroaryl (wherein 1,3,5-triazine is excluded), alternatively $C_6$ to $C_{36}$ aryl or $C_2$ to $C_{30}$ heteroaryl (wherein 1,3,5-triazine is excluded), alternatively $C_6$ to $C_3$. aryl or $C_2$ to $C_{24}$ heteroaryl (wherein 1,3,5-triazine is excluded).

$Ar^1$ may be independently $C_6$ to $C_{54}$ aryl, optionally $C_6$ to $C_{48}$ aryl, optionally $C_6$ to $C_{42}$ aryl, optionally $C_6$ to $C_{36}$ aryl, optionally $C_6$ to $C_{30}$ aryl, and optionally $C_6$ to $C_{24}$ aryl.

$Ar^1$ may be independently $C_2$ to $C_{42}$ hetroaryl (wherein 1,3,5-triazine is excluded), optionally $C_2$ to $C_{40}$ hetroaryl (wherein 1,3,5-triazine is excluded), optionally $C_2$ to $C_{36}$ hetroaryl (wherein 1,3,5-triazine is excluded), optionally $C_2$ to $C_{30}$ hetroaryl (wherein 1,3,5-triazine is excluded), and optionally $C_2$ to $C_{24}$ hetroaryl (wherein 1,3,5-triazine is excluded).

In an embodiment, $Ar^1$ is different from X.

$Ar^1$ may comprise a system of two or more anellated aromatic rings, preferably three or more anellated aromatic rings.

$Ar^1$ may comprise at least one $sp^3$-hypridized carbon atom.

$Ar^1$ may comprise at least one carbon-carbon $sp^2$ alkene bond which is not integrated into an aromatic ring structure.

In an embodiment where $Ar^1$ is independently selected from unsubstituted $C_2$ to $C_{42}$ heteroaryl, the heteroatoms may be bound into the molecular structure of $Ar^1$ by single bonds.

$Ar^1$ may be independently selected from the group consisting of phenyl, naphthyl, anthracenyl, fluoranthenyl, xanthenyl, dibenzofuranyl, dibenzothiophene-yl pyrimidinyl, pyrazinyl, spiroxanthenyl, fluorenyl, spiro-fluorenyl, triphenylsilyl, tetraphenylsilyl, a group having the formula (IIa) and a group having the formula (IIb)

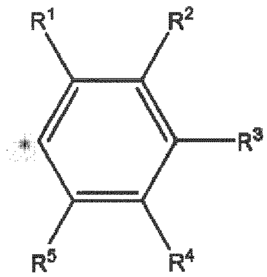

(IIa)

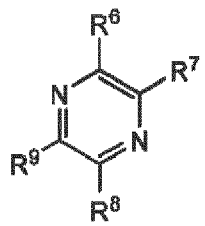

(IIb)

wherein

- the asterisk symbol "*" represents the binding position for binding the group of formula (IIa) to A; and

- $R^1$ to $R^9$ are independently selected from the group consisting of H, $C_6$ to $C_{12}$ aryl and $C_4$ to $C_{10}$ heteroraryl.

In the group of Formula (IIa), both $R^6$ and $R^7$; and/or both $R^8$ and $R^9$ may be phenyl.

If $Ar^1$ is a group having the formula (IIb), binding of the group having the formula (IIb) to A (or if A is not present in case that c =0) to X may be via any of the groups $R^6$ to $R^9$ wherein (formally) A (respectively X) replaces a terminal hydrogen atom of the respective $R^6$ to $R^9$.

$Ar^1$ may be independently selected from the group consisting of fluoranthenyl, dibenzofuranyl, pyrimidinyl pyrazinyl, , 9,9-dimethylfluorenyl, a group having the formula (IIa), a group having the formula (IIb),

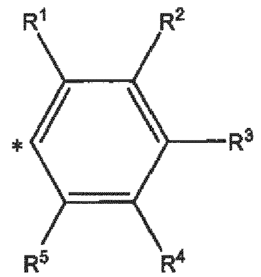
(IIa)

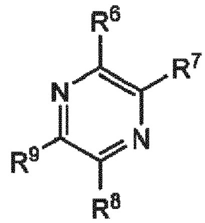
(IIb)

wherein

- the asterisk symbol "*" represents the binding position for binding the group of formula (IIa) to A; and

- $R^1$ is H and $R^2$ to $R^5$ are independently phenyl; or

- $R^1$ and $R^3$ are phenyl and $R^2$, $R^4$ and $R^5$ are H or

- $R^6$ to $R^9$ are phenyl.

If $Ar^1$ is a group having the formula (IIb), binding of the group having the formula (IIb) to A (or if A is not present in case that c =0) to X may be via any of the groups $R^6$ to $R^9$ wherein (formally) A (respectively X) replaces a terminal hydrogen atom of the respective $R^6$ to $R^9$.

In the group of Formula (IIa), at least two of $R^1$ to $R^5$, which are not H may be in ortho-position to each other. At least one of $R^1$ to $R^5$ which is not H may be in ortho-position to the *-position. In this regard, two groups are in ortho-position to each other if bound to adjacent carbon atoms of the benzene ring in Formula (IIa), respectively. In other words, it can be provided that at least one of $R^1$ and $R^5$ is phenyl; and/or $R^1$ and $R^2$ are both phenyl; and/or $R^2$ and $R^3$ are both phenyl; and/or $R^3$ and $R^4$ are both phenyl; and/or $R^4$ and $R^5$ are both phenyl.

$Ar^1$ may be selected independently from one of the following groups

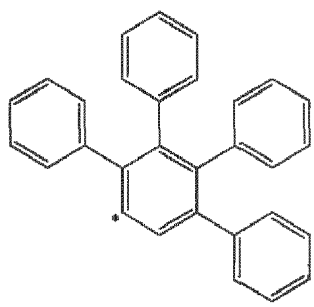
;
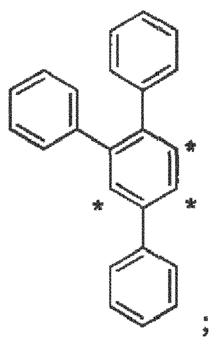
;
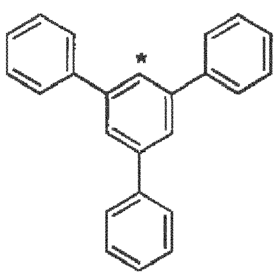
;
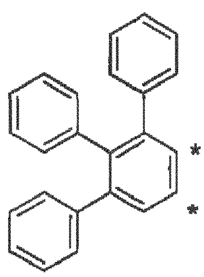
;
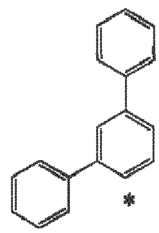

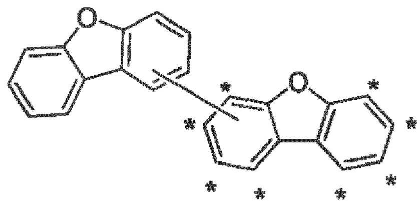 ; 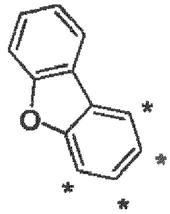 ; 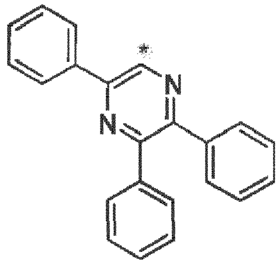 ; 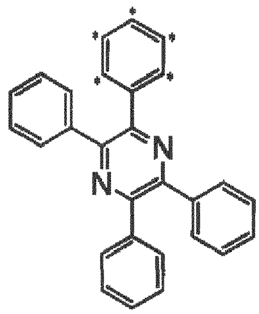

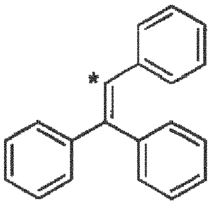 ; 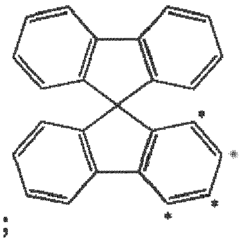 ; 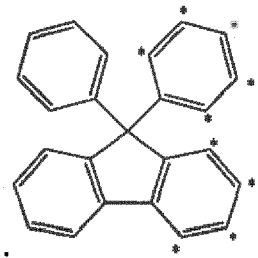 ; 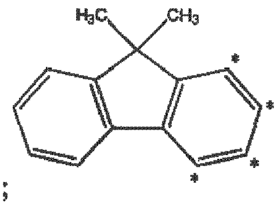

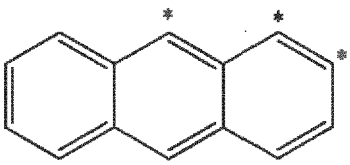 ; 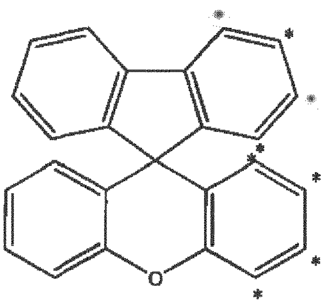 ; 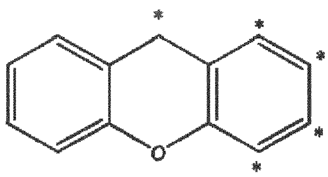 ;

wherein the asterisk symbol "*" represents the binding position for binding the to A, respectively and which may be substituted or unsubstituted, respectively.

In case that $Ar^1$ is substituted, each of the substituents may be independently selected from the group consisting of phenyl, naphtyl, biphenyl, pyridyl, picolinyl, dibenzofuranyl, dibenzothiophene-yl, and benzothiophene-yl.

A may be selected independently from the group consisting of phenylene, naphthylene, biphenylene and terphenylene which may be substituted or unsubstituted, respectively.

A may be selected independently from one of the following groups or combinations thereof

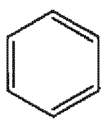 ; 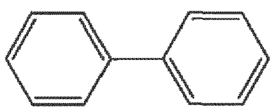 ; 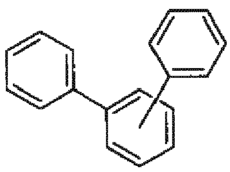 ; ; 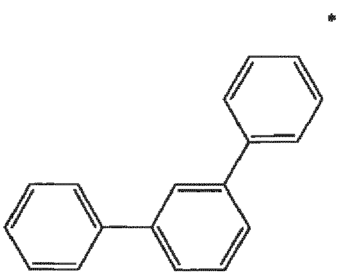 ;

wherein the binding positions for binding to $Ar^1$ and X can be freely selected, preferably

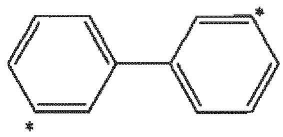 ; 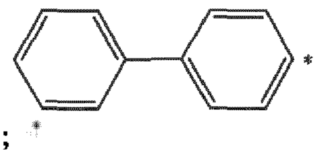 ; 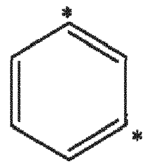 ; 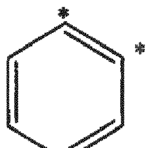 ; 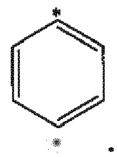 ;

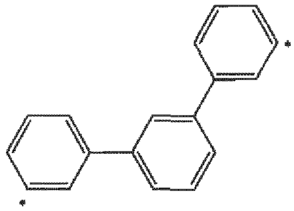 ; 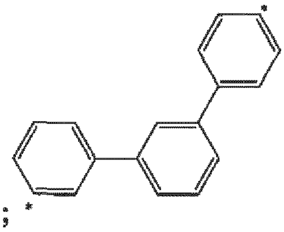 ;

In case that A is substituted, each substituent on A may be independently selected from the group consisting of phenyl and $C_1$ to $C_4$ alkyl.

X may be independently selected from the group consisting of $C_2$ to $C_{39}$ heteroaryl, optionally $C_2$ to $C_{36}$ heteroaryl, optionally $C_3$ to $C_{30}$ heteroaryl, optionally $C_3$ to $C_{27}$ heteroaryl, optionally $C_3$ to $C_{24}$ heteroaryl, and optionally $C_3$ to $C_{21}$ heteroaryl, wherein the respective group may be substituted or unsubstituted, wherein 1,3,5-triazine is excluded, respectively.

X may be independently selected from the group consisting of $C_2$ to $C_{39}$ N-containing heteroaryl and $C_2$ to $C_{39}$ O-containing heteroaryl, optionally $C_2$ to $C_{36}$ N-containing heteroaryl and $C_2$ to $C_{36}$ O-containing heteroaryl, optionally $C_3$ to $C_{30}$ N-containing heteroaryl and $C_3$ to $C_{36}$ O-containing heteroaryl, optionally $C_3$ to $C_{27}$ N-containing heteroaryl and $C_3$ to $C_{27}$ O-containing heteroaryl, optionally $C_3$ to $C_{24}$ N-containing heteroaryl and $C_3$ to $C_{24}$ O-containing heteroaryl, and optionally $C_3$ to $C_{21}$ N-containing heteroaryl and $C_3$ to $C_{21}$ O-containing heteroaryl, wherein 1,3,5-triazine is excluded, respectively.

X may be independently selected from the group consisting of $C_2$ to $C_{39}$ N-containing heteroaryl, optionally $C_2$ to $C_{36}$ N-containing heteroaryl, optionally $C_3$ to $C_{30}$ N-containing heteroaryl, optionally $C_3$ to $C_{27}$ N-containing heteroaryl, optionally $C_3$ to $C_{24}$ N-containing heteroaryl, and optionally $C_3$ to $C_{21}$ N-containing heteroaryl, , wherein 1,3,5-triazine is excluded, respectively. In this regard, it may be provided that a respective N-containing heteroaryl comprises one or more N-atoms as the only heteroatom(s).

X may be independently selected from the group consisting of pyridazinyl, pyrimidinyl, pyrazinyl, quinazolinyl, benzoquinazolinyl, benzimidazolyl, quinolinyl, benzoacridinyl, dibenzoacridinyl, phenathrolinyl, and dinaphthofuranyl, which may be substituted or unsubstituted, respectively.

X may be independently selected from the group consisting of pyrimidinyl, pyrazinyl, quinazolinyl, benzoquinazolinyl, dibenzoacridinyl, which may be substituted or unsubstituted, respectively.

X may be selected independently from one of the following groups

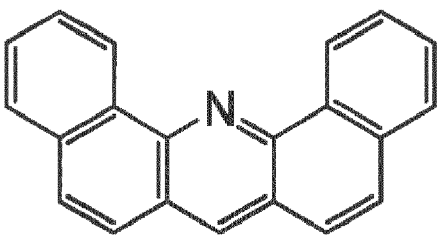 ; 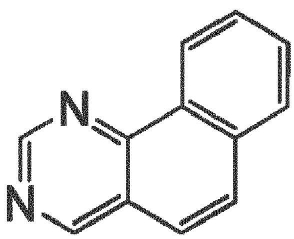 ; 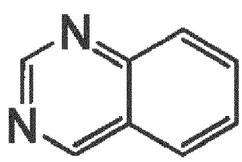

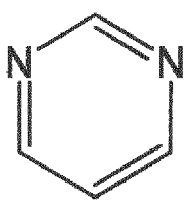 ; 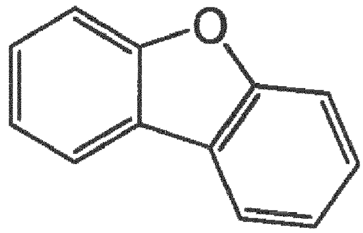 ; 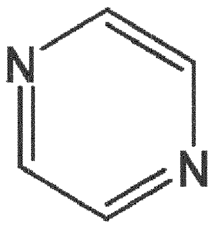

which may be substituted or unsubstituted, respectively.

X may be selected independently from one of the following groups

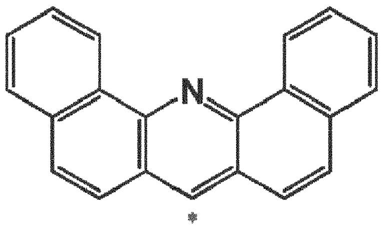 ; 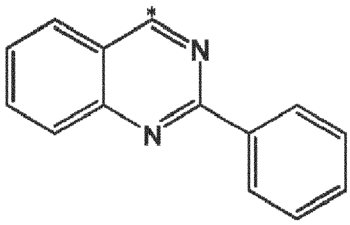 ; 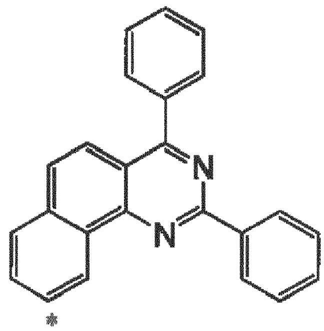 ;

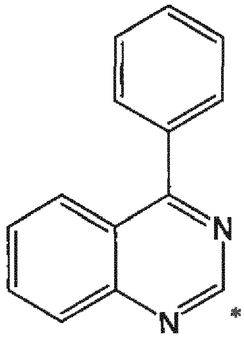 ; 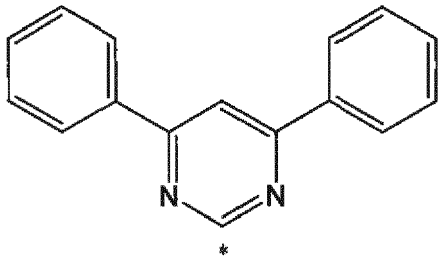 ; 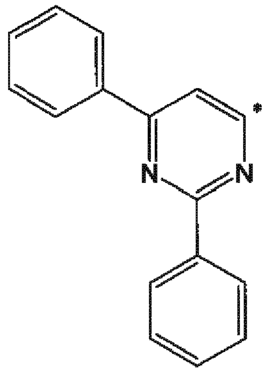 ;

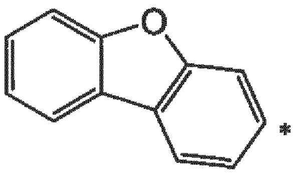 ; 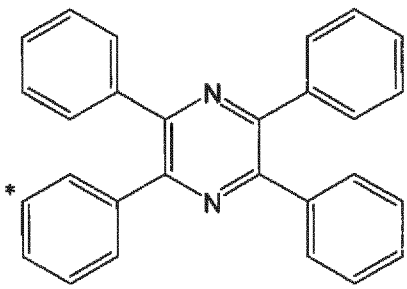 ; 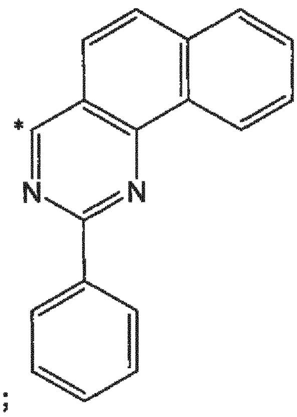 ; 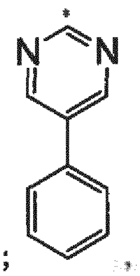

wherein the asterisk symbol "*" represents the binding position for binding the to A, respectively in case that c = 0 to $Ar^1$.

It may be provided that the compound of Formula (I) does not contain a moiety P=O. It may be provided that the compound of Formula (I) does not contain $P(=O)Aryl_2$. It may be provided that the compound of Formula (I) does not contain $P(=O)Alkyl_2$. It may be provided that the compound of Formula (I) does not contain $P(=O)Ph_2$. It may be provided that the compound of Formula (I) does not contain $P(=O)(CH_3)_2$. It may be provided that the compound of Formula (I) does not contain R'P(=O)R" wherein R' and R" are connected with each other to form a ring, that is, does not contain ring-phosphine oxide. It may be provided that the compound of Formula (I) does not contain R'P(=O)R" wherein R' and R" are connected with each other to form a 7-membered ring.

It may be provided that the compound of Formula (I) does not contain two moieties P=O. It may be provided that wherein the compound of Formula (I) does not contain two $P(=O)Aryl_2$. It may be provided that wherein the compound of Formula (I) does not contain two $P(=O)Alkyl_2$. It may be provided that wherein the compound of Formula (I) does not

contain two $P(=O)Ph_2$. It may be provided that wherein the compound of Formula (I) does not contain two $P(=O)(CH_3)_2$. It may be provided that wherein the compound of Formula (I) does not contain CN.

It may be provided that one or more of the following formulas are excluded from the scope of the Compound of Formula (I)

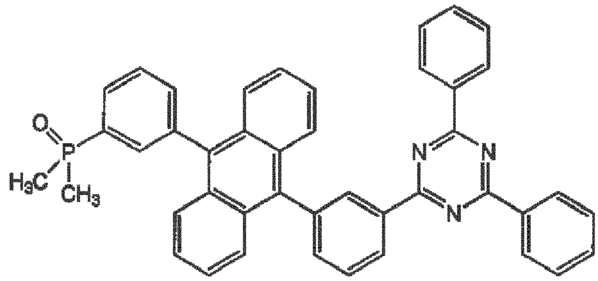
;
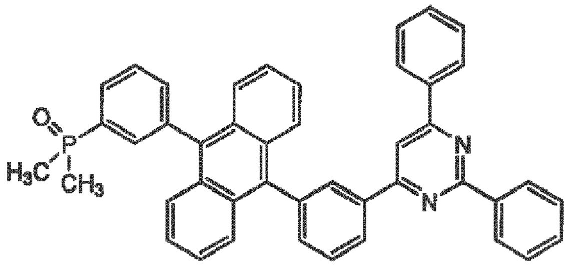
;
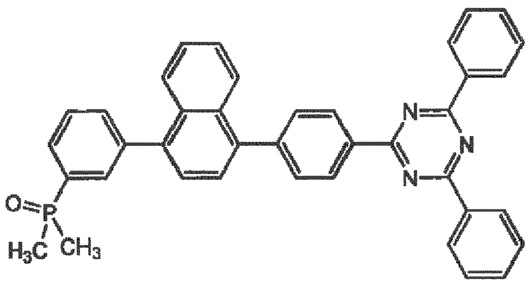
;
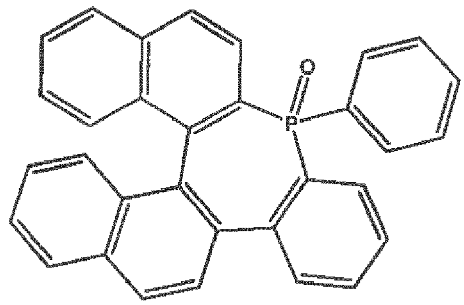
;
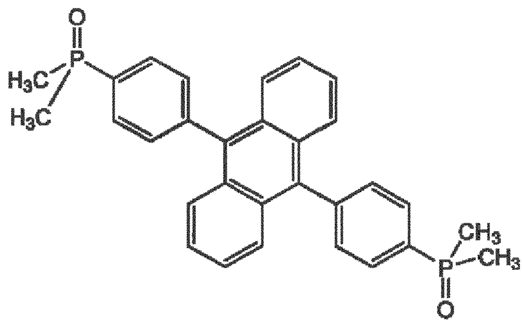
;
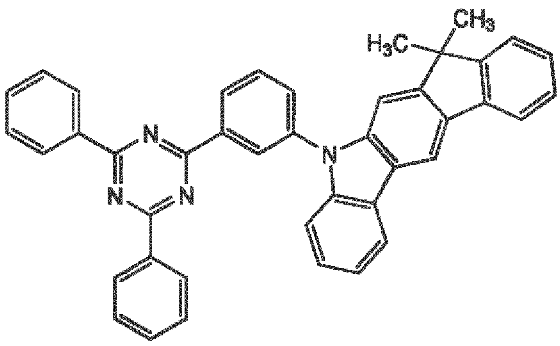
;
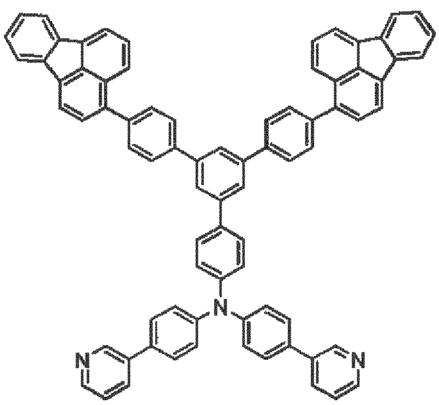
;
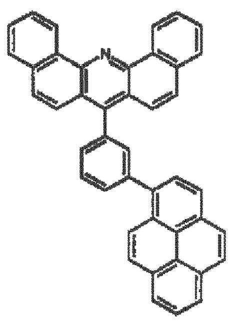

The compound of Formula (I) may comprise 6 to 14 aromatic or heteroaromatic rings, optionally 7 to 13 aromatic or heteroaromatic rings, optionally 8 to 12 aromatic or heteroaromatic rings. In this regard, an aromatic, respectively heteroaromatic ring, is a single aromatic ring, for example a 6-membered aromatic ring such as phenyl, a 6-membered

heteroaromatic ring, an example would be pyridyl, a 5-membered heteroaromatic ring an example would be pyrrolyl etc. In a system of condensed (hetero)aromatic rings, each ring is considered as a single ring in this regard. For example, naphthalene comprises two aromatic rings.

The compound of Formula (I) may have a LUMO of ≥ -1.81 eV in the absolute scale taking vacuum energy level as zero, computed by the TURBOMOLE V6.5 program package using hybrid functional B3LYP and Gaussian 6-31G* basis set.

The compound of Formula (I) may have a LUMO of ≥ -1.81 eV and ≤ -1.47 eV in the absolute scale taking vacuum energy level as zero, computed by the TURBOMOLE V6.5 program package using hybrid functional B3LYP and Gaussian 6-31G* basis set

The molecular dipole moment, computed by the TURBOMOLE V6.5 program package using hybrid functional B3LYP and Gaussian 6-31G* basis set, of the compound of formula (I) may be ≥ 0 D and ≤ 4 D; alternatively ≥ 0.2 D and ≤ 3.5 D; alternatively ≥ 0.4 D and ≤ 3.2 D; alternatively ≥ 0.8 D and ≤ 3.1 D. In this regards, the dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

The compound of Formula (I) may be selected from the compounds A-1 to A-12

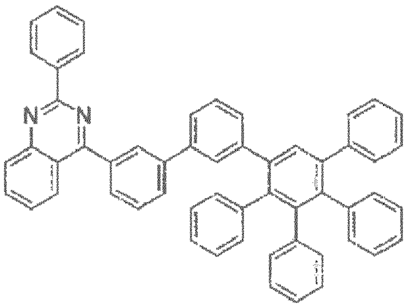

**A-1**

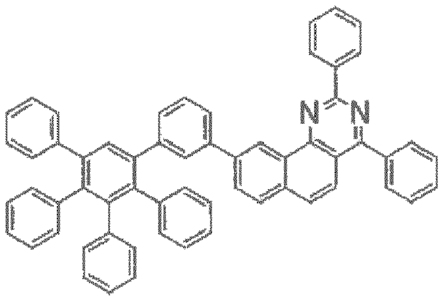

**A-2**

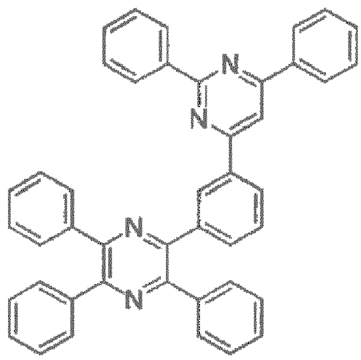

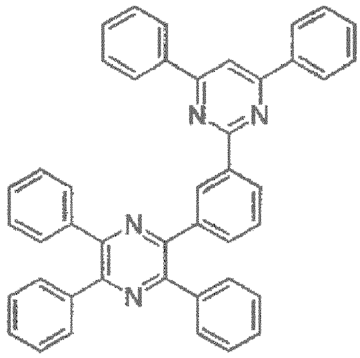

A-3

A-4

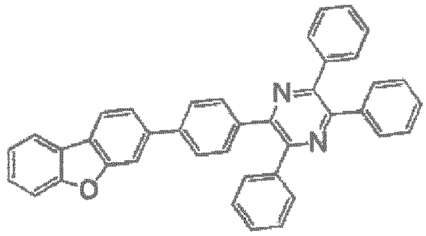

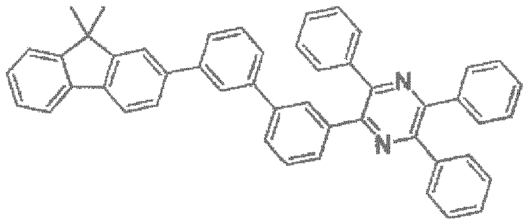

A-5

A-6

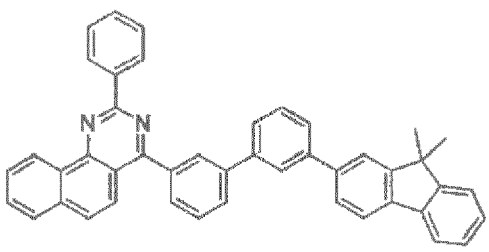

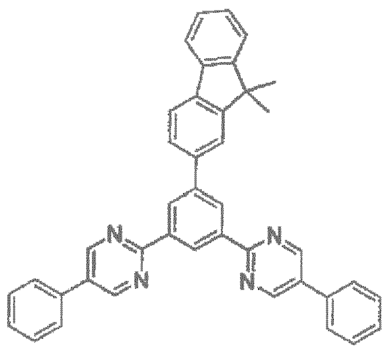

A-7

A-8

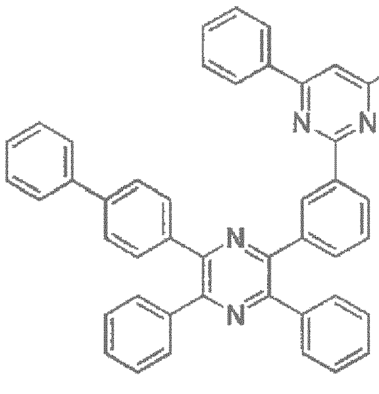

A-9

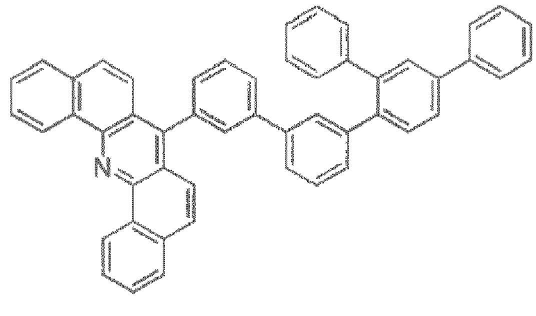

A-10

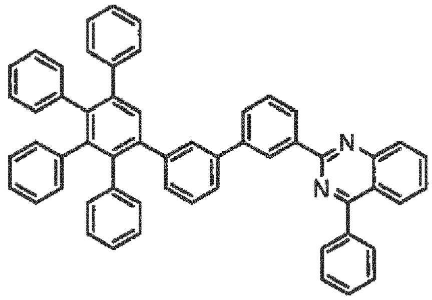

A-11

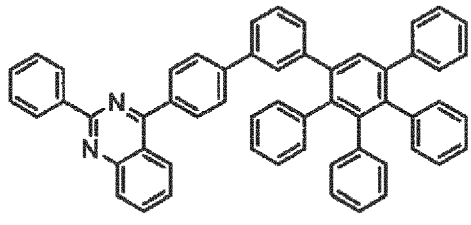

A-12

The compound of Formula (I) may be selected from A-1 to A-4, A-7, A-9, A-11 or A-12.

The first electron transport layer may be arranged between the emission layer and the second electron transport layer. The first electron transport layer may be arranged in direct contact with the emission layer. The first electron transport layer may be arranged "contacting sandwiched" between the emission layer and the second electron transport layer.

The first electron transport layer may have a thickness of < 50 nm, optionally between 1 and 30 nm, optionally between 1 and 10 nm, optionally between 1 and 5 nm.

The second electron transport layer comprises a compound of Formula (II)

$$(Ar^2)_m\text{-}(Z_k\text{-}G)_n \qquad \text{(II)}.$$

The second electron transport layer may consist of the compound of Formula (II). Alternatively, the second electron transport layer may consist of a mixture of the compound of Formula (II) and one or more further compounds, provided that none of the further compounds is an electrical dopant. The first electron transport layer may comprise more than one compound of Formula (II). The second electron transport layer may consist of a mixture of the compound of Formula (II) and further compounds known in the art as electron transport matrix compounds. Exemplary further electron transport matrix compounds which may be contained are disclosed below.

In the compound of Formula (II), the group "Z" is a spacer moiety connecting (if present, that is in case that k > 1) the groups $Ar^2$ and G. In case that the compound of Formula (II) comprises more than one groups ($Z_k$-G) the groups may or may not independently comprise the spacer Z.

In Formula (II), m and n are independently 1 or 2. In Formula (II), m and n may be 1.

In Formula (II), k is independently 0, 1 or 2. In Formula (II), k may be independently 1 or 2.

$Ar^2$ may be independently selected from the group consisting of $C_2$ to $C_{39}$ heteroaryl and $C_6$ to $C_{54}$ aryl, optionally $C_2$ to $C_{36}$ heteroaryl and $C_6$ to $C_{48}$ aryl, optionally $C_3$ to $C_{30}$ heteroaryl and $C_6$ to $C_{42}$ aryl, optionally $C_3$ to $C_{27}$ heteroaryl and $C_6$ to $C_{36}$ aryl, optionally $C_3$ to $C_{24}$ heteroaryl and $C_6$ to $C_{30}$ aryl, and optionally $C_3$ to $C_{21}$ heteroaryl and $C_6$ to $C_{24}$ aryl.

$Ar^2$ may be independently selected from the group consisting of $C_2$ to $C_{39}$ N-containing heteroaryl and $C_6$ to $C_{54}$ aryl, optionally $C_2$ to $C_{36}$ N-containing heteroaryl and $C_6$ to $C_{48}$ aryl, optionally $C_3$ to $C_{36}$ N-containing heteroaryl

and $C_6$ to $C_{42}$ aryl, optionally $C_3$ to $C_{27}$ N-containing heteroaryl and $C_6$ to $C_{36}$ aryl, optionally $C_3$ to $C_{24}$ N-containing heteroaryl and $C_6$ to $C_{30}$ aryl, and optionally $C_3$ to $C_{21}$ N-containing heteroaryl and $C_6$ to $C_{24}$ aryl. In this regard, it may be provided that a respective N-containing heteroaryl comprises one or more N-atoms as the only heteroatom(s).

$Ar^2$ may comprise at least two annelated 5- or 6-membered rings.

$Ar^2$ may be independently selected from the group consisting of pyridinyl, triazinyl, 1,2-diazinyl, 1,3-diazinyl, 1,4-diazinyl, quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl which may be substituted or unsubstituted, respectively.

$Ar^3$ may be independently selected from the group consisting of dibenzoacridinyl, 1,3-diazinyl, 1,4-diazinyl, anthracenyl, triazinyl, phenathrolinyl, triphenylenyl, pyridinyl, dinaphthofuranyl.

$Ar^2$ may be selected independently from one of the following groups

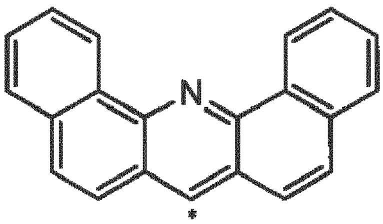 ; 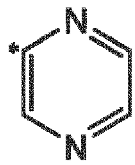 ;

; 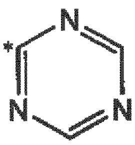 ; 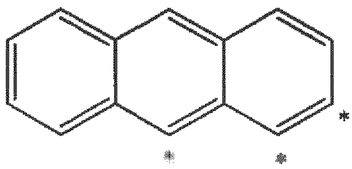 ; 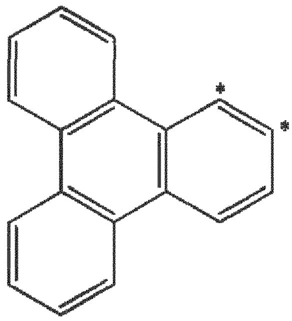 ;

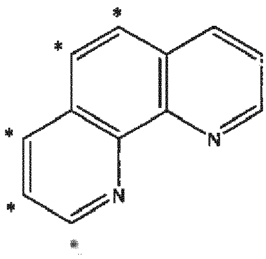 ; 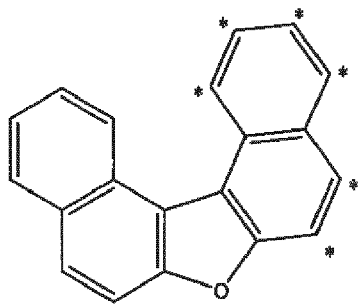 ; 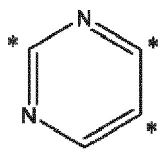

wherein the asterisk symbol "*" represents the binding position for binding the to Z, respectively.

In case that $Ar^2$ is substituted, each substituent on $Ar^2$ may be independently selected from the group consisting of phenyl, naphthyl, optionally β-naphthyl, pyridinyl and biphenyl-yl which may be substituted or unsubstituted, respectively.

In case that $Ar^2$ is substituted, each substituent on $Ar^2$ may be independently selected from the group consisting of phenyl, pyridinyl and biphenyl-yl, optionally para-biphenyl-yl.

Z may be independently selected from $C_6$ to $C_{24}$ aryl, alternatively $C_6$ to $C_{18}$ aryl, alternatively $C_6$ to $C_{12}$ aryl, which may be substituted or unsubstituted.

Z may be selected from the group consisting of phenylene, naphthylene, phenylene-naphthylene, biphenylene and terphenylene which may be substituted or unsubstituted, respectively.

Z may be selected independently from one of the following groups

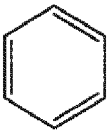 ; 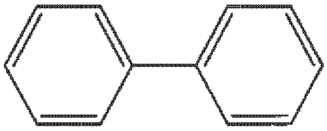 ; 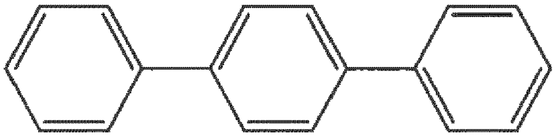 ;

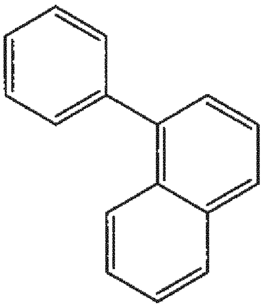 ; 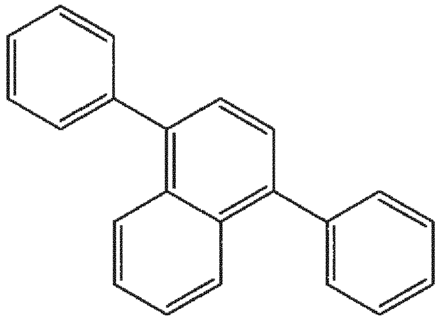

wherein the binding positions for binding to $Ar^2$ and G can be freely selected.

In case that Z is substituted, each substituent on Z may be independently selected from the group consisting of phenyl and $C_1$ to $C_4$ alkyl.

G is chosen so that the dipole moment, computed by the TURBOMOLE V6.5 program package using hybrid functional B3LYP and Gaussian 6-31G* basis set, of a compound G-phenyl is ≥ 1 D and ≤ 7 D. The unit for the dipole moment "Debye" is abbreviated with the symbol "D". The inventors have found that it is advantageous if the compound of Formula (II) comprises a group having a certain polarity, that is a specific dipole moment within the above range or the ranges mentioned below. It was further found that it is still advantageous that the compound of Formula (II) comprises such a polar group (first polar group) if the compound of Formula (II) comprises, in addition, a further polar group (second polar group) which is suitable to balance the dipole moment of the first polar group in a way that the total dipole moment of the compound of Formula (II) is low, for example, in case that the compound is a symmetrical molecule comprising a first polar group and a second polar group which are the same, the dipole moment could be 0 Debye. Therefore, the compound of Formula (II) cannot be characterized be referring to the total dipole moment of the compound. As a consequence, reference is made instead to an artificial compound comprising the polar group "G" and an unpolar group "phenyl". In this regards, the dipole moment $|\vec{\mu}|$ of a compound containing N atoms is given by:

$$\vec{\mu} = \sum_{i}^{N} q_i \vec{r}_i$$

$$|\vec{\mu}| = \sqrt{\mu_x^2 + \mu_y^2 + \mu_z^2}$$

where $q_t$ and $\vec{r}_l$ are the partial charge and position of atom i in the molecule. The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules. In this regard, the entire moiety G encompasses all possible substituents which may be comprised.

G may be selected so that the dipole moment of a compound G-phenyl is > 1 D; optionally ≥ 2 D; optionally ≥ 2.5 D, optionally ≥ 2.5 D, optionally ≥ 3 D, and optionally ≥ 3.5 D. G may be chosen so that the dipole moment of a compound G-phenyl is ≤ 7 D, optionally ≤ 6.5 D, optionally ≤ 6 D, optionally ≤ 5.5 D, optionally ≤ 5 D. If more than one conformational isomer of the compound G-phenyl is viable then the average value of the dipole moments of the conformational isomers of G-phenyl is selected to be in this range. Conformational isomerism is a form of stereoisomerism in which the isomers can be interconverted just by rotations about formally single bonds.

By selecting the G such that the dipole moment of a compound G-phenyl lies in the above range it is provided that the electron injection from the adjacent, distinct charge generation layer (CGL) is improved and voltage of the OLED

device is decreased and the cd/A efficiency of the OLED device is increased.

Exemplary compounds "G-phenyl" are listed in the following Table 1, wherein the moiety

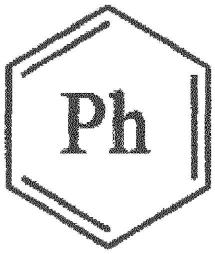

in the respective compound specifies the "phenyl" part in "G-phenyl"

Table 1:

| | Structure of G-phenyl | HOMO [eV] | LUMO [eV] | Dipole moment [D] |
|---|---|---|---|---|
| G-phenyl-1 | 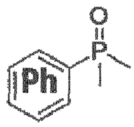 | -6.88 | -0.62 | **4.16** |
| G-phenyl-2 | 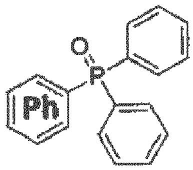 | -6.74 | -0.86 | **4.19** |
| G-phenyl-3 | 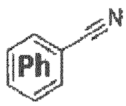 | -8.97 | 1.00 | **4.56** |
| G-phenyl-4 | 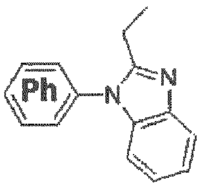 | -5.82 | -0.62 | **3.97** |
| G-phenyl-5 | 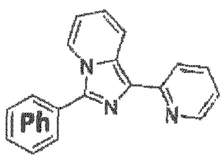 | -5.04 | -1.18 | **3.86** |
| G-phenyl-6 | 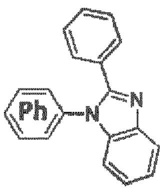 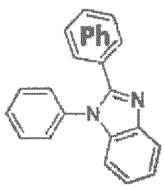 | -5.70 | -1.02 | **3.70** |

(continued)

| | Structure of G-phenyl | HOMO [eV] | LUMO [eV] | Dipole moment [D] |
|---|---|---|---|---|
| G-phenyl-7 | 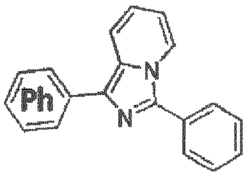 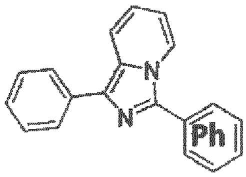 | -4.92 | -1.11 | **3.11** |
| G-phenyl-8 | 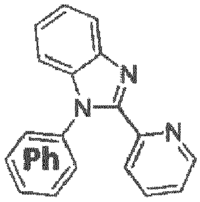 | -5.86 | -1.19 | **5.14** |
| G-phenyl-9 | 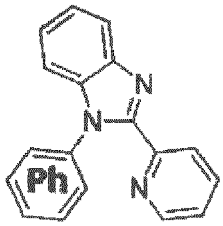 | -5.76 | -1.33 | **2.61** |
| G-phenyl-10 | 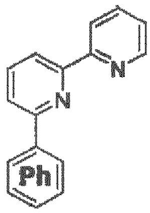 | -5.96 | -1.35 | **2.69** |
| G-phenyl-11 | 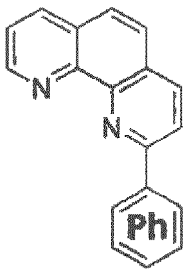 | -5.83 | -1.59 | **2.67** |
| G-phenyl-12 | 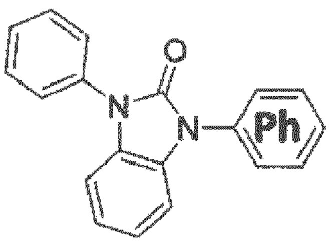 | -5.54 | -0.48 | **2.12** |

(continued)

| | Structure of G-phenyl | HOMO [eV] | LUMO [eV] | Dipole moment [D] |
|---|---|---|---|---|
| G-phenyl-13 | 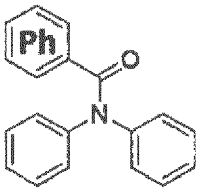 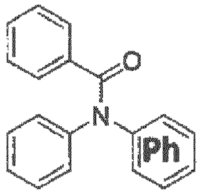 | -5.79 | -1.06 | **3.33** |
| G-phenyl-14 | 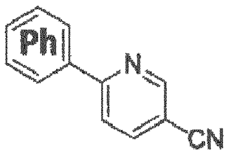 | -6.59 | -2.08 | **4.79** |
| G-phenyl-15 | 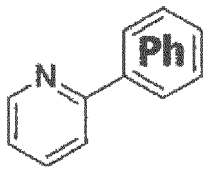 | -6.12 | -1.13 | **1.71** |
| G-phenyl-16 | 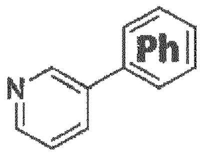 | -6.32 | -0.98 | **2.31** |
| G-phenyl-17 | 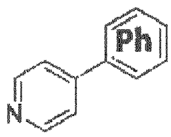 | -6.57 | -1.19 | **2.75** |
| G-phenyl-18 | 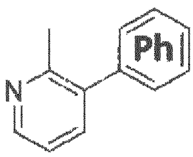 | -6.28 | -0.77 | **2.00** |
| G-phenyl-19 | 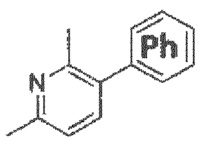 | -6.12 | -0.69 | **1.50** |
| G-phenyl-20 | 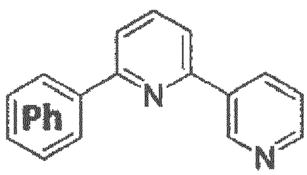 | -6.10 | -1.41 | **3.51** |

(continued)

| | Structure of G-phenyl | HOMO [eV] | LUMO [eV] | Dipole moment [D] |
|---|---|---|---|---|
| G-phenyl-21 | 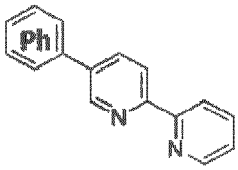 | -6.10 | -1.38 | **2.98** |
| G-phenyl-22 | 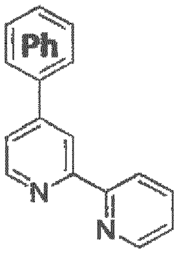 | -6.47 | -1.31 | **3.46** |
| G-phenyl-23 | 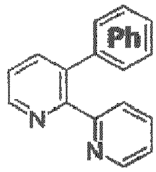 | -6.19 | -1.03 | **3.02** |
| G-phenyl-24 | 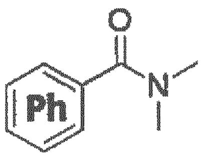 | -6.35 | -0.17 | **3.62** |
| G-phenyl-25 | 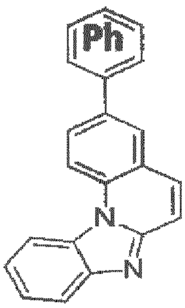 | -5.54 | -1.58 | **3.49** |
| G-phenyl-26 | 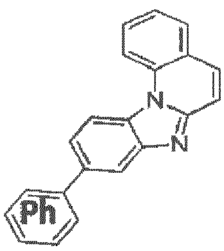 | -5.60 | -1.61 | **3.39** |

(continued)

| | Structure of G-phenyl | HOMO [eV] | LUMO [eV] | Dipole moment [D] |
|---|---|---|---|---|
| G-phenyl-27 | 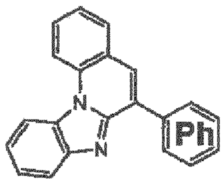 | -5.48 | -1.67 | **2.76** |
| G-phenyl-28 | 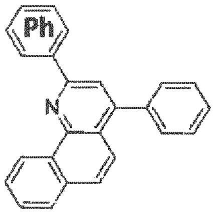 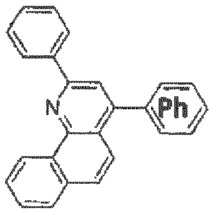 | -5.63 | -1.56 | **1.84** |
| G-phenyl-29 | 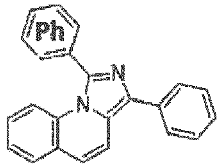 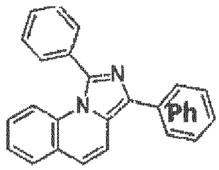 | -5.02 | -1.39 | **2.96** |
| G-phenyl-30 | 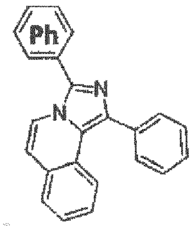 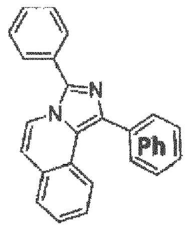 | -5.08 | -1.13 | **2.70** |
| G-phenyl-31 | 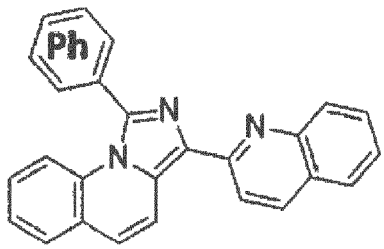 | -5.07 | -1.58 | **2.29** |
| G-phenyl-32 | 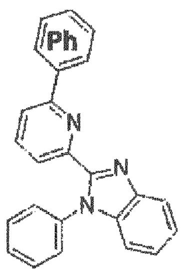 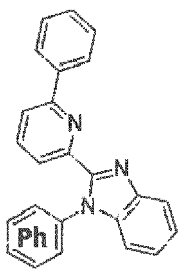 | -5.81 | -1.19 | **4.61** |

(continued)

| | Structure of G-phenyl | HOMO [eV] | LUMO [eV] | Dipole moment [D] |
|---|---|---|---|---|
| G-phenyl-33 | 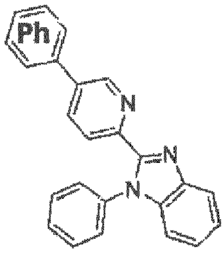 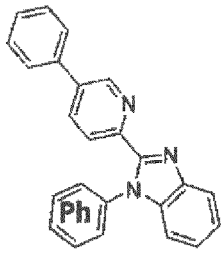 | -5.78 | -1.42 | **5.20** |
| G-phenyl-34 | 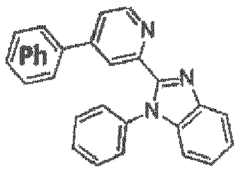 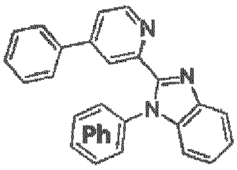 | -5.84 | -1.38 | **5.63** |
| G-phenyl-35 | 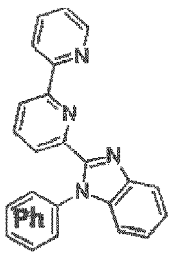 | -5.83 | -1.35 | **3.37** |
| G-phenyl-36 | 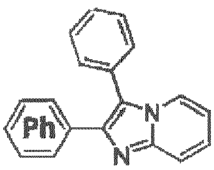 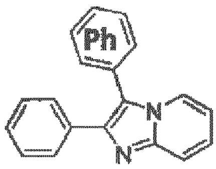 | -5.37 | -0.98 | **3.32** |
| G-phenyl-37 | 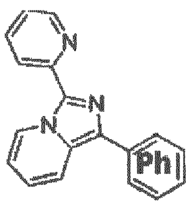 | -4.94 | -1.15 | **1.81** |
| G-phenyl-38 | 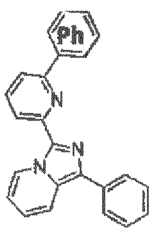 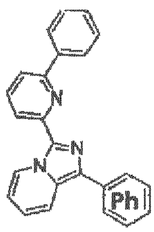 | -4.94 | -1.16 | **2.12** |

(continued)

| | Structure of G-phenyl | HOMO [eV] | LUMO [eV] | Dipole moment [D] |
|---|---|---|---|---|
| G-phenyl-39 | 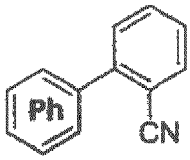 | -6.52 | -1.47 | **4.17** |
| G-phenyl-40 | 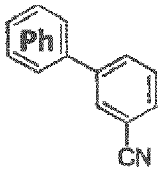 | -6.56 | -1.46 | **4.85** |
| G-phenyl-41 | 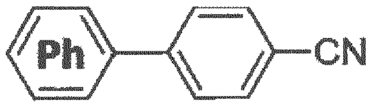 | -6.53 | -1.67 | **5.27** |
| G-phenyl-42 | 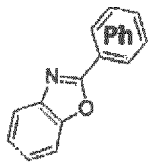 | -6.00 | -1.43 | **1.14** |
| G-phenyl-43 | 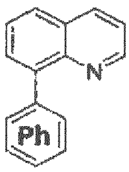 | -5.84 | -1.47 | **1.94** |
| G-phenyl-44 | 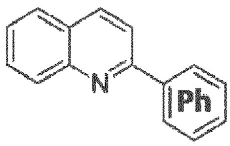 | -5.97 | -1.56 | **1-53** |
| G-phenyl-45 | 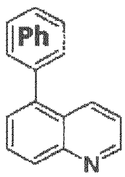 | -6.01 | -1.42 | **2.31** |
| G-phenyl-46 | 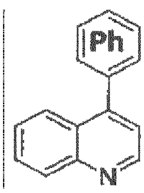 | -6.09 | -1-47 | **2.57** |

(continued)

| | Structure of G-phenyl | HOMO [eV] | LUMO [eV] | Dipole moment [D] |
|---|---|---|---|---|
| G-phenyl-47 | 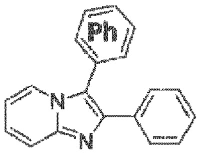 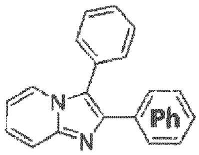 | -5.37 | -0.98 | **3.32** |
| G-phenyl-48 | 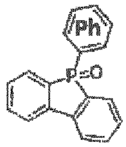 | -6.22 | -1.47 | **4.49** |
| G-phenyl-49 | 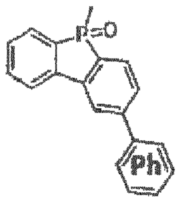 | -6.15 | -1.55 | **4.79** |
| G-phenyl-50 | 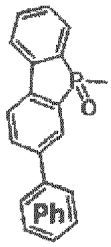 | -5.95 | -1.58 | **4.45** |
| G-phenyl-51 | 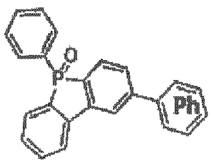 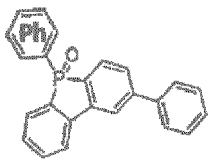 | -6.10 | -1.57 | **4.65** |
| G-phenyl-52 | 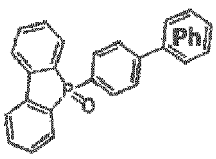 | -6.14 | -1.47 | **4.59** |
| G-phenyl-53 | 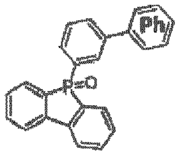 | -6.12 | -1.48 | **4.26** |

(continued)

| | Structure of G-phenyl | HOMO [eV] | LUMO [eV] | Dipole moment [D] |
|---|---|---|---|---|
| G-phenyl-54 | 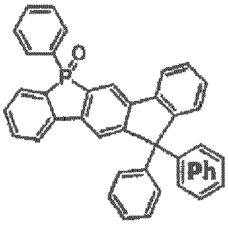 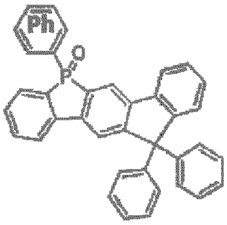 | -5.71 | -1.60 | **4.55** |
| G-phenyl-55 | 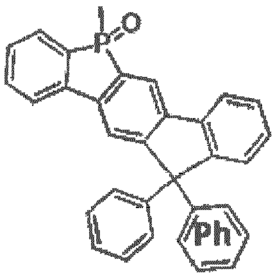 | -5.74 | -1.60 | **4.73** |
| G-phenyl-56 | 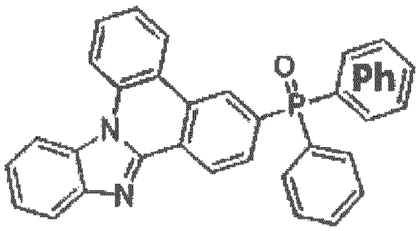 | -5.75 | -1.62 | **-6.70** |
| G-phenyl-57 | 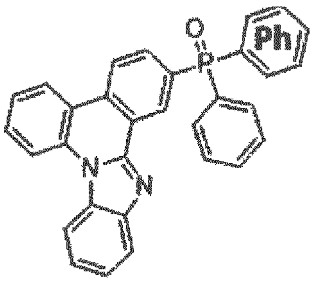 | -5.68 | -1.61 | **6.58** |
| G-phenyl-58 | 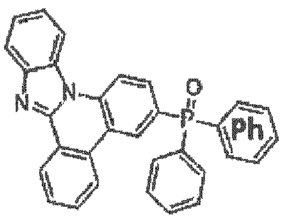 | -5.71 | -1.45 | **4.34** |

(continued)

| | Structure of G-phenyl | HOMO [eV] | LUMO [eV] | Dipole moment [D] |
|---|---|---|---|---|
| G-phenyl-59 | 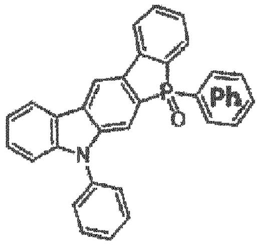 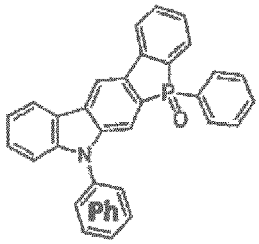 | -5.42 | -1.34 | **4.33** |
| G-phenyl-60 | 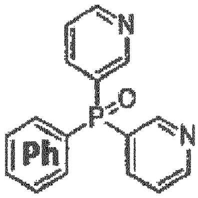 | -6.89 | -1.25 | **3.44** |
| G-phenyl-61 | 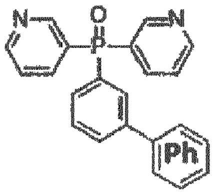 | -6.32 | -1.28 | **3.20** |
| G-phenyl-62 | 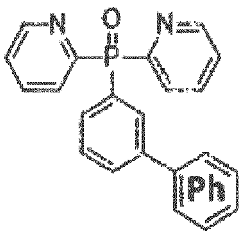 | -5.96 | -1.24 | **4.42** |
| G-phenyl-63 | 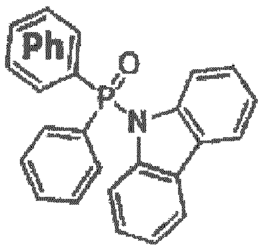 | -5.63 | -0.96 | **4.10** |
| G-phenyl-64 | 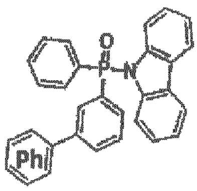 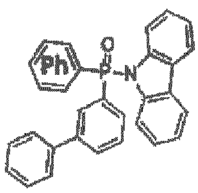 | -5.64 | -1.05 | **4-23** |

(continued)

| | Structure of G-phenyl | HOMO [eV] | LUMO [eV] | Dipole moment [D] |
|---|---|---|---|---|
| G-phenyl-65 | 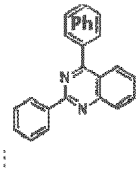 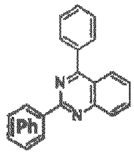 | -6.00 | -1.88 | **2.46** |
| G-phenyl-66 | 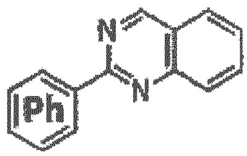 | -6.12 | -1.82 | **2.22** |
| G-phenyl-67 | 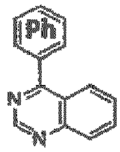 | -6.36 | -1.87 | **3.04** |
| G-phenyl-68 | 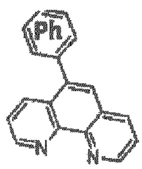 | -6.03 | -1.46 | **3.58** |
| G-phenyl-69 | 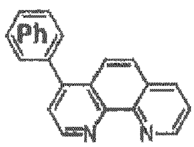 | -6.09 | -1.46 | **3.67** |
| G-phenyl-70 | 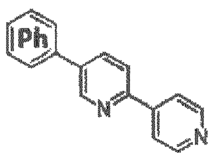 | -6.17 | -1.85 | **4.56** |
| G-phenyl-71 | 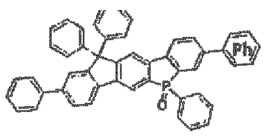 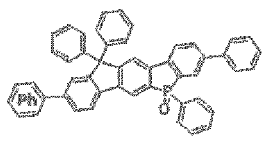 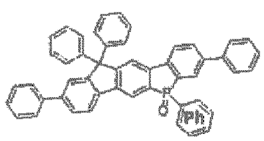 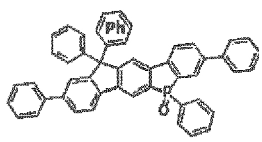 | -5.50 | -1.74 | **4.45** |

(continued)

| | Structure of G-phenyl | HOMO [eV] | LUMO [eV] | Dipole moment [D] |
|---|---|---|---|---|
| G-phenyl-72 | 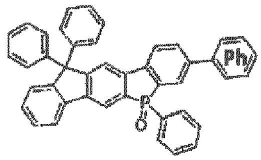 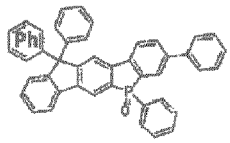 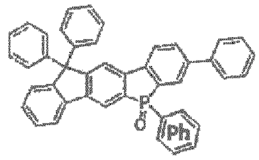 | -5.55 | -1.76 | **4.44** |
| G-phenyl-73 | 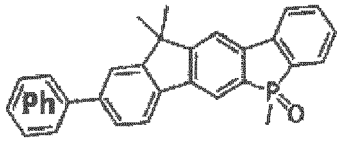 | -5.58 | -1.64 | **4.98** |
| G-phenyl-74 | 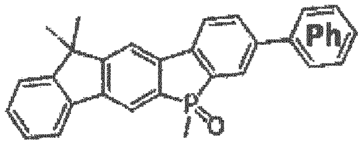 | -5.60 | -1.66 | **4.81** |
| G-phenyl-75 | 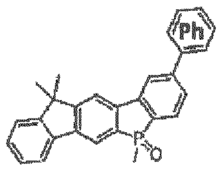 | -5.70 | -1.60 | **5.11** |
| G-phenyl-76 | 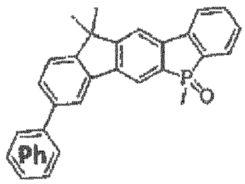 | -5.66 | -1.58 | **4.90** |
| G-phenyl-77 | 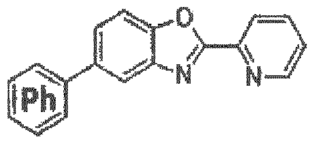 | -5.95 | -1.70 | **1.25** |
| G-phenyl-78 | 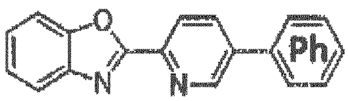 | -5.92 | -1.91 | **1.83** |

(continued)

| | Structure of G-phenyl | HOMO [eV] | LUMO [eV] | Dipole moment [D] |
|---|---|---|---|---|
| G-phenyl-79 | 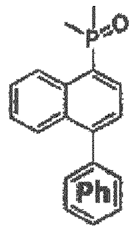 | -5.84 | -1.41 | **4.28** |
| G-phenyl-80 | 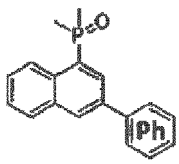 | -5.81 | -1.40 | **3.98** |
| G-phenyl-81 | 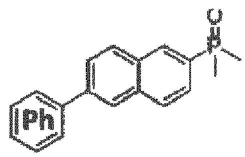 | -5.84 | -1.61 | **4.50** |
| G-phenyl-82 | 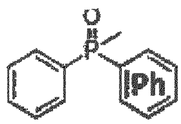 | -6.85 | -0.85 | **4.00** |
| G-phenyl-83 | 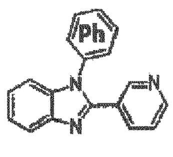 | -5.88 | -1.27 | **2.59** |
| G-phenyl-84 | 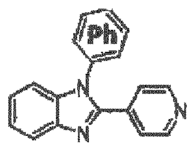 | -6.02 | -1.48 | **4.35** |

G may be selected from the group consisting of dialkylphosphinyl, diarylphosphinyl, alkylarylphosphinyl, diheteroarylphosphinyl, arylheteroarylphosphinyl, cyclic diarylphosphinyl, phosphine oxide, aryl-containing phosphine oxide, heteroaryl-containing phosphine oxide, cyclic arylheteroarylphosphinyl, cyclic heteroaryl-containing phosphine oxide, nitrile, benzonitrile, nicotinonitrile, amide, carbamide, and $C_2$ to $C_{42}$ heteroaryl; wherein G may include one or more substituents attached to the group, wherein the one or more substituents are selected from the group consisting of $C_6$ to $C_{18}$ aryl, $C_1$ to $C_{10}$ alkyl, $C_2$ to $C_{14}$ heteroaryl. In this regard, "cyclic means that the "P=O" of the phosphinyl, respectively the phosphine oxide is part of a ring formed with further moieties of the group.

G may be selected from the group consisting of di-$C_1$ to $C_{10}$-alkylphosphinyl, di-$C_6$ to $C_{10}$-arylphosphinyl, $C_{10}$-$C_{42}$ diheteroarylphosphinyl, $C_7$-$C_{42}$ arylheteroarylphosphinyl, $C_8$-$C_{42}$ phosphine oxide, $C_8$-$C_{42}$ aryl-containing phosphine oxide, $C_8$-$C_{63}$ heteroaryl-containing phosphine oxide, $C_{12}$-$C_{63}$ cyclic arylphosphinyl, $C_7$-$C_{42}$ cyclic arylheteroarylphosphinyl, $C_7$-$C_{42}$ cyclic heteroaryl-containing phosphine oxide, and $C_2$ to $C_{39}$ heteroaryl, optionally $C_2$ to $C_{35}$ heteroaryl, optionally $C_2$ to $C_{32}$ heteroaryl, optionally $C_2$ to $C_{29}$ heteroaryl, optionally $C_2$ to $C_{25}$ heteroaryl; G may include one or

more substituents attached to the group, wherein the one or more substituents are selected from the group consisting of $C_6$ to $C_{12}$ aryl, $C_1$ to $C_6$ alkyl, $C_2$ to $C_{11}$ heteroaryl.

G may be selected from the group consisting of di-$C_1$ to $C_4$-alkylphosphinyl, di-$C_6$ to $C_{10}$-arylphosphinyl, $C_{10}$ diheteroarylphosphinyl, $C_7$-$C_{25}$ arylheteroarylphosphinyl, $C_8$-$C_{42}$ phosphine oxide, $C_8$-$C_{42}$ aryl-containing phosphine oxide, $C_8$-$C_{24}$ heteroaryl-containing phosphine oxide, $C_{12}$-$C_{42}$ cyclic arylphosphinyl, $C_7$-$C_{25}$ cyclic arylheteroarylphosphinyl, $C_7$-$C_{25}$ cyclic heteroaryl-containing phosphine oxide, and $C_2$ to $C_{25}$ heteroaryl; wherein the respective G may include one or more substituents attached to the group, wherein the one or more substituents are selected from the group consisting of $C_6$ to $C_{10}$ aryl, $C_1$ to $C_4$ alkyl, $C_2$ to $C_5$ heteroaryl.

G is selected from the group consisting of dialkylphosphinyl, diarylphosphinyl, alkylarylphosphinyl, diheteroarylphosphinyl, arylheteroarylphosphinyl, cyclic diarylphosphinyl, phosphine oxide, aryl-containing phosphine oxide, heteroaryl-containing phosphine oxide, cyclic arylheteroarylphosphinyl, cyclic heteroaryl-containing phosphine oxide, nitrile, benzonitrile, nicotinonitrile, amide-yl, carbamide-yl and $C_2$ to $C_{17}$ heteroaryl; wherein the respective G may include one or more substituents attached to the group, wherein the one or more substituents are selected from the group consisting of phenyl, methyl, ethyl, and pyridyl.

G may be selected independently from the group consisting of dimethylphosphinyl, diphenylphosphinyl, nitrile, benzonitrile, nicotinonitrile, di-hydro-benzoimidazolone-yl, diphenyl-propane-yl, *N,N*-dimethylacetamid, amide, carbamide, imidazolyl, phenylbenzoimidazolyl, ethylbenzoimidazolyl phenylbenzoquinolinyl, phenylbenzoimidazoquinolinyl, pyridinyl, bipyridinyl, picolinyl, lutidenyl, pyridazinyl, pyrimidinyl, pyrazinyl, triphenyl-pyrazinyl, benzoquinolinyl, phenanthrolinyl, phenylphenanthrolinyl, quinazolinyl, benzoxazolyl, benzimidazolyl, pyridinylimidazopyridinyl;

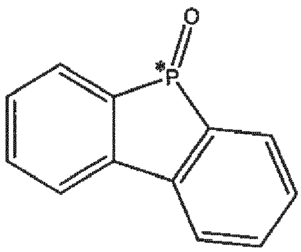 ; 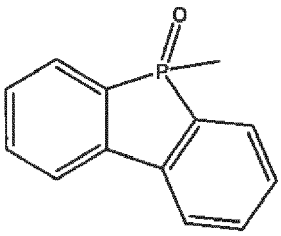 ; 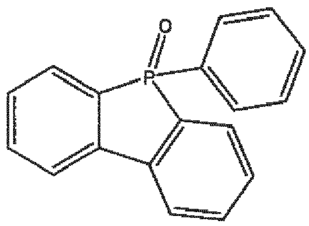 ;

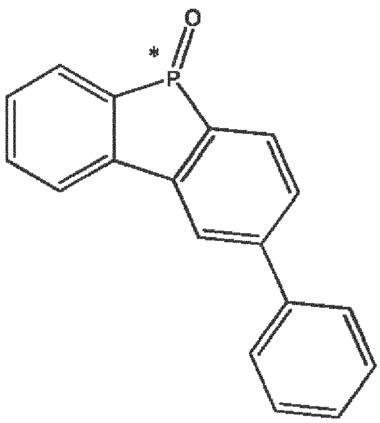 ; 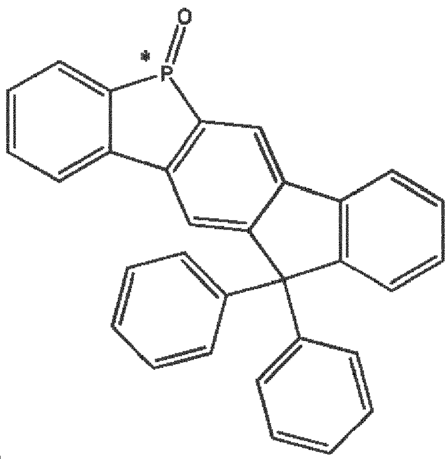 ;

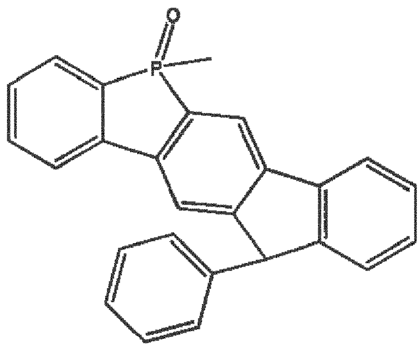
;
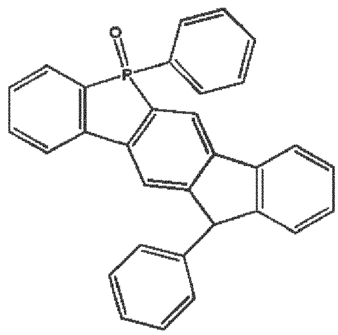
;
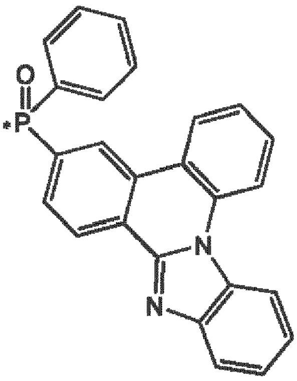
;

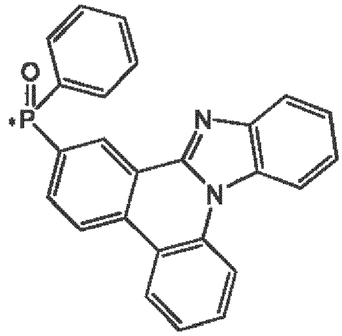
;
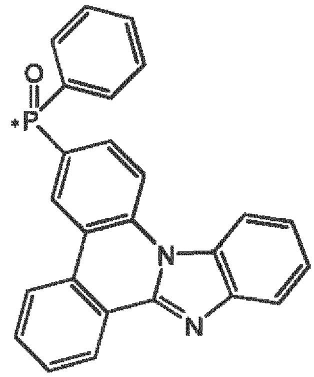
;
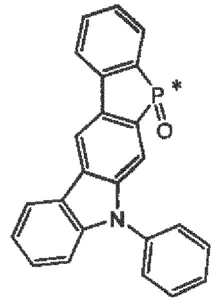
;

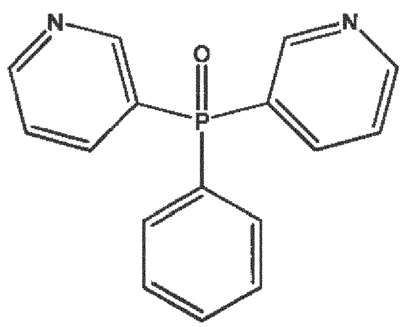
;
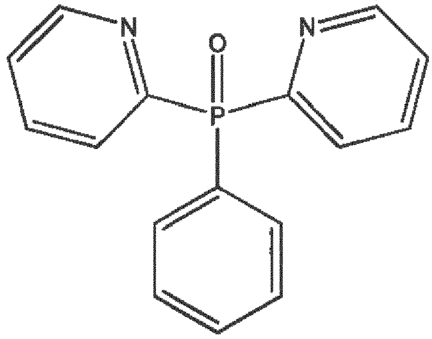
;
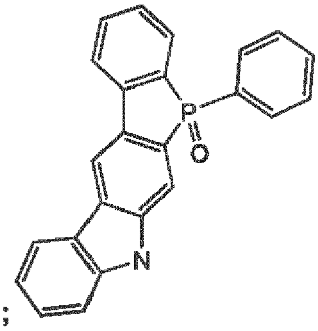
;

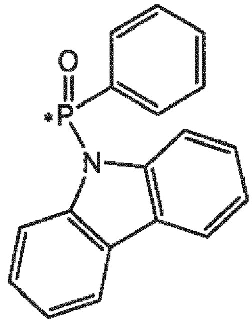
;
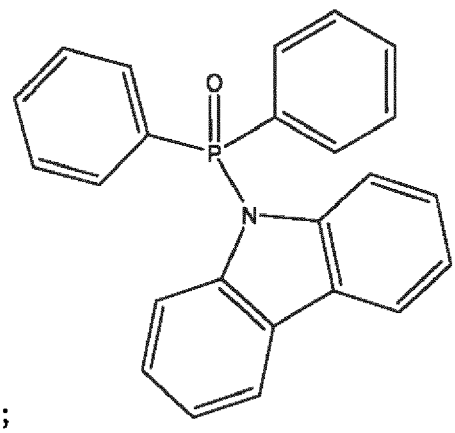
;
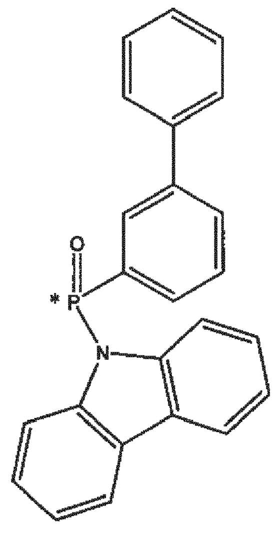
;

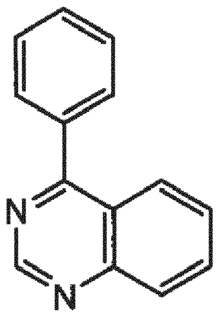
;

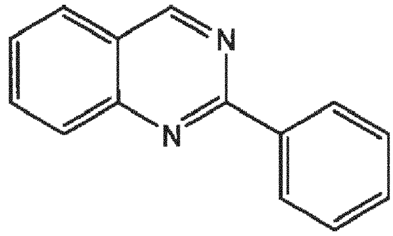
;

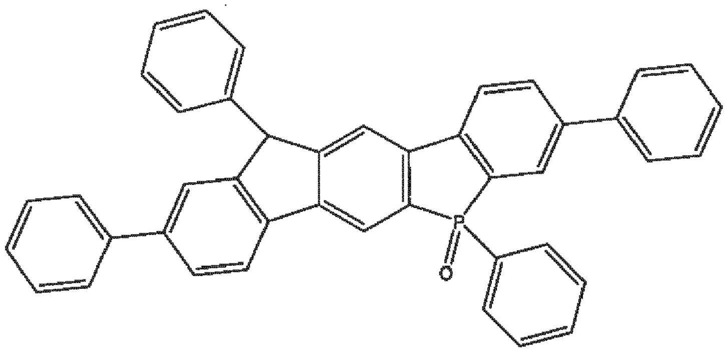
;

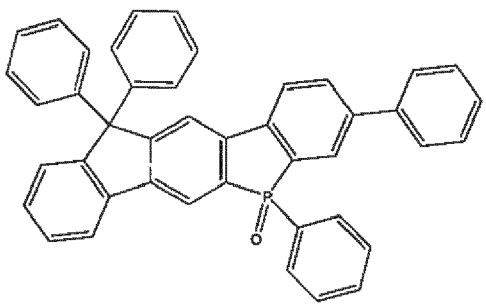
;

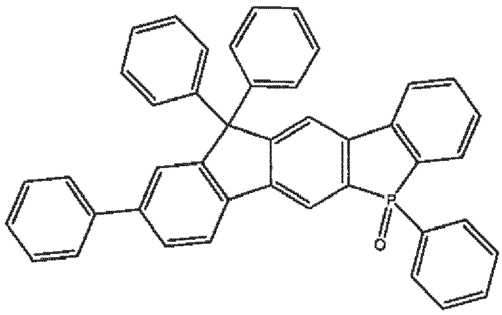
;

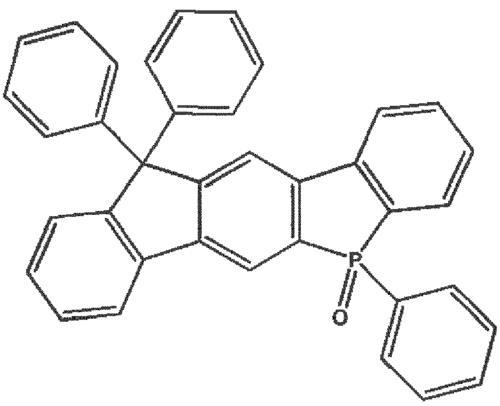
;

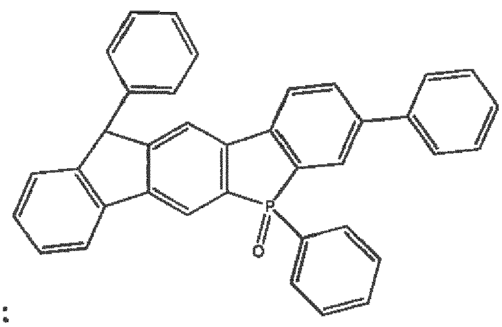
;

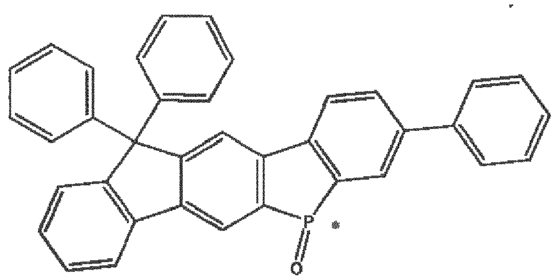

:

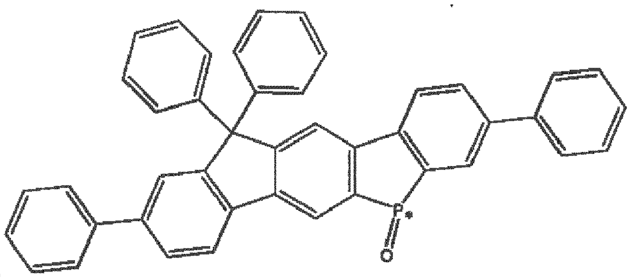

;

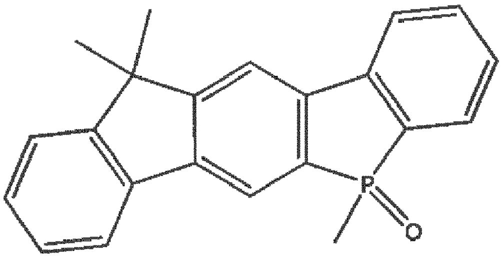

;

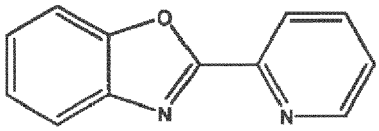

;

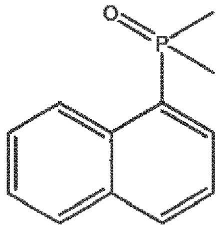

;

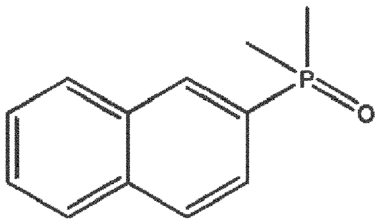

;

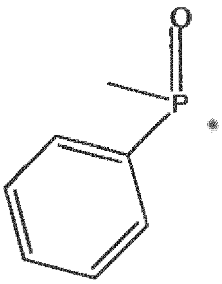

;

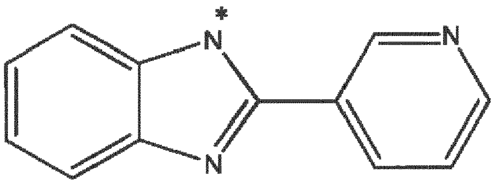

;

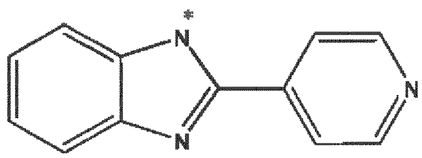

wherein the asterisk symbol "*" represents the binding position.

G may be selected independently from the group consisting of dimethylphosphinyl, diphenylphosphinyl, 2-phenyl-1H-benzo[d]imidazolyl, 2-ethyl-1H-benzo[d]imidazolyl, 2-phenylbenzo[h]quinolinyl, pyridinyl, 2,2'-bipyridinyl, 5-phenylbenzo[4,5]imidazo[1,2-a]quinolinyl, 9-phenyl-1,10-phenanthrolinyl, 2-quinazolinyl, 4-quinazolinyl, 4-phenyl-2-quinazolinyl and (pyridme-2-yl)imidazo[1,5-a]pyridinyl;

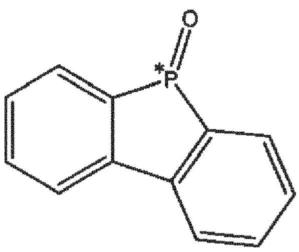

;

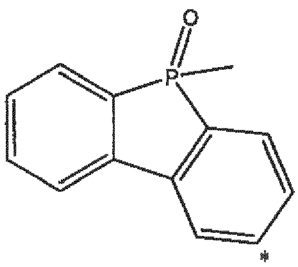

;

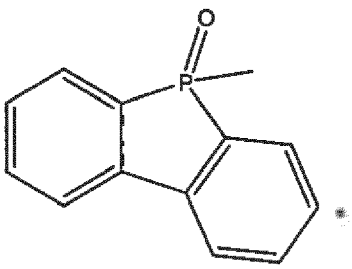

;

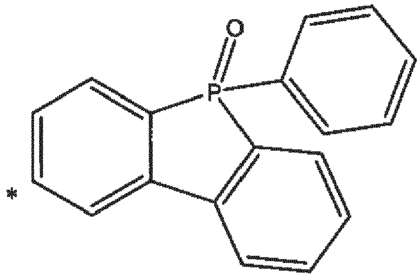 ; 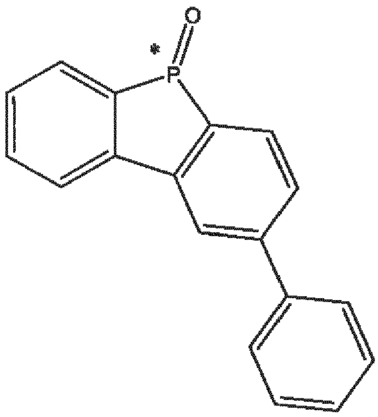 ; 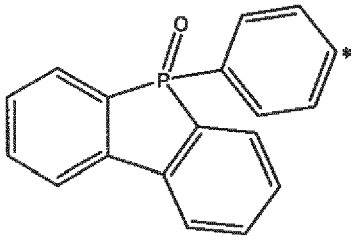 ;

;

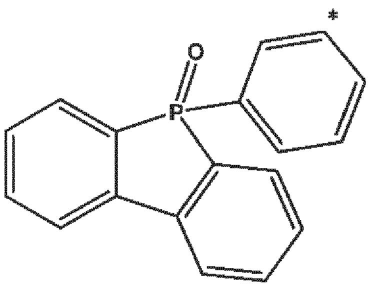 ; 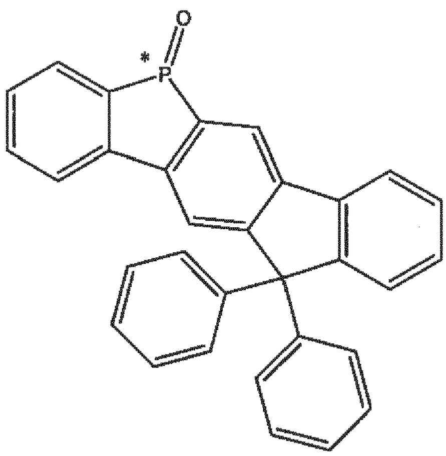 ;

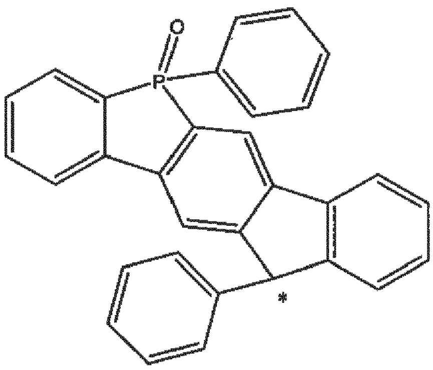 ; 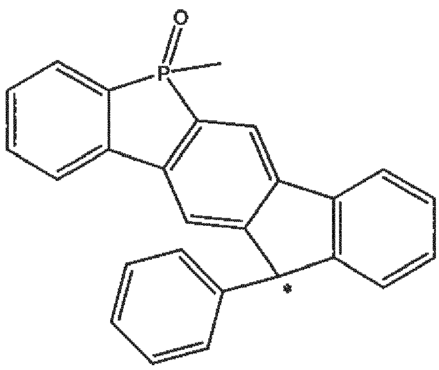 ; 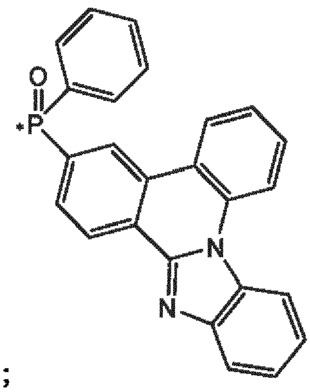 ;

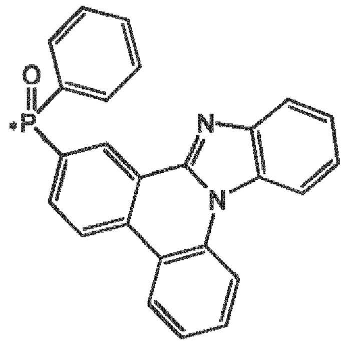 ; 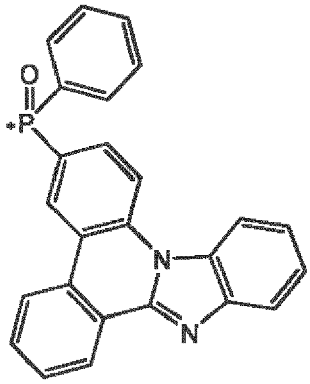 ; 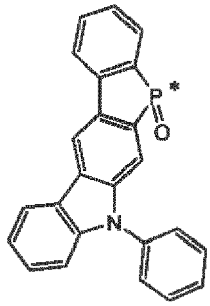 ; 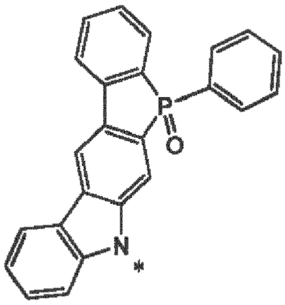 ;

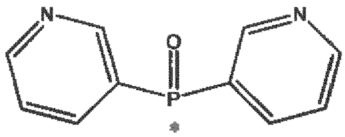 ; 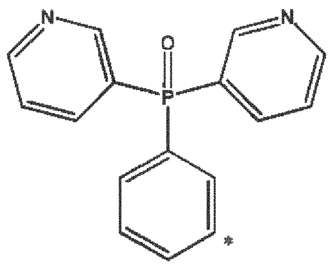 ; 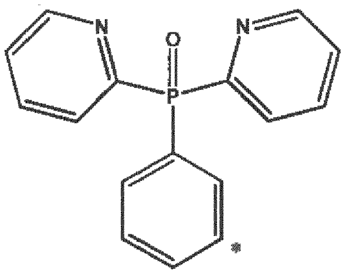 ;

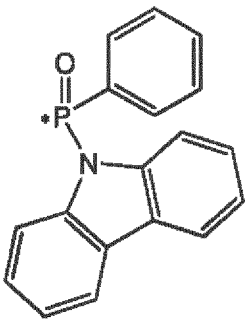 ; 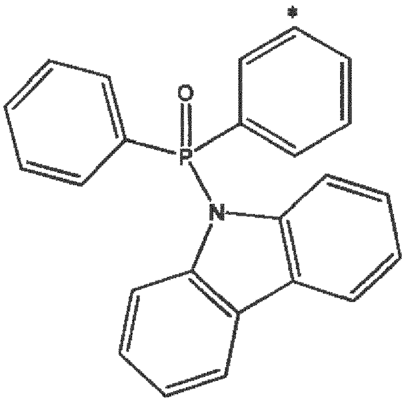 ; 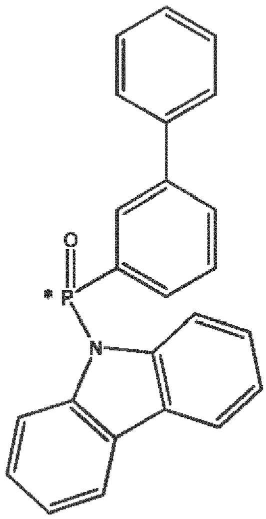 ; 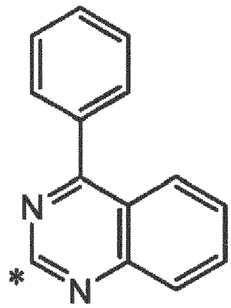

; 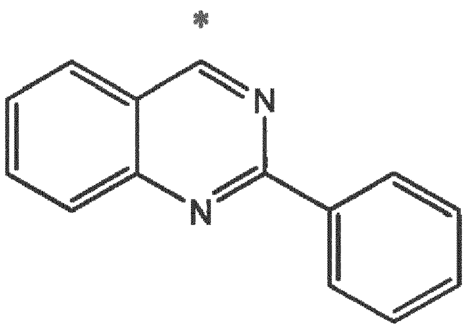 ; 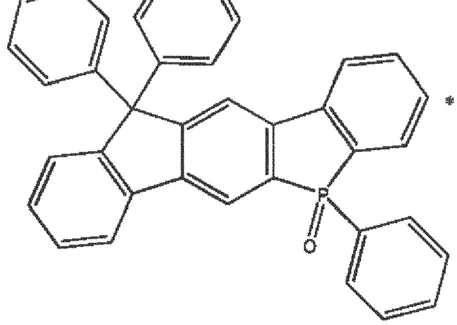 ;

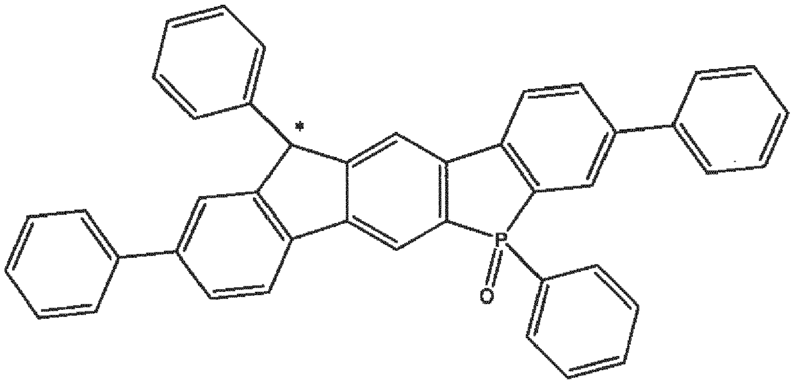

;

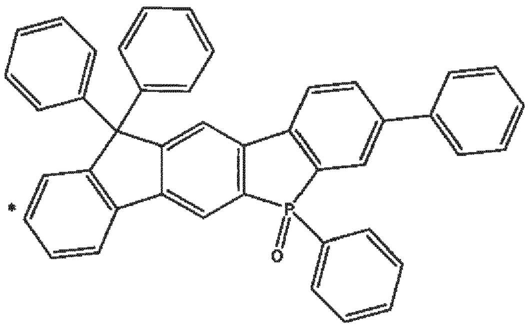

;

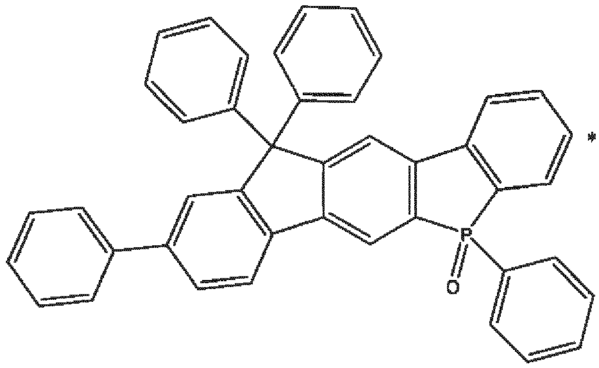

;

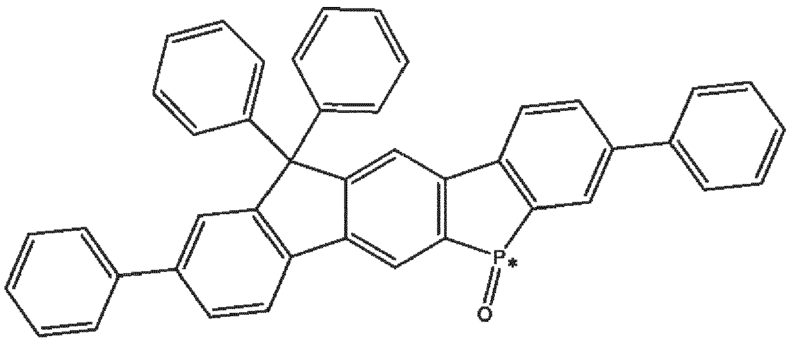

;

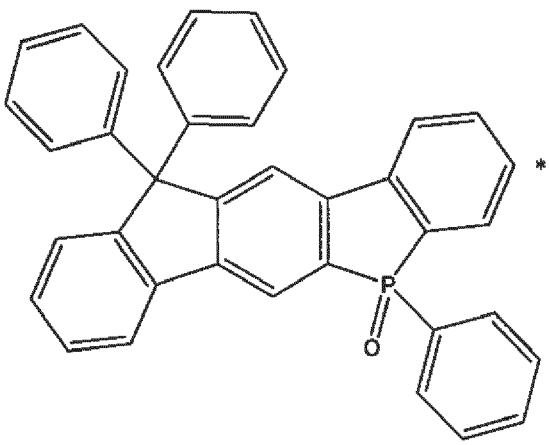

;

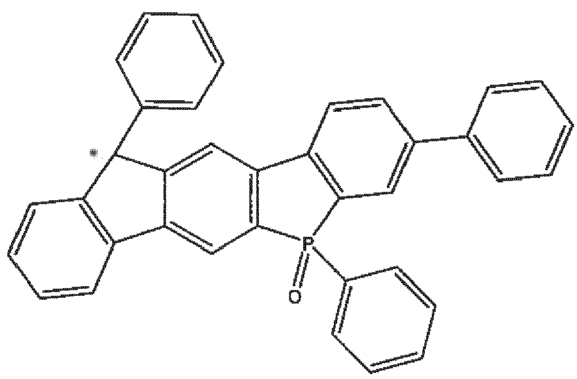

;

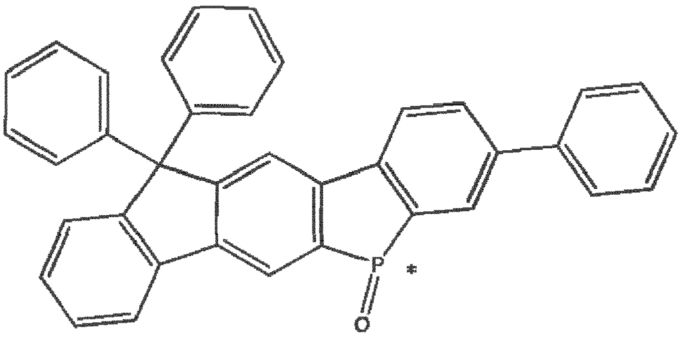

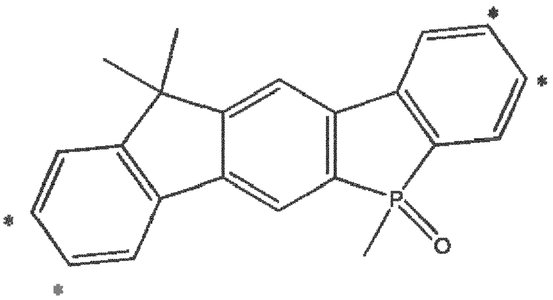

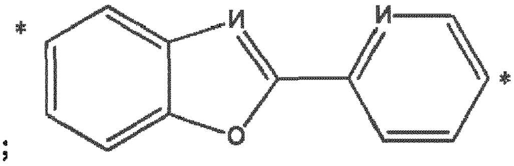

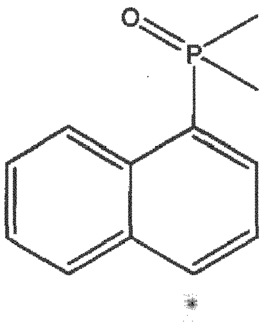

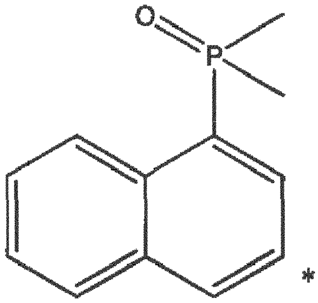

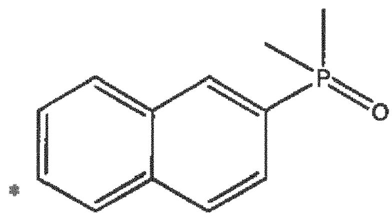

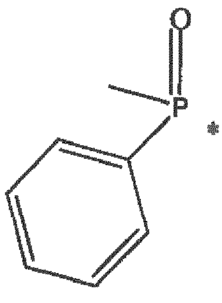

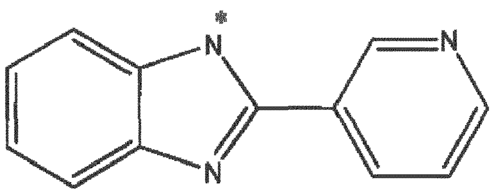

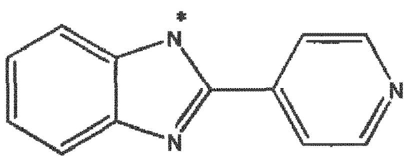

wherein the asterisk symbol "*" represents the binding position.

The compound of Formula (II) may be selected from the compounds B-1 to B-25 of the following Table 2.

Table 2:

| Name | Structure | HOMO [eV] | LUMO [eV] | Dipole moment [D] |
|---|---|---|---|---|
| B-1 | 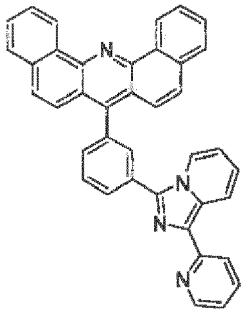 | -5.03 | -1.81 | 0.98 |
| B-2 | 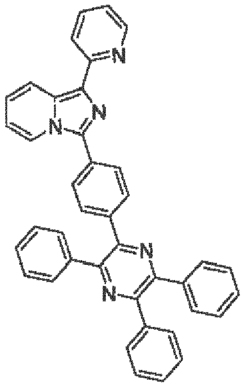 | -4.94 | -1.61 | 1.77 |
| B-3 | 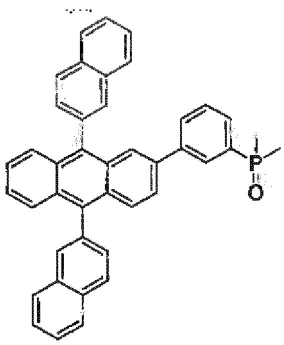 | -5.11 | -1.75 | 3.78 |
| B-4 | 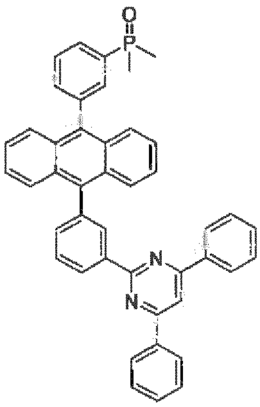 | -5.20 | -1.75 | 6.15 |

(continued)

| Name | Structure | HOMO [eV] | LUMO [eV] | Dipole moment [D] |
|---|---|---|---|---|
| B-5 | 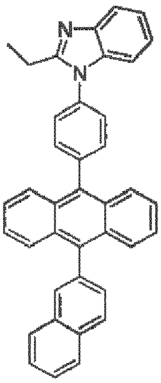 | -5.26 | -1.81 | -/- |
| B-6 | 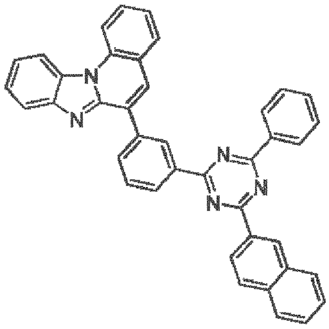 | -5.56 | -1.85 | 3.39 |
| B-7 | 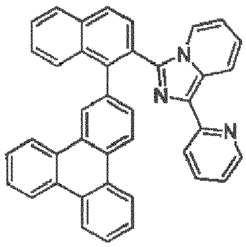 | -5.11 | -1.28 | 3.89 |
| B-8 | 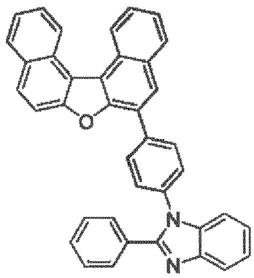 | -5.48 | -1.69 | 4.58 |
| B-9 | 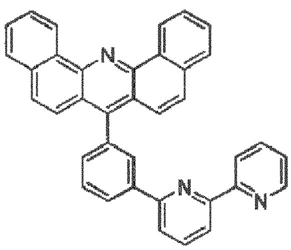 | -5.48 | -1.59 | 4.68 |

(continued)

| Name | Structure | HOMO [eV] | LUMO [eV] | Dipole moment [D] |
|---|---|---|---|---|
| B-10 | 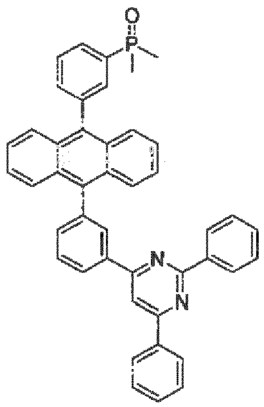 | -5.34 | -1.86 | 2.59 |
| B-11 | 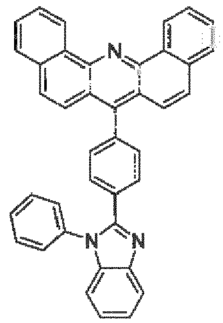 | -5.61 | -1.79 | 3.80 |
| B-12 | 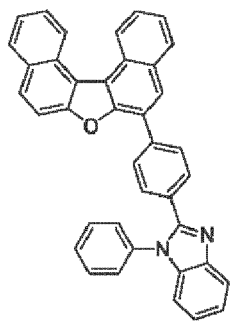 | -5.33 | -1.61 | 3.86 |
| B-13 | 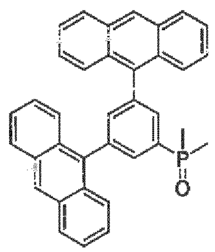 | -5.19 | -1.81 | 4.11 |

(continued)

| Name | Structure | HOMO [eV] | LUMO [eV] | Dipole moment [D] |
|---|---|---|---|---|
| B-14 | 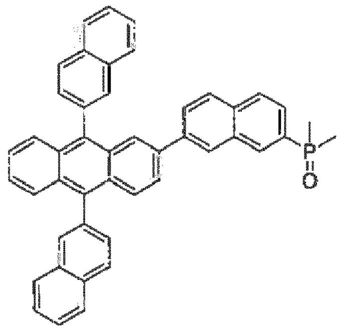 | -5.11 | -1.80 | 3.84 |
| B-15 | 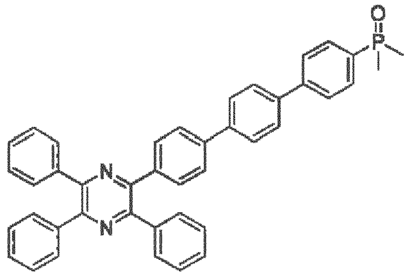 | -5.69 | -1.67 | 4.37 |
| B-16 | 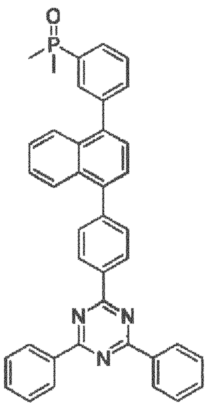 | -5.76 | -1.97 | 4.27 |
| B-17 | 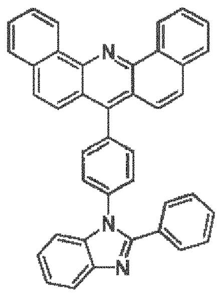 | -5.77 | -1.91 | 2.15 |

(continued)

| Name | Structure | HOMO [eV] | LUMO [eV] | Dipole moment [D] |
|---|---|---|---|---|
| B-18 | 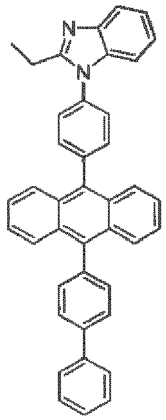 | -5.29 | -1.80 | 4.46 |
| B-19 | 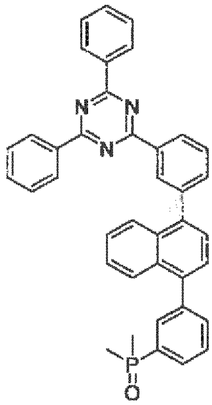 | -5.73 | -1.90 | 4.49 |
| B-20 | 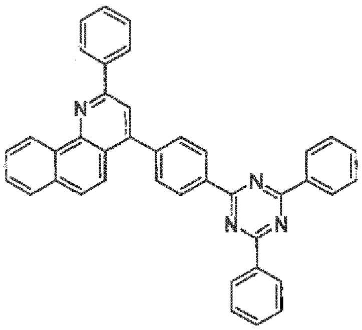 | -5.67 | -2.04 | 1.82 |
| B-21 | 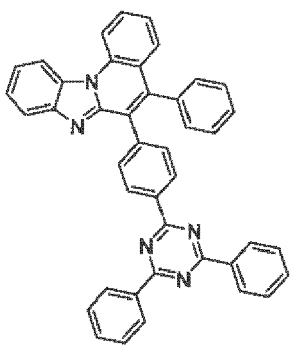 | -5.49 | -1.89 | 3.36 |

(continued)

| Name | Structure | HOMO [eV] | LUMO [eV] | Dipole moment [D] |
|---|---|---|---|---|
| B-22 | 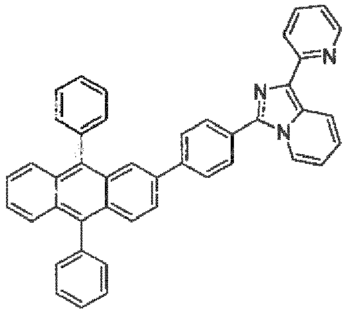 | -4.86 | -1.77 | 2.03 |
| B-23 | 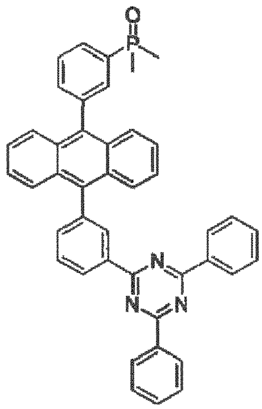 | -5.28 | -1.90 | 4.47 |
| B-24 | 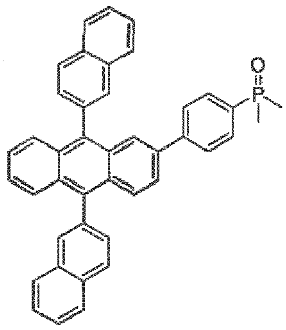 | -5.17 | -1.84 | 4.22 |
| B-25 | 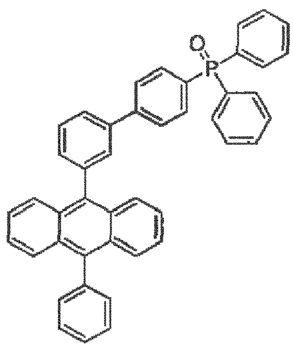 | -5.16 | -1.67 | 4.13 |

In an embodiment the LUMO energy level of the compound of formula (II) in the absolute scale taking vacuum energy level as zero, computed by the TURBOMOLE V6.5 program package using hybrid functional B3LYP and Gaussian 6-31G* basis set, is in the range from - 2.30 eV to -1.20 eV, preferably from -2.10 eV to -1.28 eV.

In an embodiment the compound of formula (II) comprises one polar group "G".

The compound of Formula (II) may comprise 6 to 12 aromatic or heteroaromatic rings, optionally 6 to 11 aromatic

or heteroaromatic rings, optionally 7 to 10 aromatic or heteroaromatic rings. optionally 7 to 9 aromatic or heteroaromatic rings. In this regard, an aromatic, respectively heteroaromatic ring, is a single aromatic ring, for example a 6-membered aromatic ring such as phenyl, a 6-membered heteroaromatic ring, an example would be pyridyl, a 5-membered heteroaromatic ring an example would be pyrrolyl etc. In a system of condensed (hetero)aromatic rings, each ring is considered as a single ring in this regard. For example, naphthalene comprises two aromatic rings.

It may be provided that the compound of Formula (II) does not contain a moiety P=O. It may be provided that the compound of Formula (II) does not contain $P(=O)Aryl_2$. It may be provided that the compound of Formula (II) does not contain $P(=O)Alkyl_2$. It may be provided that the compound of Formula (II) does not contain $P(=O)Ph_2$. It may be provided that the compound of Formula (II) does not contain $P(=O)(CH_3)_2$. It may be provided that the compound of Formula (II) does not contain R'P(=O)R" wherein R' and R" are connected with each other to form a ring, that is, does not contain ring-phosphine oxide. It may be provided that the compound of Formula (II) does not contain R'P(=O)R" wherein R' and R" are connected with each other to form a 7-membered ring.

It may be provided that the compound of Formula (II) does not contain two moieties P=O. It may be provided that wherein the compound of Formula (II) does not contain two $P(=O)Aryl_2$. It may be provided that wherein the compound of Formula (II) does not contain two $P(=O)Alkyl_2$. It may be provided that wherein the compound of Formula (II) does not contain two $P(=O)Ph_2$. It may be provided that wherein the compound of Formula (II) does not contain two $P(=O)(CH_3)_2$. It may be provided that wherein the compound of Formula (II) does not contain CN.

It may be provided that one or more of the following formulas are excluded from the scope of the Compound of Formula (II)

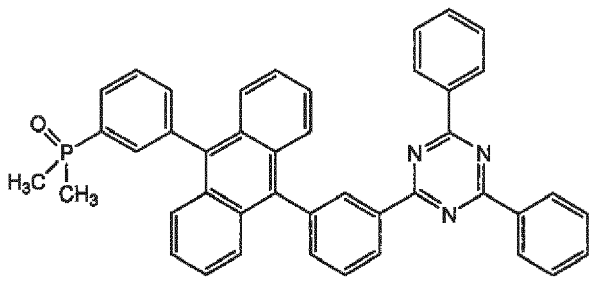 ; 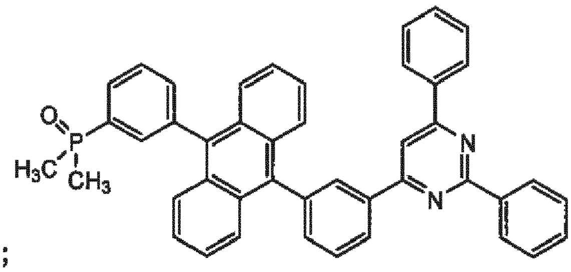 ;

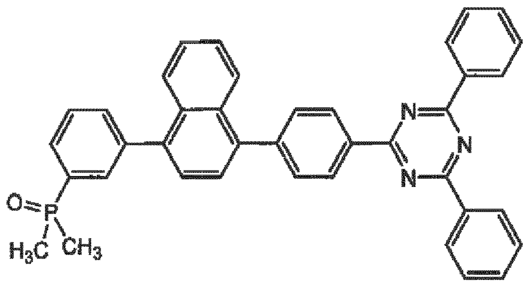 ; 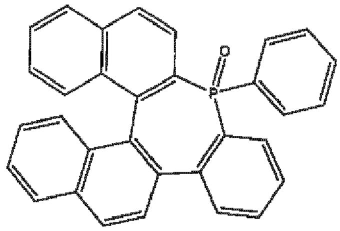 ;

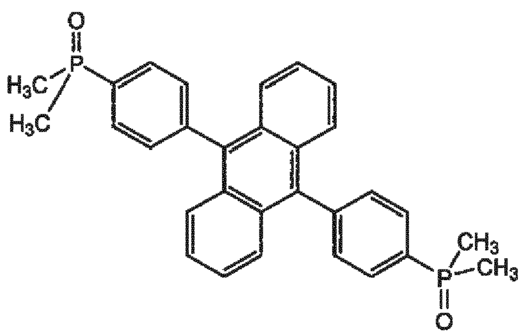 ; 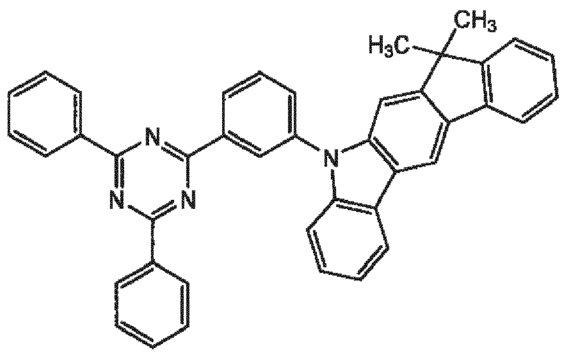 ;

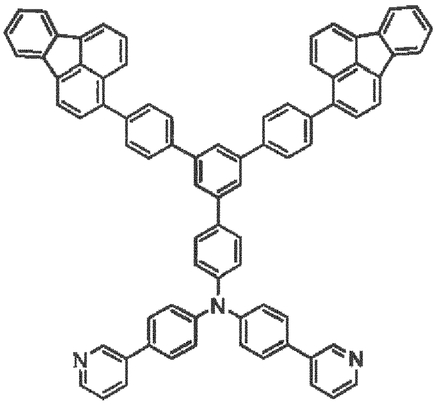

;

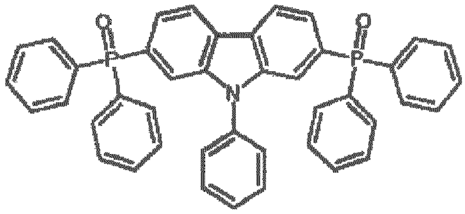

;

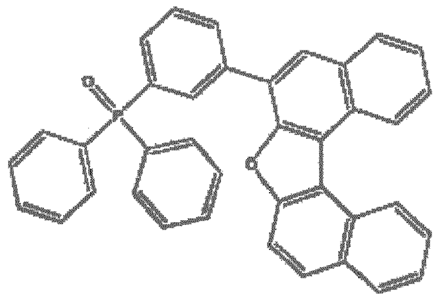

It may be provided that if the second electron transport layer comprises a compound of Formula (II) and a compound (III), the following combinations of compounds (with reference to Tables 2 and 2 in the specified amounts are excluded:

| | |
|---|---|
| B-23:C-3 | 30:70 v:v; |
| C-3:B-10 | 30:70 v:v; |
| B-23:C-5 | 30:70 v:v; |
| B-10:C-5 | 30:70 v:v; |
| B-23:C-6 | 30:70 v:v; |
| B-10:C-6 | 30:70 v:v. |

The following organic light emitting diodes a) and b) comprising the following compounds

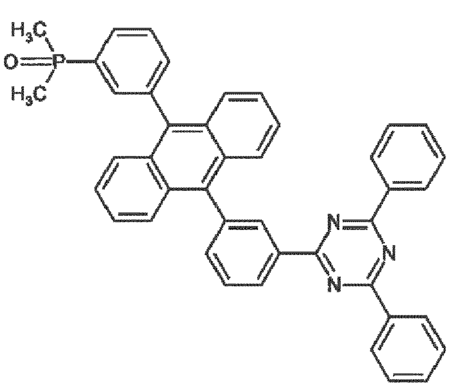

B-23

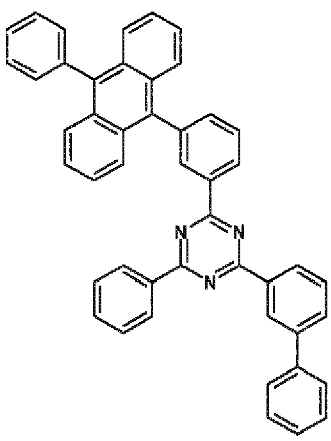

C-3

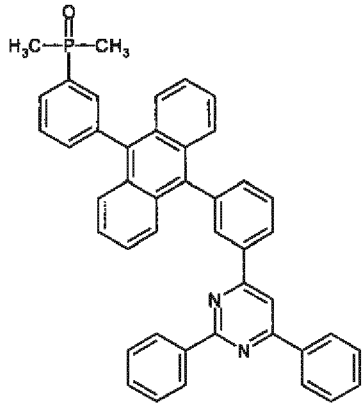

B-10

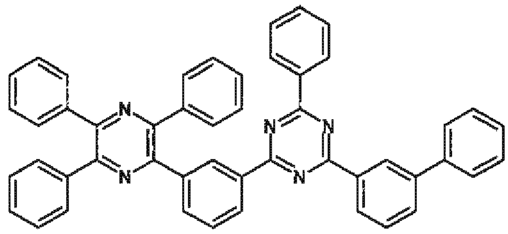

C-6

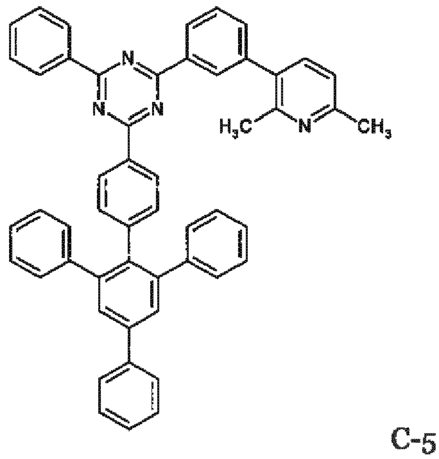

C-5

may be excluded:

a) tandem OLED, wherein the electron transport layer is arranged adjacent to and in direct contact with a n-doped charge generation layer made of compound E

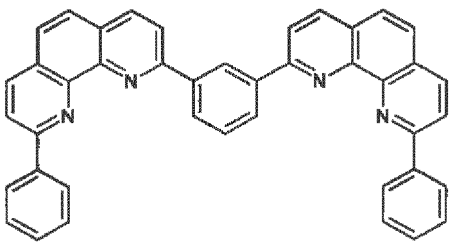

E

and metallic lithium in weight ratio E:Li equal to 98:2, the composition of the electron transport layer is selected from B-23:C-3, B-10:C-3, B-23:C-5, B-10:C-5, B-23:C-6, B-10:C-6; and weight ratio of the first and the component in each of these compositions is 30:70;

b) top emitting blue OLED having structure

| layer | Material | d [nm] |
|---|---|---|
| Anode | Ag | 100 |
| HIL | HT-3:D-1 (92:8 v/v) | 10 |
| HTL | HT-3 | 118 |
| EBL | F2 | 5 |

(continued)

| layer | Material | d [nm] |
|---|---|---|
| EML | H09:BD200 (97:3 v/v) | 20 |
| HBL | C-1 | 5 |
| ETL | B-23:C-3 (30:70 v/v) | 31 |
| EIL | Yb | 2 |
| Cathode | Ag:Mg (90:10) | 13 |
| Cap | HT-3 | 70 |

wherein

HT-3 is

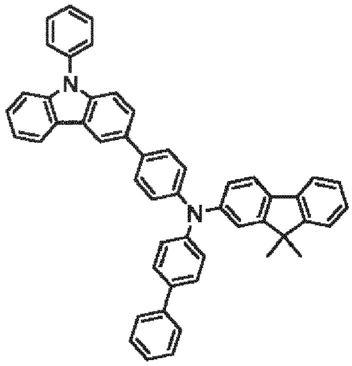

F2 is

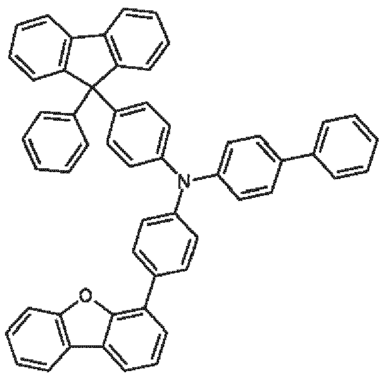

C-1 is

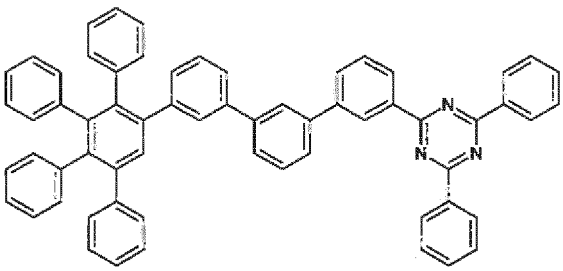

D-1 is

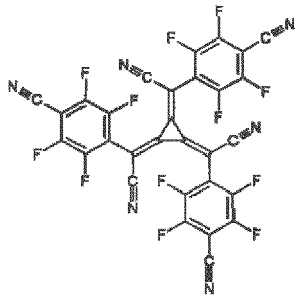

,

H09 is a commercial blue emitter host and BD200 is a commercial blue emitter, both supplied by SFC, Korea.

The second electron transport layer may further comprise a compound (III), wherein the compound (III) comprises 8 to 13 aromatic or heteroaromatic rings, optionally 8 to 11 aromatic or heteroaromatic rings, optionally 9 to 11 aromatic or heteroaromatic rings, and optionally 9 aromatic or heteroaromatic rings, wherein one or more of the aromatic or heteroaromatic rings may be substituted with $C_1$ to $C_4$ alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The compound (III) may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the compound (III) may be 6-membered rings.

The heteroaromatic rings of the compound (III) may be a N-containing heteroaromatic ring, optionally all of the heteroaromatic rings are N-containing heteroaromatic rings, optionally all of the heteroaromatic rings heteroaromatic rings contain N as the only type of heteroatom.

The compound (III) may comprise at least one 6-membered heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the compound (III) may be an azine. The at least one 6-membered heteroaromatic ring comprised in the compound (III) may be triazine, diazine, pyrazine.

If the compound (III) comprises two or more heteroaromatic rings, the heteroaromatic rings may be separated from each other by at least one aromatic ring which is free of a heteroatom. In an embodiment, the heteroatoms in the heteroaromatic rings of compound (III) are bound into the molecular structure of compound (III) by at least one double bond.

The molecular dipole moment, computed by the TURBOMOLE V6.5 program package using hybrid functional B3LYP and Gaussian 6-31G* basis set, of the compound (III) may be $\geq$ 0 D and $\leq$ 4 D; alternatively $\geq$ 0.1 D and $\leq$ 3.9 D; alternatively $\geq$ 0.2 D and $\leq$ 3.7 D; alternatively $\geq$ 0.3 D and $\leq$ 3.5 D.

By choosing compound (III) according to these embodiments it is provided that the mobility of the second electron transport layer is further improved and voltage of the OLED device is decreased and the cd/A efficiency of the OLED device is increased.

In an embodiment the compound (HI) is not a compound of formula (II). The compound of Formula (III) may be selected from the compounds C-1 to C-6 of the following Table 3.

Table 3:

| Name | Structure | HOMO [eV] | LUMO [eV] | Dipole moment [D] |
|---|---|---|---|---|
| C-1 | 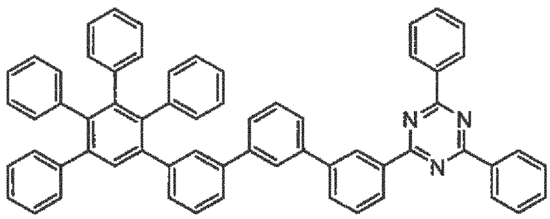 | -5.72 | -1.82 | 0.30 |

(continued)

| Name | Structure | HOMO [eV] | LUMO [eV] | Dipole moment [D] |
|---|---|---|---|---|
| C-2 | 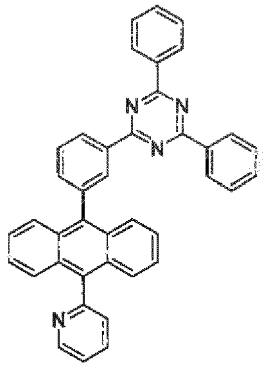 | -5-11 | -1.83 | 1.98 |
| C-3 | 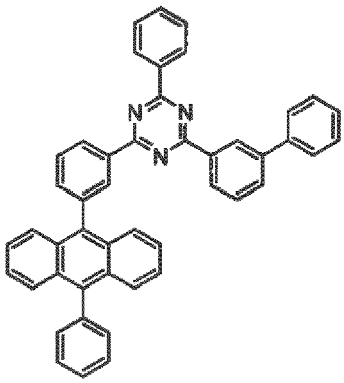 | -5.19 | -1.84 | 0.37 |
| C-4 | 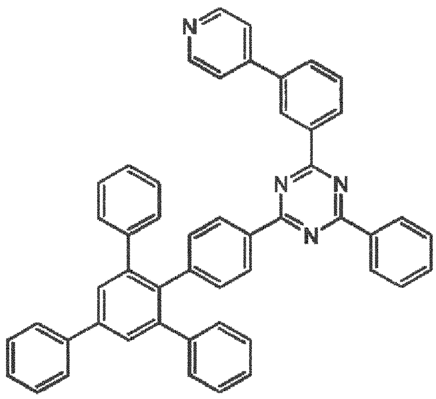 | -5.87 | -1.94 | 3.24 |

(continued)

| Name | Structure | HOMO [eV] | LUMO [eV] | Dipole moment [D] |
|---|---|---|---|---|
| C-5 | 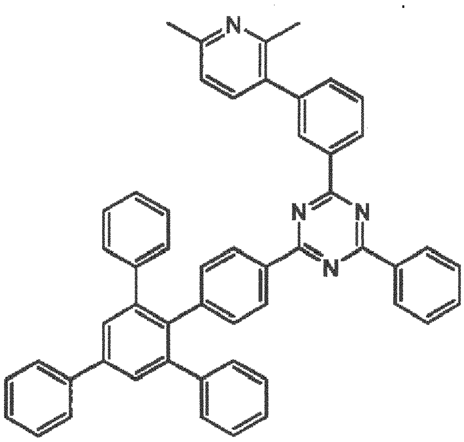 | -5.83 | -1.86 | 1.61 |
| C-6 | 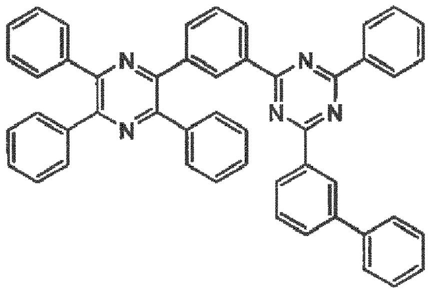 | -5.67 | -1.85 | 0.40 |

It may be provided that the compound of Formula (III) does not contain a moiety P=O. It may be provided that the compound of Formula (III) does not contain P(=O)$Aryl_2$. It may be provided that the compound of Formula (III) does not contain P(=O)$Alkyl_2$. It may be provided that the compound of Formula (III) does not contain P(=O)$Ph_2$. It may be provided that the compound of Formula (III) does not contain P(=O)$(CH_3)_2$. It may be provided that the compound of Formula (III) does not contain R'P(=O)R" wherein R' and R" are connected with each other to form a ring, that is, does not contain ring-phosphine oxide. It may be provided that the compound of Formula (II) does not contain R'P(=O)R" wherein R' and R" are connected with each other to form a 7-membered ring.

It may be provided that the compound of Formula (III) does not contain two moieties P=O. It may be provided that wherein the compound of Formula (III) does not contain two P(=O)$Aryl_2$. It may be provided that wherein the compound of Formula (III) does not contain two P(=O)$Alkyl_2$. It may be provided that wherein the compound of Formula (III) does not contain two P(=O)$Ph_2$. It may be provided that wherein the compound of Formula (III) does not contain two P(=O)$(CH_3)_2$. It may be provided that wherein the compound of Formula (III) does not contain CN.

It may be provided that one or more of the following formulas are excluded from the scope of the Compound of Formula (III)

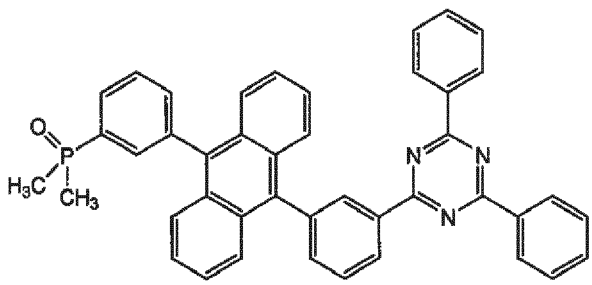 ; 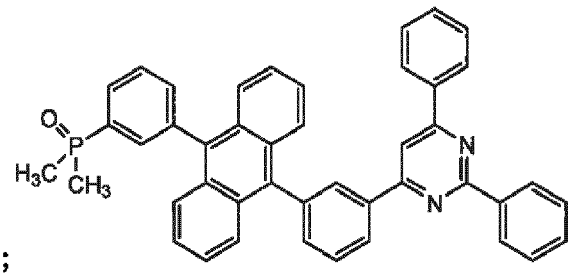 ;

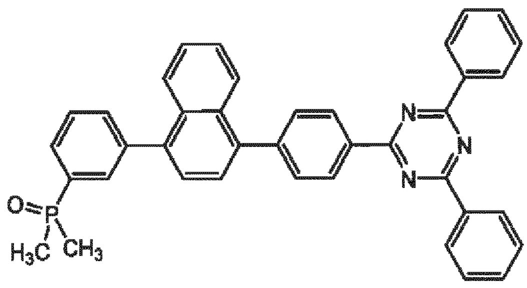
;
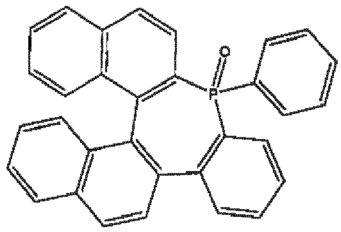
;
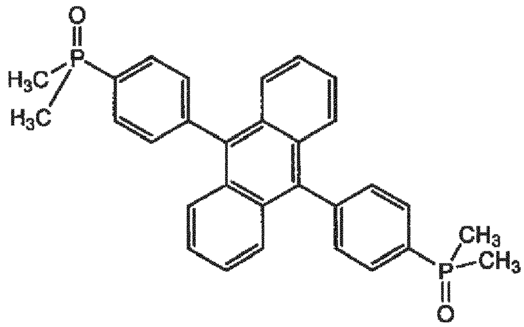
;
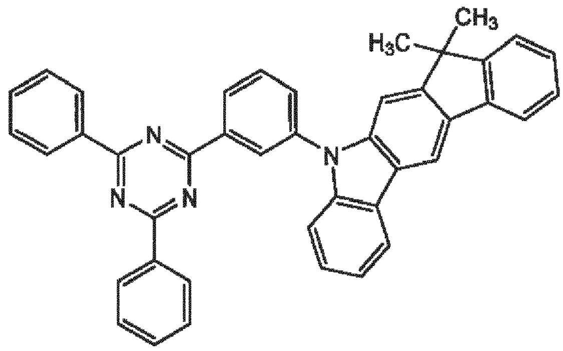
;
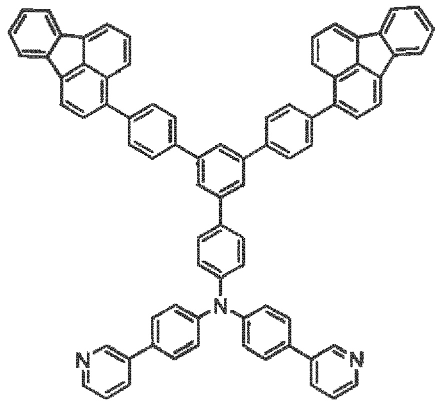
;
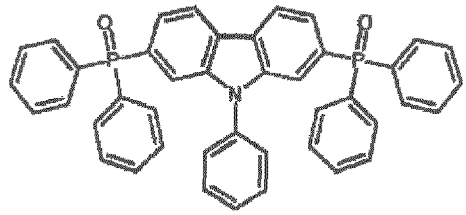
;
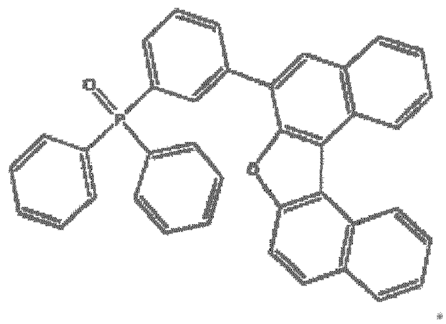

In case that the second electron transport layer comprises both the compound of Formula (II) and compound (III), the weight ratio of Formula (II) to compound (III) may be 1:99 to 99:1, alternatively 10:90 to 60:40, alternatively 20:80 to 50:50, alternatively 25:75 to 40:60, alternatively about 30:70.

In an embodiment the LUMO energy level of the compound of formula (III) in the absolute scale taking vacuum energy level as zero, computed by the TURBOMOLE V6.5 program package using hybrid functional B3LYP and Gaussian 6-31G* basis set, is in the range from - 2.00 eV to -1.70 eV, preferably from -1.95 eV to -1.80 eV.

In an embodiment the compound (III) comprises one nitrogen-containing six-membered ring.

In another embodiment the compound (III) comprises two nitrogen-containing six-membered rings.

In an embodiment the compound of formula (I) is not a compound of formula (II). In a further embodiment the compound of formula (II) is not a compound (III). In another embodiment the compound of formula (I) is not a compound (III). In a further embodiment of the invention all three compounds, namely the compound of formula (I), the compound of formula (II) and compound (III), are different from each other in that they have different molecular structure formulas.

The second electron transport layer may be arranged between the first electron transport layer and the charge generation layer. The second electron transport layer may be arranged in direct contact with the charge generation layer. The second electron transport layer may be arranged in direct contact with the n-type CGL.

The second electron transport layer may be arranged "contacting sandwiched" between the first electron transport layer and the n-type CGL.

It may be provided that the first electron transport layer, the second electron transport layer, and the charge generation layer are each different from each other.

The second electron transport layer may have a thickness of < 100 nm, optionally between 10 and 90 nm, optionally between 10 and 60nm, optionally between 10 and 50 nm.

Further possible characteristics of the OLED

The organic light emitting diode according to the invention comprises at least two emission layers, namely the first emission layer and the second emission layer. The organic light-emitting diode may, in addition, comprise further emission layers (third emission layer, fourth emission layer etc.). In case that the organic light-emitting diode comprises more than two emission layers, only one electron transport layer stack may be provided only between two of the emission layers. Alternatively, more than one electron transport layer stack may present. For example, in case that the organic light-emitting diode comprises a first emission layer, a second emission layer and a third emission layer, a first electron transport layer stack may be arranged between the first emission layer and the second emission layer and a second electron transport layer stack may be arranged between the second emission layer and the third emission layer.

In case that the organic light-emitting diode comprises more than two emission layers more than one charge generation layers may present. For example, in case that the organic light-emitting diode comprises a first emission layer, a second emission layer and a third emission layer, a first charge generation layer may be arranged between the first emission layer and the second emission layer and a second charge generation layer may be arranged between the second emission layer and the third emission layer.

In terms of the present disclosure, a layer stack is an arrangement of two or more distinct layers. The layers of the layer stack may be distinguished from each other be the chemical nature of the materials comprised in the respective layers, that is, may be made of different compounds. An electron transport layer stack in terms of the present disclosure comprises at least two different layers made of electron transport materials, respectively.

The compound of Formula (I) and the compound of Formula (II) may be different from each other. That is, that the compounds of Formula (I) and the compound of Formula (II) may differ from each other with respect to at least one structural aspect from each other, in particular may differ from each other by at least one atom and/or group.

The first electron transport layer and the second electron transport layer are free of an electrical dopant. In this regard, "free of" means that respective compounds (electrical dopants) are only contained in the respective layers which cannot be avoided by standard purification methods and common technical means during preparation of the respective layer. In this regards, electrical dopants are in particular, but not limited thereto, electrical n-dopants. The electrical n-dopant may be selected from a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof. In particular, the first electron transport layer and the second electron transport layer may be free of an electrical n-dopant. The electrical n-dopant may be a metal salt comprising at least one metal cation and at least one anion. The metal cation of the metal salt may be selected from the group consisting of alkali metals, alkaline earth metals, and rare earth metals, alternatively from the group consisting of Li, Na, K, Rb, Cs, Mg, Ca, Sr, and Ba; alternatively from Li, Mg, Ca, and Sr. The anion of the metal salt may be selected from the group consisting of quinolinolate, phosphine oxide phenolate and borate.

In this regard, electrical n-dopants are in particular, but not limited thereto, an elemental metal, alternatively an electropositive metal selected from alkali metals, alkaline earth metals, rare earth metals and transition metals, transition metals; a metal salt, alternatively an alkali metal salt, alkaline earth metal salt and/or rare earth metal salt, or a metal complex, alternatively an alkali metal complex, alkaline earth metal complex, transition metal complex and/or rare earth metal complex. Examples of n-doping metal salts can be LiF, LiCl, LiBr, LiI, metal borates, metal quinolinolates or mixtures thereof. Further examples of electrical n-dopants are strong chemical reducing agents. This class of "redox" n-dopants may be generically characterized by energy level of the highest occupied molecular orbital (HOMO) comparable with lowest unoccupied molecular orbital Energy Level of corresponding electron transport matrices, which is in usual OLED transport materials about -3.0 eV or less. It is to be understood that the term "about -3.0 eV or less" means less

negative values than -3.0 eV, for example -2.8 eV, -2.5 eV, -2.3 eV, -2.1 eV or vales less negative than -2.0 eV.

Electrical n-dopants may be organic compounds as disclosed in EP1837926A1, WO07107306A1 or WO07107356A1.

It is provided that the electrical dopant is essentially non-emissive.

The first electron transport layer and the second electron transport layer may be in direct contact with each other.

The electron transport layer stack may consist of the first electron transport layer and the second electron transport layer.

The second electron transport layer may be in direct contact with the electron injection layer.

The electron injection layer may consist of a number of individual electron injection sublayers.

The electron injection layer may comprise a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof.

The electron injection layer may consist of a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof.

It maybe provided that the compound of Formula (II) is not comprised in the electron injection layer. It may be provided that the compound of Formula (I) is not comprised in the electron injection layer. It may be provided that compound (III) is not comprised in the electron injection layer.

The compound of Formula (I), the compound of Formula (II) and the compound (III) may be different from each other and/or may not be comprised in the electron injection layer, respectively.

The first electron transport layer stack may be arranged between the first emission layer and the first charge generation layer. The second electron transport layer of the first electron transport layer stack may be in direct contact with the first charge generation layer.

The first electron transport layer and the second electron transport layer may be in direct contact with each other.

The electron transport layer stack may consist of the first electron transport layer and the second electron transport layer.

The charge generation layer may comprise a p-type sublayer and a n-type sublayer and the second electron transport layer may be in direct contact with the n-type sublayer.

The first charge generation layer may comprise a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof.

The charge generation layer may comprise a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof in the n-type sublayer thereof.

The first charge generation layer may consist of a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof.

The n-type sublayer of the first charge generation layer may consist of a metal, alternatively an alkali metal, a metal salt alternatively an alkaline earth metal salt and/or rare earth metal salt, or an organic alkali metal complex, alternatively an alkali metal complex, alternatively LiF, LiCl, LiBr, LiI, LiQ, a metal borate, or mixtures thereof.

The organic light emitting diode may further comprise an electron injection layer and a second electron layer stack and the second electron layer stack is in direct contact with the electron injection layer. The second electron transport layer stack may contain the same compounds (I), (II), and (III) as the first layer stack, that is, as defined in the present disclosure, wherein the respective compounds may be selected independently.

It may be provided that the compound of Formula (II) is not comprised in the first charge generation layer. It may be provided that the compound of Formula (I) is not comprised in the first charge generation layer. It may be provided that compound (III) is not comprised in the first charge generation layer.

The compound of Formula (I), the compound of Formula (II) and the compound (III) may be different from each other and/or may not be comprised in the first charge generation layer, respectively.

In case that the organic light emitting device comprises more than one electron transport layer stack (that is, other electron transport layer stacks besides the first electron transport layer stack) all of the above features regarding the first electron transport layer stack may independently apply for each of the electron transport layer stacks.

In case that the organic light emitting device comprises more than one charge generation layers (that is, other charge generation layers besides the first charge generation layer) all of the above features regarding the first charge generation layer may independently apply for each of the charge generation layers.

The organic light emitting diode may further comprise a substrate, wherein the substrate may be transparent or non-transparent.

Exemplary embodiments

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$$(Ar^1\text{-}A_c)_a\text{-}X_b \qquad (I);$$

  - a and b are independently 1 or 2;
  - c is independently 0 or 1;

- $Ar^1$ is independently selected from $C_6$ to $C_{30}$ aryl or $C_2$ to $C_{24}$ heteroaryl,
- wherein each $Ar^1$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- wherein each $C_6$ to $C_{12}$ aryl substituent on $Ar^1$ and each $C_3$ to $C_{11}$ heteroaryl substituent on $Ar^1$ may be substituted with $C_1$ to $C_4$ alkyl or halogen;
- A is independently selected from $C_6$ to $C_{18}$ aryl,
- wherein each A may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- wherein each $C_6$ to $C_{12}$ aryl substituent on A may be substituted with $C_1$ to $C_4$ alkyl or halogen;
- X is independently selected from the group consisting of $C_3$ to $C_{21}$ heteroaryl,
- wherein each X may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- wherein each $C_6$ to $C_{12}$ aryl substituent on X and each $C_3$ to $C_{11}$ heteroaryl substituent on X may be substituted with $C_1$ to $C_4$ alkyl or halogen;

- the molecular dipole moment of the compound of formula (I) is $\geq$ 0 D and $\leq$ 3.5 D;

- the second electron transport layer comprises a compound of Formula (II)

$$(Ar^2)_m\text{-}(Z_k\text{-}G)_n \quad (II);$$

- m and n are independently 1 or 2;

- k is independently 0, 1 or 2;

- $Ar^2$ is independently selected from the group consisting of $C_3$ to $C_{30}$ heteroaryl and $C_6$ to $C_{42}$ aryl,

- wherein each $Ar^2$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;

- Z is independently selected from $C_6$ to $C_{18}$ aryl,

- wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on Z may be substituted with $C_1$ to $C_4$ alkyl or halogen;

- G is chosen so that the dipole moment of a compound G-phenyl is $\geq$ 2 D and $\leq$ 6 D; and

- the first electron transport layer and the second electron transport layer are free of an electrical dopant; and

- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;

- the first electron transport layer stack is arranged between the first emission layer and the second emission layer;

- the first electron transport layer stack comprises a first electron transport layer and a second electron transport layer;

- the first electron transport layer comprises a compound of Formula (I)

$$(Ar^1\text{-}A_c)_a\text{-}X_b \quad (I);$$

- a and b are independently 1 or 2;

- c is independently 0 or 1;

- $Ar^1$ is independently selected from $C_6$ to $C_{30}$ aryl or $C_2$ to $C_{24}$ heteroaryl,
- wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;
- A is independently selected from $C_6$ to $C_{18}$ aryl,
- wherein each A may be substituted with one or two substituents independently selected from the group consisting of phenyl and $C_1$ to $C_4$ alkyl;
- X is independently selected from the group consisting of $C_3$ to $C_{21}$ N-containing heteroaryl and $C_3$ to $C_{21}$ O-containing heteroaryl,
- wherein each X may be substituted with one or two substituents independently selected from the group consisting of phenyl, naphthyl and biphenyl-yl;
- the molecular dipole moment of the compound of formula (I) is ≥ 0.2 D and ≤ 3.5 D;
- the second electron transport layer comprises a compound of Formula (II)

$$(Ar^2)_m\text{-}(Z_k\text{-}G)_n \qquad \text{(II);}$$

- m and n are independently 1 or 2;
- k is independently o, 1 or 2;

- $Ar^2$ is independently selected from the group consisting of $C_3$ to $C_{21}$ heteroaryl and $C_6$ to $C_{24}$ aryl,
- wherein each $Ar^2$ may be substituted with one or two substituents independently selected from the group consisting of phenyl, pyridinyl and biphenyl-yl, optionally para-biphenyl-yl.;
- Z is independently selected from $C_6$ to $C_{18}$ aryl,
- wherein each Z may be substituted with one or two substituents independently selected from the group consisting of phenyl and $C_1$ to $C_4$ alkyl;
- G is selected from the group consisting of dialkylphosphinyl, diarylphosphinyl, alkylarylphosphinyl, nitrile, benzonitrile, nicotinonitrile, amide, carbamide, and $C_2$ to $C_{42}$ heteroaryl; wherein G may include one or more substituents attached to the group, wherein the one or more substituents are selected from the group consisting of $C_6$ to $C_{18}$ aryl, $C_1$ to $C_{10}$ alkyl, $C_2$ to $C_{14}$ heteroaryl; and
- the first electron transport layer and the second electron transport layer are free of an electrical dopant; and
- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$(Ar^1\text{-}A_c)_a\text{-}X_b$ (I);

- a and b are independently 1 or 2;
- c is independently 0 or 1;

- $Ar^1$ is independently selected from the group consisting of phenyl, naphthyl, anthracenyl, fluoranthenyl, xanthenyl, dibenzofuranyl, pyrimidinyl, pyrazinyl, spiro-xanthenyl, fluorenyl, spiro-fluorenyl, triphenylsilyl, tetraphenylsilyl, a group having the formula (IIa) and a group having the formula (IIb)

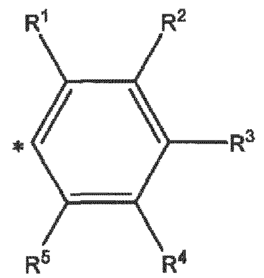

(IIa)

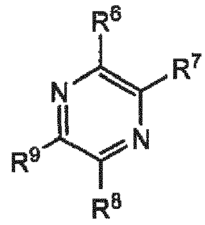

(IIb)

wherein

- the asterisk symbol "*" represents the binding position for binding the group of formula (IIa) to A; and
- $R^1$ to $R^9$ are independently selected from the group consisting of H, $C_6$ to $C_{12}$ aryl and $C_4$ to $C_{10}$ heteroaryl,
  - wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;
  - A is independently selected from phenylene, naphthylene, biphenylene and terphenylene,
  - wherein each A may be substituted with one or two substituents independently selected from the group consisting of phenyl and $C_1$ to $C_4$ alkyl;
  - X is independently selected from the group consisting of $C_3$ to $C_{21}$ N-containing heteroaryl,
  - wherein each X may be substituted with one or two substituents independently selected from the group consisting of phenyl, naphthyl and biphenyl-yl;
  - the molecular dipole moment of the compound of formula (I) is $\geq$ 0.4 D and $\leq$ 3.2 D;
  - the second electron transport layer comprises a compound of Formula (II)

$(Ar^2)_m\text{-}(Z_k\text{-}G)_n$ (II);

  - m and n are independently 1 or 2;
  - k is independently 1 or 2;
- $Ar^2$ is independently selected from the group consisting of $C_3$ to $C_{21}$ N-containing heteroaryl and $C_6$ to $C_{24}$ aryl,
- wherein each $Ar^2$ may be substituted with one or two substituents independently selected from the group consisting of phenyl, pyridinyl and biphenyl-yl, optionally para-biphenyl-yl.;
- Z is independently selected from the group consisting of phenylene, naphthylene, phenylene-naphthylene, biphenylene and terphenylene,

- wherein each Z may be substituted with one or two substituents independently selected from the group consisting of phenyl and $C_1$ to $C_4$ alkyl;
- G is selected from the group consisting of $C_2$ to $C_{25}$ heteroaryl; G may include one or more substituents attached to the group, wherein the one or more substituents are selected from the group consisting of $C_6$ to $C_{12}$ aryl, $C_1$ to $C_6$ alkyl, $C_2$ to $C_{11}$ heteroaryl; and
- the first electron transport layer and the second electron transport layer are free of an electrical dopant; and
- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$$(Ar^1\text{-}A_c)_a\text{-}X_b \qquad \text{(I);}$$

  - a and b are independently 1 or 2;
  - c is independently 0 or 1;

- $Ar^1$ is independently selected from the group consisting of fluoranthenyl, dibenzofuranyl, pyrimidinyl pyrazinyl, , 9,9-dimethylfluorenyl, a group having the formula (IIa), a group having the formula (IIb),

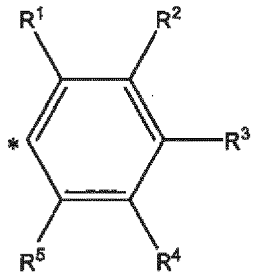
(IIa)

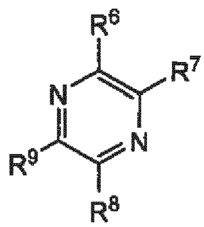
(IIb)

wherein

- the asterisk symbol "*" represents the binding position for binding the group of formula (IIa) to A; and
- $R^1$ is H and $R^2$ to $R^5$ are independently phenyl; or
- $R^1$ and $R^3$ are phenyl and $R^2$, $R^4$ and $R^5$ are H or
- $R^6$ to $R^9$ are phenyl,
  - wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;

- A is independently selected from phenylene, naphthylene, biphenylene and terphenylene,
- wherein each A may be substituted with one or two substituents independently selected from the group consisting of phenyl and $C_1$ to $C_4$ alkyl;
- X is independently selected from the group consisting of pyridazinyl, pyrimidinyl, pyrazinyl, quinazolinyl, benzoquinazolinyl, benzimidazolyl, quinolinyl, benzoacridinyl, dibenzoacridinyl, phenathrolinyl, and dinaphthofuranyl which may be substituted or unsubstituted, respectively,
- wherein each X may be substituted with one or two substituents independently selected from the group consisting of phenyl, naphthyl and biphenyl-yl;
- the molecular dipole moment of the compound of formula (I) is ≥ 0.4 D and ≤ 3.2 D;
- the second electron transport layer comprises a compound of Formula (II)

$$(Ar^2)_m\text{-}(Z_k\text{-}G)_n \qquad \text{(II)};$$

  - m and n are independently 1 or 2;
  - k is independently 1 or 2;

- $Ar^2$ is independently selected from the group consisting of pyridinyl, triazinyl, 1,2-diazinyl, 1,3-diazinyl, 1,4-diazinyl, quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl,
- wherein each $Ar^2$ may be substituted with one or two substituents independently selected from the group consisting of phenyl, pyridinyl and biphenyl-yl, optionally para-biphenyl-yl.;
- Z is independently selected from the group consisting of phenylene, naphthylene, phenylene-naphthylene, biphenylene and terphenylene,
- wherein each Z may be substituted with one or two substituents independently selected from the group consisting of phenyl and $C_1$ to $C_4$ alkyl;
- G is selected from the group consisting of di-$C_1$ to $C_4$-alkylphosphinyl, di-$C_6$ to $C_{10}$-arylphosphinyl, and $C_2$ to $C_{25}$ heteroaryl; wherein the respective G may include one or more substituents attached to the group, wherein the one or more substituents are selected from the group consisting of $C_6$ to $C_{10}$ aryl, $C_1$ to $C_4$ alkyl, $C_2$ to $C_5$ heteroaryl; and
- the first electron transport layer and the second electron transport layer are free of an electrical dopant;
- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$$(Ar^1\text{-}A_c)_a\text{-}X_b, \qquad \text{(I)};$$

  - a and b are independently 1 or 2;

- c is independently 0 or 1;

- $Ar^1$ is independently selected from the group consisting of fluoranthenyl, dibenzofuranyl, pyrimidinyl pyrazinyl, 9,9-dimethylfluorenyl, a group having the formula (IIa), a group having the formula (IIb),

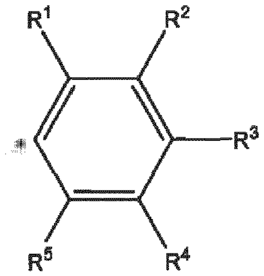

(IIa)

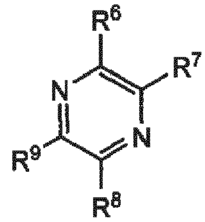

(IIb)

wherein

- the asterisk symbol "*" represents the binding position for binding the group of formula (IIa) to A; and

- $R^1$ is H and $R^2$ to $R^5$ are independently phenyl; or

- $R^1$ and $R^3$ are phenyl and $R^2$, $R^4$ and $R^5$ are H or

- $R^6$ to $R^9$ are phenyl;

  - wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;

  - A is independently selected from phenylene and biphenylene;

  - wherein each A may be substituted with one or two substituents independently selected from the group consisting of phenyl and $C_1$ to $C_4$ alkyl;

  - X is independently selected from the group consisting of pyrimidinyl, pyrazinyl, quinazolinyl, benzoquinazolinyl, benzoacridinyl, dibenzoacridinyl, which may be substituted or unsubstituted, respectively.,

  - wherein each X may be substituted with one or two substituents independently selected from the group consisting of phenyl, naphthyl and biphenyl-yl;

  - the molecular dipole moment of the compound of formula (I) is ≥ 0.4 D and ≤ 3.2 D;

  - the second electron transport layer comprises a compound of Formula (II)

$$(Ar^2)_m\text{-}(Z_k\text{-}G)_n \qquad \text{(II);}$$

    - m and n are independently 1 or 2;
    - k is independently 1 or 2;

- $Ar^2$ is independently selected from the group consisting of dibenzoacridinyl, 1,3-diazinyl, 1,4-diazinyl, anthracenyl, triazinyl, phenathrolinyl, triphenylenyl, pyridinyl, dinaphthofuranyl,

- wherein each $Ar^2$ may be substituted with one or two substituents independently selected from the group consisting of phenyl, pyridinyl and biphenyl-yl, optionally para-biphenyl-yl.;

- Z is independently selected from the group consisting of phenylene, naphthylene, phenylene-naphthylene, biphenylene and terphenylene,

- wherein each Z may be substituted with one or two substituents independently selected from the group consisting of phenyl and $C_1$ to $C_4$ alkyl;

- G is selected from the group consisting of dimethylphosphinyl, diphenylphosphinyl, nitrile, benzonitrile, nicotinonitrile, di-hydro-benzoimidazolone-yl, diphenyl-propane-yl, *N,N*-dimethylacetamid, amide, carbamide, imidazolyl, phenylbenzoimidazolyl, ethylbenzoimidazolyl phenylbenzoquinolinyl, phenylbenzoimidazoquinolinyl, pyridinyl, bipyridinyl, picolinyl, lutidenyl, pyridazinyl, pyrimidinyl, pyrazinyl, triphenyl-pyrazinyl, benzoquinolinyl, phenanthrolinyl, phenylphenanthrolinyl and pyridinylimidazopyridinyl; and

- the first electron transport layer and the second electron transport layer are free of an electrical dopant;

- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;

- the first electron transport layer stack is arranged between the first emission layer and the second emission layer;

- the first electron transport layer stack comprises a first electron transport layer and a second electron transport layer;

- the first electron transport layer comprises a compound selected from the compounds A-1 to A-12;

- the second electron transport layer comprises a compound selected from the compounds B-1 to B-25 shown in Table 2;

- the first electron transport layer and the second electron transport layer are free of an electrical dopant; and

- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;

- the first electron transport layer stack is arranged between the first emission layer and the second emission layer;

- the first electron transport layer stack comprises a first electron transport layer and a second electron transport layer;

- the first electron transport layer comprises a compound of Formula (I)

$$(Ar^1\text{-}A_c)_a\text{-}X_b, \qquad (I);$$

  - a and b are independently 1 or 2;

  - c is independently 0 or 1;

- $Ar^1$ is independently selected from $C_6$ to $C_{30}$ aryl or $C_2$ to $C_{24}$ heteroaryl,

- wherein each $Ar^1$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $FY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl,

partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on $Ar^1$ and each $C_3$ to $C_{11}$ heteroaryl substituent on $Ar^1$ may be substituted with $C_1$ to $C_4$ alkyl or halogen;

- A is independently selected from $C_6$ to $C_{18}$ aryl,

- wherein each A may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $FY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on A may be substituted with $C_1$ to $C_4$ alkyl or halogen;

- X is independently selected from the group consisting of $C_3$ to $C_{21}$ heteroaryl,

- wherein each X may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on X and each $C_3$ to $C_{11}$ heteroaryl substituent on X may be substituted with $C_1$ to $C_4$ alkyl or halogen;

  - the molecular dipole moment of the compound of formula (I) is $\geq 0$ D and $\leq 3.5$ D;

- the second electron transport layer comprises a compound of Formula (II)

$$(Ar^2)_m\text{-}(Z_k\text{-}G)_n \qquad \text{(II)};$$

  - m and n are independently 1 or 2;

  - k is independently 0, 1 or 2;

- $Ar^2$ is independently selected from the group consisting of $C_3$ to $C_{30}$ heteroaryl and $C_6$ to $C_{42}$ aryl,

- wherein each $Ar^2$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;

- Z is independently selected from $C_6$ to $C_{18}$ aryl,

- wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on Z may be substituted with $C_1$ to $C_4$ alkyl or halogen;

- G is chosen so that the dipole moment of a compound G-phenyl is $\geq 2$ D and $\leq 6$ D;

- the second electron transport layer further comprises a compound (III), wherein the compound (III) comprises 8 to 13 aromatic or heteroaromatic rings;

- the first electron transport layer and the second electron transport layer are free of an electrical dopant; and

- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;

- the first electron transport layer stack is arranged between the first emission layer and the second emission layer;

- the first electron transport layer stack comprises a first electron transport layer and a second electron transport layer;

- the first electron transport layer comprises a compound of Formula (I)

$$(Ar^1\text{-}A_c)_a\text{-}X_b \qquad \text{(I);}$$

  - a and b are independently 1 or 2;
  - c is independently 0 or 1;

- $Ar^1$ is independently selected from $C_6$ to $C_{30}$ aryl or $C_2$ to $C_{24}$ heteroaryl,

- wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;

- A is independently selected from $C_6$ to $C_{18}$ aryl,

- wherein each A may be substituted with one or two substituents independently selected from the group consisting of phenyl and $C_1$ to $C_4$ alkyl;

- X is independently selected from the group consisting of $C_3$ to $C_{21}$ N-containing heteroaryl and $C_3$ to $C_{21}$ O-containing heteroaryl,

- wherein each X may be substituted with one or two substituents independently selected from the group consisting of phenyl, naphthyl and biphenyl-yl;

- the molecular dipole moment of the compound of formula (I) is $\geq 0.2$ D and $\leq 3.5$ D;

- the second electron transport layer comprises a compound of Formula (II)

$$(Ar^2)_m\text{-}(Z_k\text{-}G)_n \qquad \text{(II);}$$

- m and n are independently 1 or 2;
- k is independently 0, 1 or 2;

- $Ar^2$ is independently selected from the group consisting of $C_3$ to $C_{21}$ heteroaryl and $C_6$ to $C_{24}$ aryl,
- wherein each $Ar^2$ may be substituted with one or two substituents independently selected from the group consisting of phenyl, pyridinyl and biphenyl-yl, optionally para-biphenyl-yl.;
- Z is independently selected from $C_6$ to $C_{18}$ aryl,
- wherein each Z may be substituted with one or two substituents independently selected from the group consisting of phenyl and $C_1$ to $C_4$ alkyl;
- G is selected from the group consisting of dialkylphosphinyl, diarylphosphinyl, alkylarylphosphinyl, nitrile, benzonitrile, nicotinonitrile, amide, carbamide, and $C_2$ to $C_{42}$ heteroaryl; wherein G may include one or more substituents attached to the group, wherein the one or more substituents are selected from the group consisting of $C_6$ to $C_{18}$ aryl, $C_1$ to $C_{10}$ alkyl, $C_2$ to $C_{14}$ heteroaryl;
- the second electron transport layer may further comprises a compound (III), wherein the compound (III) comprises 8 to 13 aromatic or heteroaromatic rings;
- compound (III) comprises at least one heteroaromatic ring;
- the first electron transport layer and the second electron transport layer are free of an electrical dopant; and
- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$$(Ar^{1}\text{-}A_c)_a\text{-}X_b \qquad (I);$$

  - a and b are independently 1 or 2;
  - c is independently 0 or 1;

- $Ar^1$ is independently selected from the group consisting of phenyl, naphthyl, anthracenyl, fluoranthenyl, xanthenyl, dibenzofuranyl, pyrimidinyl, pyrazinyl, spiro-xanthenyl, fluorenyl, spiro-fluorenyl, triphenylsilyl, tetraphenylsilyl, a group having the formula (IIa) and a group having the formula (IIb)

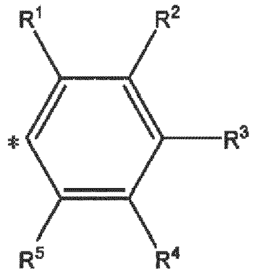

(IIa)

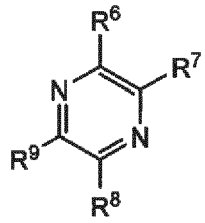

(IIb)

wherein

- the asterisk symbol "*" represents the binding position for binding the group of formula (IIa) to A; and
- $R^1$ to $R^9$ are independently selected from the group consisting of H, $C_6$ to $C_{12}$ aryl and $C_4$ to $C_{10}$ heterorayl,
  - wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;
  - A is independently selected from phenylene, naphthylene, biphenylene and terphenylene,
  - wherein each A may be substituted with one or two substituents independently selected from the group consisting of phenyl and $C_1$ to $C_4$ alkyl;
  - X is independently selected from the group consisting of $C_3$ to $C_{21}$ N-containing heteroaryl,
  - wherein each X may be substituted with one or two substituents independently selected from the group consisting of phenyl, naphthyl and biphenyl-yl;
  - the molecular dipole moment of the compound of formula (I) is ≥ 0.4 D and ≤ 3.2 D;
  - the second electron transport layer comprises a compound of Formula (II)

$$(Ar^2)_m\text{-}(Z_k\text{-}G)_n \qquad (II);$$

    - m and n are independently 1 or 2;
    - k is independently 1 or 2;
  - $Ar^2$ is independently selected from the group consisting of $C_3$ to $C_{21}$ N-containing heteroaryl and $C_6$ to $C_{24}$ aryl,
  - wherein each $Ar^2$ may be substituted with one or two substituents independently selected from the group consisting of phenyl, pyridinyl and biphenyl-yl, optionally para-biphenyl-yl.;
  - Z is independently selected from the group consisting of phenylene, naphthylene, phenylene-naphthylene, biphenylene and terphenylene,
  - wherein each Z may be substituted with one or two substituents independently selected from the group consisting of phenyl and $C_1$ to $C_4$ alkyl;
  - G is selected from the group consisting of $C_2$ to $C_{25}$ heteroaryl; G may include one or more substituents attached to the group, wherein the one or more substituents are selected from the group consisting of $C_6$ to $C_{12}$ aryl, $C_1$ to $C_6$ alkyl, $C_2$ to $C_{11}$ heteroaryl;
  - the second electron transport layer may further comprises a compound (III), wherein the compound (III) comprises 8 to 13 aromatic or heteroaromatic rings;

- compound (III) comprises at least one heteroaromatic ring;

- the aromatic or heteroaromatic rings of the compound (III) are 6-membered rings;

- the first electron transport layer and the second electron transport layer are free of an electrical dopant; and

- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;

- the first electron transport layer stack is arranged between the first emission layer and the second emission layer;

- the first electron transport layer stack comprises a first electron transport layer and a second electron transport layer;

- the first electron transport layer comprises a compound of Formula (I)

$$(Ar^1\text{-}A_c)_a\text{-}X_b \qquad (I);$$

- a and b are independently 1 or 2;

- c is independently 0 or 1;

- $Ar^1$ is independently selected from the group consisting of fluoranthenyl, dibenzofuranyl, pyrimidinyl pyrazinyl, 9,9-dimethylfluorenyl, a group having the formula (IIa), a group having the formula (IIb),

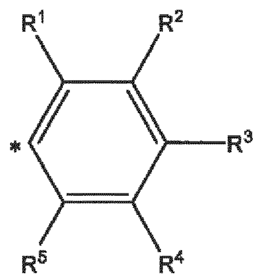
(IIa)

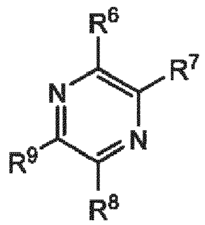
(IIb)

wherein

- the asterisk symbol "*" represents the binding position for binding the group of formula (IIa) to A; and

- $R^1$ is H and $R^2$ to $R^5$ are independently phenyl; or

- $R^1$ and $R^3$ are phenyl and $R^2$, $R^4$ and $R^5$ are H or

- $R^6$ to $R^9$ are phenyl,

  - wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;

  - A is independently selected from phenylene, naphthylene, biphenylene and terphenylene,

  - wherein each A may be substituted with one or two substituents independently selected from the group consisting of phenyl and $C_1$ to $C_4$ alkyl;

- X is independently selected from the group consisting of pyridazinyl, pyrimidinyl, pyrazinyl, quinazolinyl, benzoquinazolinyl, benzimidazolyl, quinolinyl, benzoacridinyl, dibenzoacridinyl, phenathrolinyl, and dinaphthofuranyl which may be substituted or unsubstituted, respectively,
- wherein each X may be substituted with one or two substituents independently selected from the group consisting of phenyl, naphthyl and biphenyl-yl;
- the molecular dipole moment of the compound of formula (I) is ≥ 0.4 D and ≤ 3.2 D;
- the second electron transport layer comprises a compound of Formula (II)

$$(Ar^2)_m\text{-}(Z_k\text{-}G)_n \qquad (II);$$

  - m and n are independently 1 or 2;
  - k is independently 1 or 2;
- $Ar^2$ is independently selected from the group consisting of pyridinyl, triazinyl, 1,2-diazinyl, 1,3-diazinyl, 1,4-diazinyl, quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl,
- wherein each $Ar^2$ may be substituted with one or two substituents independently selected from the group consisting of phenyl, pyridinyl and biphenyl-yl, optionally para-biphenyl-yl.;
- Z is independently selected from the group consisting of phenylene, naphthylene, phenylene-naphthylene, biphenylene and terphenylene,
- wherein each Z may be substituted with one or two substituents independently selected from the group consisting of phenyl and $C_1$ to $C_4$ alkyl;
- G is selected from the group consisting of di-$C_1$ to $C_4$-alkylphosphinyl, di-$C_6$ to $C_{10}$-arylphosphinyl, and $C_2$ to $C_{25}$ heteroaryl; wherein the respective G may include one or more substituents attached to the group, wherein the one or more substituents are selected from the group consisting of $C_6$ to $C_{10}$ aryl, $C_1$ to $C_4$ alkyl, $C_2$ to $C_5$ heteroaryl;
- the second electron transport layer may further comprises a compound (III), wherein the compound (III) comprises 8 to 13 aromatic or heteroaromatic rings;
- compound (III) comprises at least one heteroaromatic ring;
- the aromatic or heteroaromatic rings of the compound (III) are 6-membered rings;
- the heteroaromatic rings of the compound (III) are N-containing heteroaromatic rings;
- the first electron transport layer and the second electron transport layer are free of an electrical dopant;
- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;

- the first electron transport layer comprises a compound of Formula (I)

$$(Ar^1\text{-}A_c)_a\text{-}X_b \qquad (I);$$

  - a and b are independently 1 or 2;
  - c is independently 0 or 1;

- $Ar^1$ is independently selected from the group consisting of fluoranthenyl, dibenzofuranyl, pyrimidinyl pyrazinyl, , 9,9-dimethylfluorenyl, a group having the formula (IIa), a group having the formula (IIb),

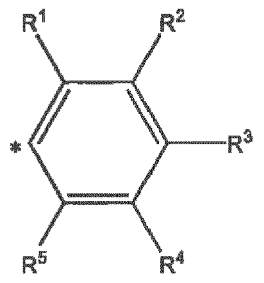

(IIa)

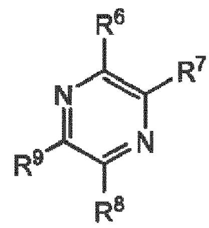

(IIb)

wherein

- the asterisk symbol "*" represents the binding position for binding the group of formula (IIa) to A; and
- $R^1$ is H and $R^2$ to $R^5$ are independently phenyl; or
- $R^1$ and $R^3$ are phenyl and $R^2$, $R^4$ and $R^5$ are H or
- $R^6$ to $R^9$ are phenyl;
  - wherein each $Ar^1$ may be substituted with one or two substituents independently selected from the group consisting of phenyl, naphtyl, biphenyl, pyridyl, picolinyl, lutidinyl, dibenzofuranyl, dibenzothiophene-yl, and benzothiophene-yl;
  - A is independently selected from phenylene and biphenylene;
  - wherein each A may be substituted with one or two substituents independently selected from the group consisting of phenyl and $C_1$ to $C_4$ alkyl;
  - X is independently selected from the group consisting of pyrimidinyl, pyrazinyl, quinazolinyl, benzoquinazolinyl, benzoacridinyl, dibenzoacridinyl, which may be substituted or unsubstituted, respectively,
  - wherein each X may be substituted with one or two substituents independently selected from the group consisting of phenyl, naphthyl and biphenyl-yl;
  - the molecular dipole moment of the compound of formula (I) is $\geq$ 0.4 D and $\leq$ 3.2 D;
  - the second electron transport layer comprises a compound of Formula (II)

$$(Ar^2)_m\text{-}(Z_k\text{-}G)_n \qquad (II);$$

    - m and n are independently 1 or 2;
    - k is independently 1 or 2;

- $Ar^2$ is independently selected from the group consisting of dibenzoacridinyl, 1,3-diazinyl, 1,4-diazinyl, anthracenyl,

triazinyl, phenathrolinyl, triphenylenyl, pyridinyl, dinaphthofuranyl,

- wherein each $Ar^2$ may be substituted with one or two substituents independently selected from the group consisting of phenyl, pyridinyl and biphenyl-yl, optionally para-biphenyl-yl.;

- Z is independently selected from the group consisting of phenylene, naphthylene, phenylene-naphthylene, biphenylene and terphenylene,

- wherein each Z may be substituted with one or two substituents independently selected from the group consisting of phenyl and $C_1$ to $C_4$ alkyl;

- G is selected from the group consisting of dimethylphosphinyl, diphenylphosphinyl, nitrile, benzonitrile, nicotinonitrile, di-hydro-benzoimidazolone-yl, diphenyl-propane-yl, methylacetamid, amide, carbamide, imidazolyl, phenylbenzoimidazolyl, ethylbenzoimidazolyl phenylbenzoquinolinyl, phenylbenzoimidazoquinolinyl, pyridinyl, bipyridinyl, picolinyl, lutidenyl, pyridazinyl, pyrimidinyl, pyrazinyl, triphenyl-pyrazinyl, benzoquinolinyl, phenanthrolinyl, phenylphenanthrolinyl and pyridinylimidazopyridinyl;

- the second electron transport layer may further comprises a compound (III), wherein the compound (III) comprises 8 to 13 aromatic or heteroaromatic rings;

- compound (III) comprises at least one heteroaromatic ring;

- the aromatic or heteroaromatic rings of the compound (III) are 6-membered rings;

- the heteroaromatic rings of the compound (III) are N-containing heteroaromatic rings;

- the compound (III) may comprises at least one 6-membered heteroaromatic ring containing one to three N-atoms in each heteroaromatic ring;

- the first electron transport layer and the second electron transport layer are free of an electrical dopant;

- the compound of Formula (I) is free of 1,3,5-triazine.

According to one embodiment, there is provided an organic light emitting diode comprising an anode, a cathode, a first emission layer, a second emission layer, a first charge generation layer and a first electron transport layer stack; wherein

- the first charge generation layer is arranged between the first emission layer and the second emission layer;

- the first electron transport layer stack is arranged between the first emission layer and the second emission layer;

- the first electron transport layer stack comprises a first electron transport layer and a second electron transport layer;

- the first electron transport layer comprises a compound selected from the compounds A-1 to A-12;

- the second electron transport layer comprises a compound selected from the compounds B-1 to B-25 shown in Table 2;

- the and second electron transport layer further comprises a compound selected from the compounds C-1 to C-6 shown in Table 3;

- the first electron transport layer and the second electron transport layer are free of an electrical dopant; and

- the compound of Formula (I) is free of 1,3,5-triazine.

<u>Further layers</u>

In accordance with the invention, the organic electronic device may comprise, besides the layers already

mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys. The transparent or semitransparent anode may facilitate light emission through the anode.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys. The transparent or semitransparent anode may facilitate light emission through the anode.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane ($F_4TCNQ$), 2,2'-(perfluoronaphthalen-2,6-diylidene)-dimalononitrile, 4,4',4"-((1E,1'E,1"E)-cyclopropane-1,2,3-triylidenetris(cyanomethanylylidene))tris(2,3,5,6-tetrafluorobenzonitrile) or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations

can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-i-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formulas (I), (II) and/or the compound (III).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2Ir(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

*Electron transport layer (ETL)*

The OLED according to the present invention comprises at least two electron transport layers (ETLs). At least two of the electron transport layers are the first electron transport layer and the second electron transport layer as defined herein. In addition, the OLED may comprise further ETLs which may or may not be as defined above. If the additional ETL(s) is/are not as defined above, the characteristics thereof may be as follows.

According to various embodiments the OLED may comprise an electron transport layer stack comprising at least a first electron transport layer (ETL-1) comprising the compound of formula (I) and at least a second electron transport layer (ETL-2) comprising a compound of formula (II).

By suitably adjusting energy levels of particular layers of the ETL, the injection and transport of the electrons may be controlled, and the holes may be efficiently blocked. Thus, the OLED may have long lifetime, improved performance and stability.

Electron injection *layer (EIL)*

An EIL, which may facilitate injection of electrons from the cathode, optionally into a second electron transport layer stack, may be formed on the second electron transport layer stack, preferably directly on the second electron transport layer stack, preferably directly on the second electron transport layer of the second electron layer stack, preferably in direct contact with the second electron transport layer of the second electron layer stack. Examples of materials for forming the EIL or being comprised in the EIL include lithium 8-hydroxyquinolinolate (LiQ), LiF, NaCl, CsF, $Li_2O$, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may comprise an organic matrix material doped with an n-type dopant. The matrix material may be selected from materials conventionally used as matrix materials for electron transport layers.

The EIL may consist of a number of individual EIL sublayers. In case the EIL consists of a number of individual EIL sublayers, the number of sublayers is preferably 2. The individual EIL sublayers may comprise different materials for forming the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

The electron transport stack of the present invention is not part of the electron injection layer.

The compound of formula (I) may not be comprised in the electron injection layer.

*Cathode electrode*

The cathode electrode is formed on the EIL if present, preferably directly on the EIL, preferably in direct contact with the EIL. In the sense of this invention the cathode and the EIL can be regarded as one functional part enabling the injection of electrons into the electron transport layer stack. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy. The transparent or semitransparent cathode may facilitate light emission through the cathode.

It is to be understood that the cathode electrode and the electron injection layer are not part of the second electron transport layer or of any other part of the electron transport layer stack.

*Charge generation layer*

The charge generation layer (CGL), that is, the first CGL as well as any other CGL comprised in the inventive OLED, may comprise a p-type CGL and an n-type CGL. An interlayer may be arranged between the p-type layer and the n-type layer.

Typically, the charge generation layer GCL is a pn junction joining an n-type charge generation layer (electron generating layer, n-type CGL) and a-type charge generation layer (hole generating layer, p-type CGL). The n-side of the pn junction generates electrons and injects them into the layer which is adjacent in the direction to the anode. Analogously, the p-side of the pn junction generates holes and injects them into the layer which is adjacent in the direction to the cathode.

Charge generation layers are used in tandem OLEDs (such as that of the present disclosure) comprising, between the cathode and anode, two or more emission layers. In a tandem OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N’-di(naphthalen-1-yl)-N,N-diphenyl-benzidine (NPB), N,N’-diphenyl-N,N’-bis(3-methylphenyl)-1, 1-biphenyl-4,4’-diamine (TPD) and N,N’,N’-tetranaphthyl-benzidine (TNB). The p-type charge generation layer may consist of CNHAT.

The n-type charge generation layer may be layer of a neat n-type dopant, for example of an electropositive metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the n-type charge generation layer layer may be the materials conventionally used as matrix materials for electron injection or electron transport layers. The matrix material can be for example one selected from a group consisting of N-containing heterocyclic compounds like triazine compounds or phenathroline compounds such as compound E or bipyridyl or terpyridyl compounds, hydroxyquinoline derivatives like tris(8-hydroxyquinoline)aluminum, benzazole derivatives, and silole derivatives.

The hole generating layer may be arranged in direct contact to the n-type charge generation layer.

The electron transport stack of the present invention is not part of the charge generation layer.

It may be provided that the compound of formula (I) is not comprised in the charge generation layer.

<u>Organic light-emitting diode (OLED)</u>

According to various embodiments of the present invention, there may be provided OLEDs layers arranged between the above mentioned layers, on the substrate or on the top electrode.

According to one aspect of the present invention, there is provided an organic light-emitting diode comprising:

a substrate, an anode, a first hole injection layer, a first hole transport layer, a first electron blocking layer, a first emission layer, a first optional hole blocking layer, a first electron transport layer stack, an n-type charge generation layer, a hole generating layer, a second hole transport layer, a second electron blocking layer, a second emission layer, a second optional hole blocking layer, a second electron transport layer stack, a second electron injection layer and a cathode.

According to one aspect of the present invention, there is provided an organic light-emitting diode comprising: a substrate, an anode, a hole injection layer, a first hole transport layer, a first electron blocking layer, a first emission layer a first electron transport layer stack comprising a first electron transport layer and a second electron transport layer, a first charge generation layer, a second hole transport layer, a second electron blocking layer, a second emission layer, a second electron transport layer stack comprising a third electron transport layer (= first electron transport layer of the second stack) and a fourth electron transport layer (= second electron transport layer of the second stack), a second charge generation layer, a third hole transport layer, a third electron blocking layer, a third emission layer, a third electron transport layer stack comprising a fifth electron transport layer (= first electron transport layer of the third stack) and a sixth electron transport layer (= second electron transport layer of the third stack), an electron injection layer and a cathode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer stack comprising a first electron transport layer and a second electron transport layer, the first electron transport layer stack is adjacent arranged to an electron injection layer, the electron injection layer is adjacent arranged to the cathode electrode.

For example, the OLED according to Fig. 1 may be formed by a process, wherein on a substrate 110, an anode 120, a first hole injection layer 130, a first hole transport layer 140, a first electron blocking layer 145, a first emission layer 150, a first electron transport layer stack 160, an n-type charge generation layer 185, a hole generating layer 135, a second hole transport layer 141, a second electron blocking layer 146, a second emission layer 151, a second electron transport layer stack 165, a second electron injection layer 181 and a cathode 190 are subsequently formed in that order.

For example, the OLED according to Fig. 2 may be formed by a process, wherein on an anode 120, a hole injection layer 130, a first hole transport layer 140, a first electron blocking layer 145, a first emission layer 150, a first electron transport layer stack 160 comprising a first electron transport layer 161 and a second electron transport layer 162, a first charge generation layer 184 comprising a n-type CGL sublayer 184a and a p-type CGL sublayer 184b, a second hole transport layer 141, a second electron blocking layer 146, a second emission layer 151, a second electron transport layer stack 165 comprising a third electron transport layer (= first electron transport layer of the second stack) 166 and a fourth electron transport layer (= second electron transport layer of the second stack) 167, a second charge generation layer 186 comprising a n-type CGL sublayer 186a and a p-type CGL sublayer 186b, a third hole transport layer 142, a third electron blocking layer 147, a third emission layer 152, a third electron transport layer stack 168 comprising a fifth electron transport layer (= first electron transport layer of the third stack) 163 and a sixth electron transport layer (= second electron transport layer of the third stack) 164, an electron injection layer 181 and a cathode 190 are subsequently formed in that order.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;

- deposition via solution processing, preferably the processing is selected from spin-coating, printing, casting; and/or

- slot-die coating.

In case that (one or more of) the second electron transport layer(s) comprise(s) a compound (III) and a compound of formula (II) the two compounds may be deposited by co-deposition from two separate deposition sources or deposited as a pre-mix for one single source.

According to various embodiments of the present invention, the method may further include forming on the anode electrode, an emission layer and at least one layer selected from the group consisting of forming a hole injection layer, forming a hole transport layer, or forming an electron hole blocking layer, between the anode electrode and the

first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, and on the electron transport layer stack a charge generation layer is formed,
- and finally a cathode electrode is formed,
- optional a hole injection layer, a hole transport layer, formed in that order between the first anode electrode and the emission layer,
- an charge generation layer is formed between the electron transport layer stack and the cathode electrode.

According to various embodiments, the OLED may have the following layer structure, wherein the layers having the following order:
anode, hole injection layer, hole transport layer, electron blocking layer, emission layer, optional hole blocking layer, electron transport layer stack, n-type CGL, p-type CGL, hole transport layer, electron blocking layer, emission layer, optional hole blocking layer, electron transport layer stack, n-type CGL, p-type CGL, hole transport layer, electron blocking layer, emission layer, optional hole blocking layer, electron transport layer stack, electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device or a lighting device.

In one embodiment, the organic electronic device according to the invention may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

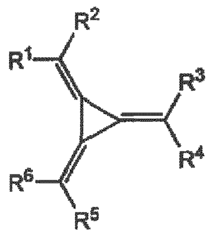
(XX)
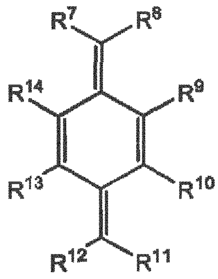
(XXIa)
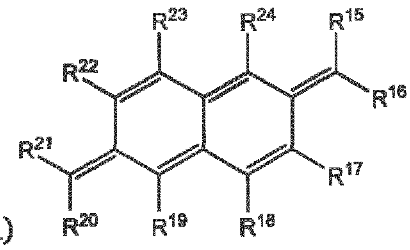
(XXIb),

wherein $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned

electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Hereinafter, the embodiments are illustrated in more detail with reference to examples. However, the present disclosure is not limited to the following examples. Reference will now be made in detail to the exemplary aspects.

Compound

According to one aspect of the invention, there is provided a compound of the following formula (IV)

$$(Ar'^1\text{-}A'_d)_eX'_f \qquad (IV)$$

wherein

- e and f are independently 1 or 2;
- d is independently 0 or 1;
- Ar'1 is independently selected from the group consisting of a group having the formula (IVa) and a group having the formula (IVb)

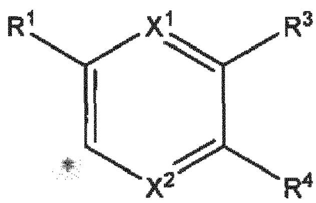

(IVa)

wherein in (IVa)

- the asterisk symbol "*" represents the binding position for binding the group of formula (IVa) to A';
- $X^1$ and $X^2$ are both N; or $X^1$ is $CR^2$ and $X^2$ is $CR^5$; and
- at least one of $R^1$ and $R^5$ is phenyl; and/or $R^1$ and $R^2$ are both phenyl; and/or $R^2$ and $R^3$ are both phenyl; and/or $R^3$ and $R^4$ are both phenyl; and/or $R^4$ and $R^5$ are both phenyl;

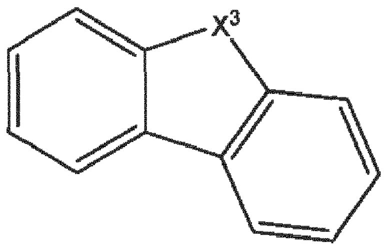

(VIb)

wherein in (IVb)

- the asterisk symbol "*" represents the binding position for binding the group of formula (IVb) to A' or, in case that d = 0 to X; and
- $X^3$ is selected from $C(CH_3)_2$, O or S;
- wherein each $Ar^1$ may be substituted with one or two phenyl, preferably one phenyl
- A' is independently selected from phenylene or biphenylene;

- X' is independently selected from pyrimidinyl, pyrazinyl, quinazolinyl, benzoquinazolinyl, benzoacridinyl and dibenzoacridinyl;

  wherein each pyrimidinyl, pyrazinyl, quinazolinyl, and benzoquinazolinyl, may be independently be substituted with one or more phenyl, preferably one or two phenyl;

  wherein it is provided that the following compounds are excluded from Formula (IV)

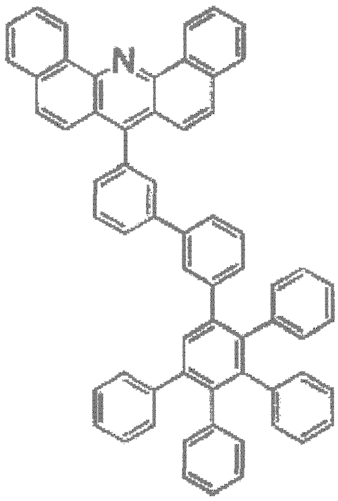

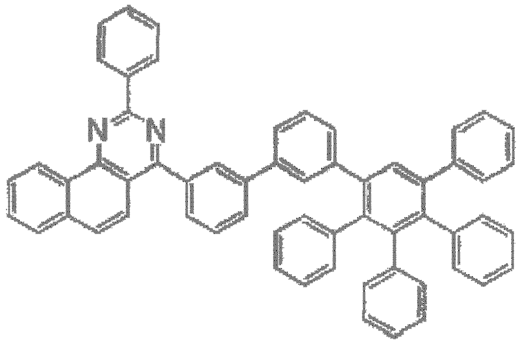

.

Ar$^1$ may be independently selected from the group consisting of a group having the formula (IVa) and a group having the formula (IVb)

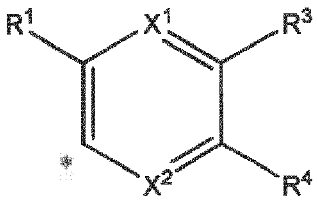

(IVa)

wherein in (IVa)

- the asterisk symbol "*" represents the binding position for binding the group of formula (IVa) to A' or, in case that d = 0 to X';
- $X^1$ is $CR^2$; $X^2$ is $CR^5$; $R^1$ is H and $R^2$ to $R^5$ are each phenyl; or
- $X^1$ is $CR^2$; $X^2$ is $CR^5$; $R^1$ and $R^3$ are phenyl and $R^2$, $R^4$ and $R^5$ are H;

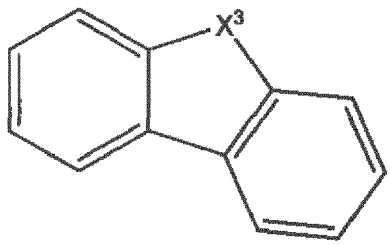

(VIb)

wherein in (IVb)

- the asterisk symbol "*" represents the binding position for binding the group of formula (IVb) to A' or, in case that d = 0 to X'; and
- $X^3$ is selected from $C(CH_3)_2$, or O.

$Ar^1$ may be independently selected from the group consisting of a group having the formula (IVa) and a group having the formula (IVb)

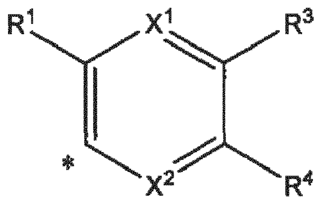

(IVa)

wherein in (IVa)

- the asterisk symbol "*" represents the binding position for binding the group of formula (IVa) to A' or, in case that d = 0 to X';

$X^1$ is $CR^2$; $X^2$ is $CR^5$; $R^1$ is H and $R^2$ to $R^5$ are each phenyl;

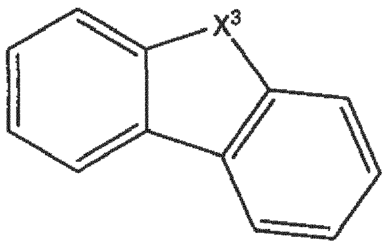

(VIb)

wherein in (IVb)

- the asterisk symbol "*" represents the binding position for binding the group of formula (IVb) to A' or, in case that d = 0 to X'; and
- X3 is selected from $C(CH_3)_2$.

In the compound of Formula (IV), e and f are independently 1 or 2. Alternatively, e and f may both be 1.
A' may be selected from

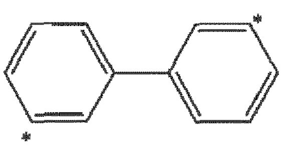 ; 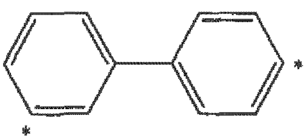 ; 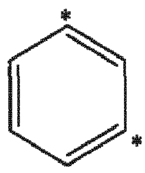 ; 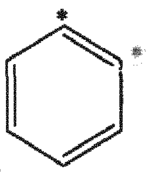 ; 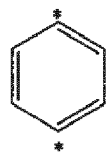 ;

wherein the asterisk symbols "*" represents the binding positions for binding A' to Ar'[1] and X', respectively.
X' may be independently selected from substituted or unsubstuted pyrimidinyl, quinazolinyl, and benzoquinazolinyl.
X' may be independently selected from pyrimidinyl substituted with two phenyl groups, quinazolinyl substituted with two phenyl groups, benzoquinazolinyl substituted with two phenyl groups.
X' may be selected independently from one of the following groups

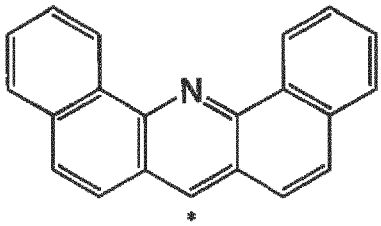 ; 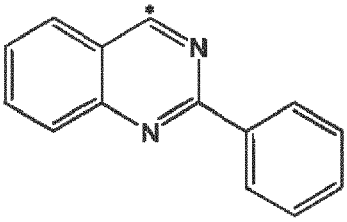 ; 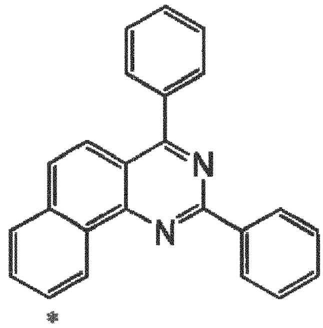 ;

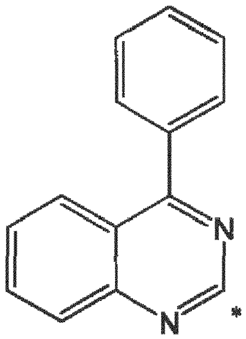 ; 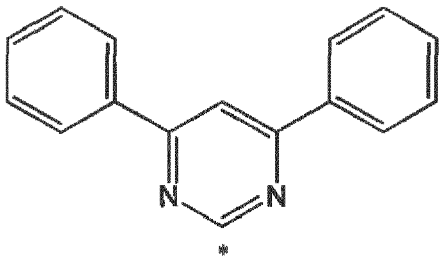 ; 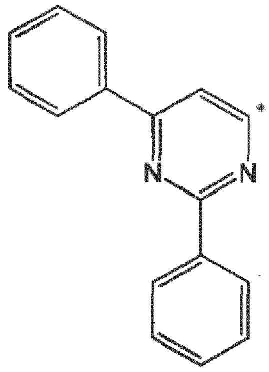 ;

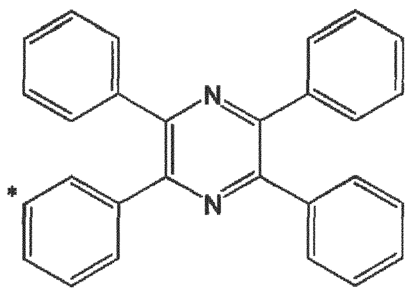 ; 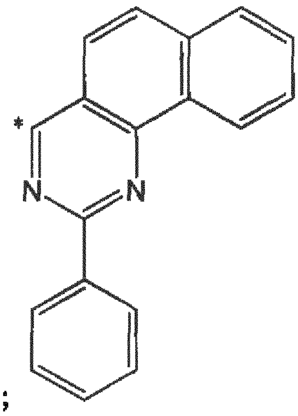 ; 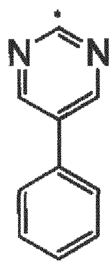 ,

wherein the asterisk symbol "*" represents the binding position for binding the to A', respectively in case that d = 0 to $Ar'^1$.

The compound of Formula (IV) may be selected from A-1 to A-12

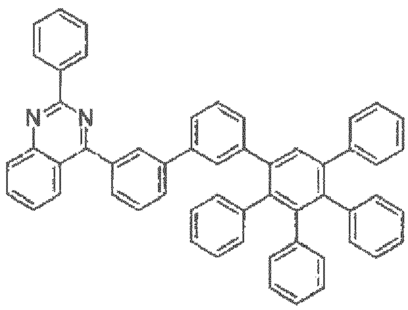

A-1

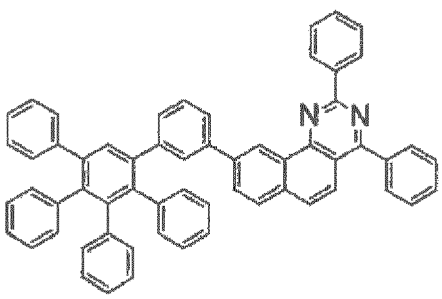

A-2

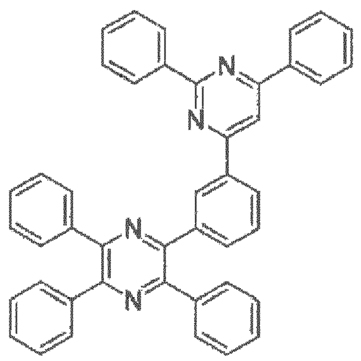

A-3

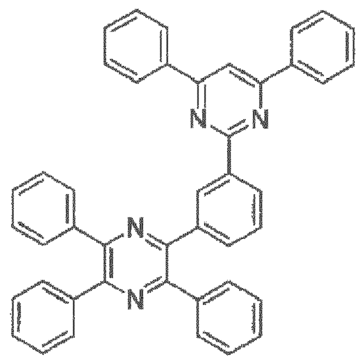

A-4

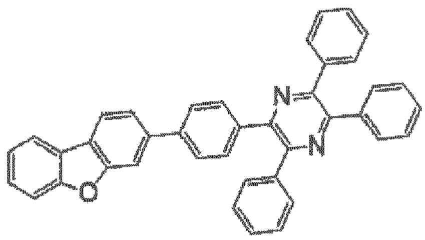

A-5

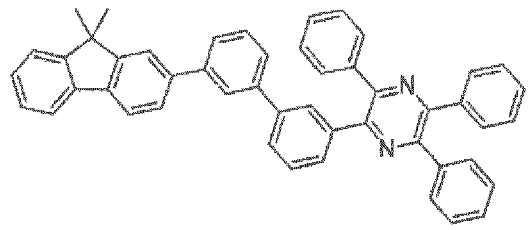

A-6

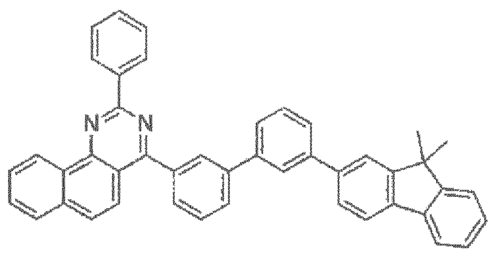

A-7

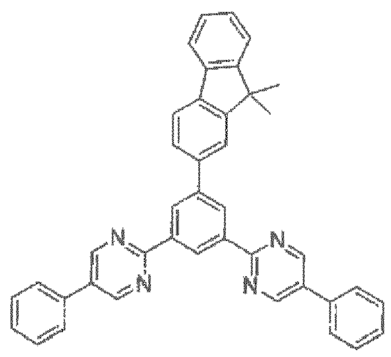

A-8

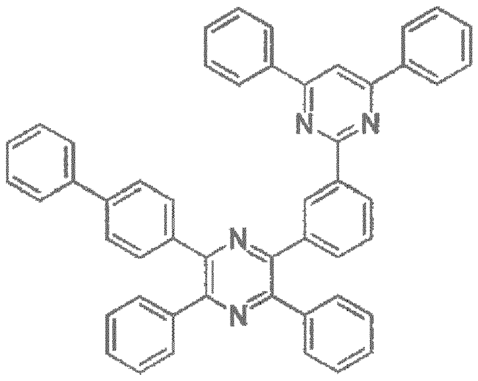

A-9

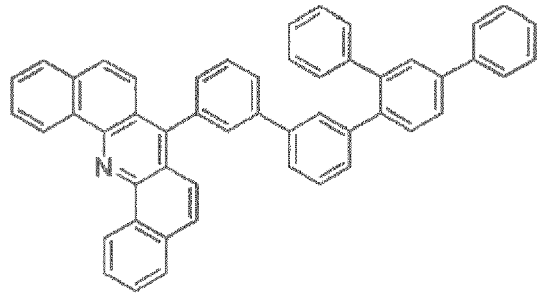

A-10

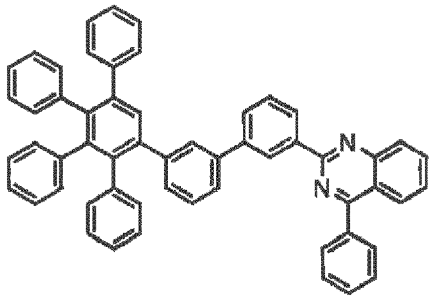

A-11

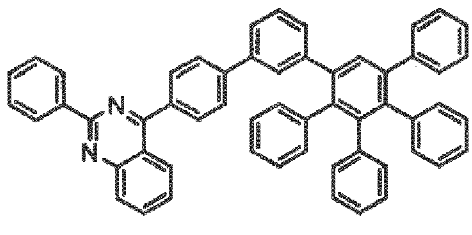

A-12

The compound of Formula (IV) may be selected from A-1 to A-4, A-7, A-9, A-11 or A-12.

GENERAL DEFINITIONS

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms.

Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light trough a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer

to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound of Formula (I) is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

## BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of a multi-emission layer organic light-emitting diode (OLED), according to an exemplary embodiment of the present invention;

FIG. 2 is a schematic sectional view of a multi-emission layer OLED, according to an exemplary embodiment of the present invention.

## DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 200, according to an exemplary embodiment of the present invention. The OLED 200 includes a substrate 110, an anode 120, a first hole injection layer (HIL-1) 130, a first hole transport layer (HTL-1) 140, a first electron blocking layer (EBL-1) 145, a first emission layer

(EML-1) 150, a first electron transport layer (ETL-1) stack 160 comprising a first electron transport layer of the first stack 161 and a second electron transport layer of the first stack 162, an n-type charge generation layer (n-type CGL) 185, a hole generating layer (p-type charge generation layer; p-type GCL) 135, a second hole transport layer (HTL-2) 141, a second electron blocking layer (EBL-2) 146, a second emission layer (EML-2) 151, a second electron transport layer (ETL-2) stack 165 comprising a third electron transport layer (= first electron transport layer of the second stack) 166 and a fourth electron transport layer (= second electron transport layer of the second stack) 167, an electron injection layer (EIL) 181 and a cathode 190.

Referring to Fig. 2, the OLED 200 includes an anode 120, a hole injection layer (HIL) 130, a first hole transport layer (HTL-1) 140, a first electron blocking layer (EBL-1) 145, a first emission layer (EML-1) 150, a first electron transport layer (ETL-1) stack 160 comprising a first electron transport layer 161 and a second electron transport layer 162, a first charge generation layer (CGL-1) 184, a second hole transport layer (HTL-2) 141, a second electron blocking layer (EBL-2) 146, a second emission layer (EML-2) 151, a second electron transport layer (ETL-2) stack 165 comprising a third electron transport layer (= first electron transport layer of the second stack) 166 and a fourth electron transport layer (= second electron transport layer of the second stack) 167, a second charge generation layer (CGL-2) 186, a third hole transport layer (HTL-3) 142, a third electron blocking layer (EBL-3) 147, a third emission layer (EML-3) 152, a third electron transport layer (ETL-3) stack 168 comprising a fifth electron transport layer (= first electron transport layer of the third stack) 163 and a sixth electron transport layer (= second electron transport layer of the third stack) 164, an electron injection layer (EIL) 181 and a cathode 190. The OLED shown in Fig. 2 has a first emitting part A, a second emitting part B, and a third emitting part C.

While not shown in Fig. 1 and Fig. 2 a sealing layer may further be formed on the cathode electrodes 190, in order to seal the OLEDs 200. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

## DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 100, according to an exemplary embodiment of the present invention. The OLED 100 includes a non-transparent substrate 110, an anode 120, a hole injection layer (HIL) 130, a hole transport layer (HTL) 140, an emission layer (EML) 150, an electron transport layer (ETL) stack 160 comprising a first electron transport layer 161 and a second electron transport layer 162. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145.

Referring to Fig. 2, the OLED 100 includes a non-transparent substrate 110, an anode 120, a hole injection layer (HIL) 130, a hole transport layer (HTL) 140, an electron blocking layer (EBL) 145, an emission layer (EML) 150, an electron transport layer (ETL) stack 160 comprising a first electron transport layer 161 and a second electron transport layer 162, an electron injection layer (EIL) 180 and a cathode electrode 190.

While not shown in Fig. 1 and Fig. 2 a sealing layer may further be formed on the cathode electrodes 190, in order to seal the OLEDs 100 and 200. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature (TRO) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10-5 mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Synthesis procedure

## Compound A-7

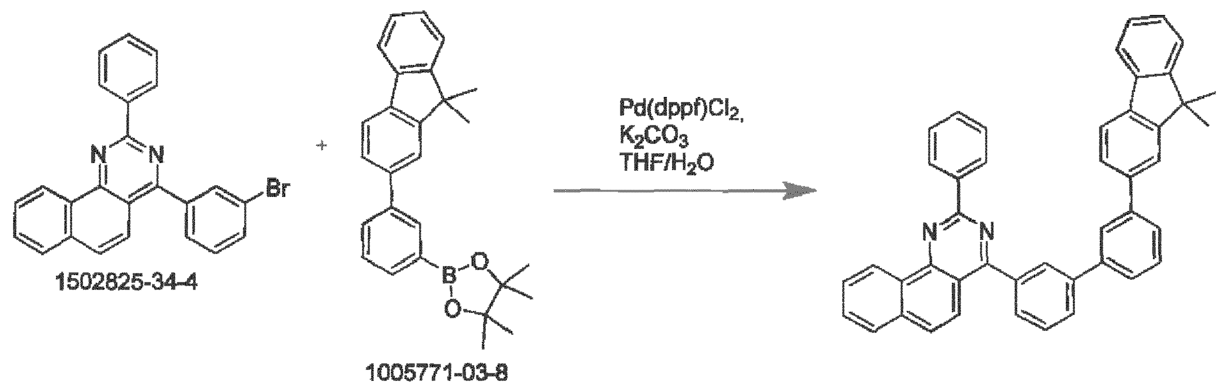


1502825-34-4

1005771-03-8

A flask was flushed with nitrogen and charged with 4-(3-bromophenyl)-2-phenylbenzo[h]quinazoline (CAS 1502825-34-4, 7.5 g, 18.2 mmol), 2-(3-(9,9-dimethyl-9H-fluoren-2-yl)phenyl)-4,4,5,5-tetramethyl-1,3,2-dioxaborolane (CAS 1005771-03-8, 7.9 g, 20.0 mmol), [1,1'-Bis(diphenylphosphino)ferrocene]dichloropalladium(II) (CAS 14221-01-3, 0.40 g, 0.5 mmol) and $K_2CO_3$ (7.6 g, 54.6 mmol). A mixture of deaerated THF/water (4:1) was added and the reaction mixture was heated to reflux under a nitrogen atmosphere overnight. After cooling to room temperature, phases were separeted and the organic phase was dried under reduced pressure. Obtained solid was dissolved in 100 ml DCM, washed with 2 x 50 ml $H_2O$, dried over $Na_2SO_4$ and filtered through a Florisil pad. The solvent was partially removed under reduced pressure and hexane was added to induce precipitation. Product was filtered, washed with hexane and further purified via column chromatography using DCM/hexane 1:1 to yield 7.35 g (67%) pale yellow solid after drying. Final purification was achieved by sublimation. m/z = 601 ($[M+H]^+$).

## Compound A-3

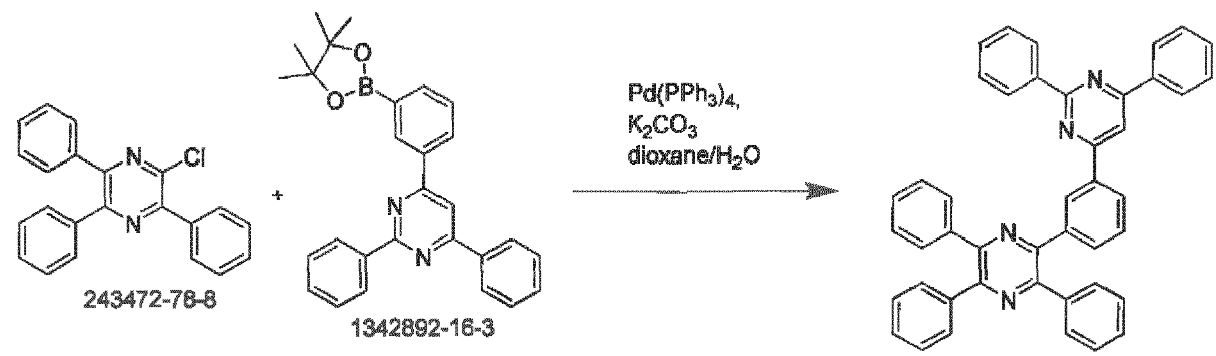


243472-78-8

1342892-16-3

A flask was flushed with nitrogen and charged with 2-chloro-3,5,6-triphenylpyrazine (CAS 243472-78-8,11.2 g, 32.5 mmol), 2,4-diphenyl-6-(3-(4,4,5,5-tetramethyl-1,3,2-dioxaborolan-2-yl)phenyl)pyrimidine (CAS 1342892-16-3, 17,0 g, 39.0 mmol), tetrakis(triphenylphosphine)palladium(0) (CAS 14221-01-3, 0.75 g, 0.7 mmol) and $K_2CO_3$ (9,0 g, 65.1 mmol). A mixture of deaerated dioxane/water (4:1) was added and the reaction mixture was heated to 80 °C under a nitrogen atmosphere overnight. After cooling to room temperature, the resulting precipitate was isolated by suction filtration, washed with $H_2O$ (2 x 200 mL) and dioxane (175 ml) and dried. The crude product was then dissolved in DCM (1 L), filtered through a Florisil pad and rinsed with an additional 500ml DCM. After removing the solvent under reduced pressure, the product was recrystallized from toluene (100ml) to yield 12.3 g (62%) of a white solid after drying. Final purification was achieved by sublimation. m/z = 615 ($[M+H]^+$).

## Compound A-9

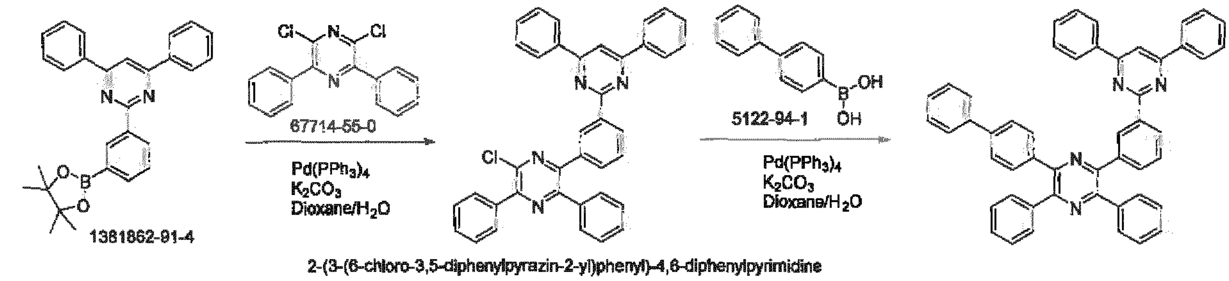


A flask was flushed with nitrogen and charged with 2,6-dichloro-3,5-diphenylpyrazine (CAS 67714-55-0,21,0 g, 69,8 mmol), 4,6-diphenyl-2-(3-(4,4,5,5-tetramethyl-1,3,2-dioxaborolan-2-yl)phenyl)pyrimidine (CAS 1381862-91-4, 30,3 g, 69,8 mmol), tetrakis(triphenylphosphine)palladium(o) (CAS 14221-01-3, 2,02 g, 1,8 mmol) and $K_2CO_3$ (19,3 g, 139,6 mmol). A mixture of deaerated dioxane/water (4:1) was added and the reaction mixture was heated to 80 °C under a nitrogen atmosphere overnight. After cooling to room temperature, the resulting precipitate was isolated by suction filtration, washed with $H_2O$, dioxane and MeOH and dried. The crude product was then recrystallized from toluene, washed with n-hexane and dried. The product was then purified through column chromatography with DCM/Hex (3:2) to yield 10,4 g (26%) of a white solid after drying. HPLC/ESI-MS: 100%, m/z = 574 ($[M+H]^+$).

A flask was flushed with nitrogen and charged with 2-(3-(6-chloro-3,5-diphenylpyrazin-2-yl)phenyl)-4,6-diphenylpyrimidine (19,0 g, 33,2 mmol), [1,1'-biphenyl]-4-ylboronic acid (CAS 5122,94-1,7,9 g, 39,8 mmol), tetrakis(triphenylphosphine)palladium(o) (CAS 14221-01-3, 0,8 g, 0,66 mmol) and $K_2CO_3$ (9,2 g, 66,3 mmol). A mixture of deaerated dioxane/water (4:1) was added and the reaction mixture was heated to 80 °C under a nitrogen atmosphere over three days. After cooling to room temperature, the resulting precipitate was isolated by suction filtration, washed with dioxane. The crude product was then dissolved in DCM and extracted in water. The organic phase was dried with $Na_2SO_4$ and filtered though a Florisil pad and rinsed with 100 mL DCM. After removing the solvents in vacuum the product was recrystallized in toluene and overlayed with EtOH. The crude solid was filtered and recrystallized from THF to yield 15,8 g of the product (69%) as an off white solid. Final purification was realized through sublimation, m/z = 1381 ($[2M+H]^+$).

## Compound A-2

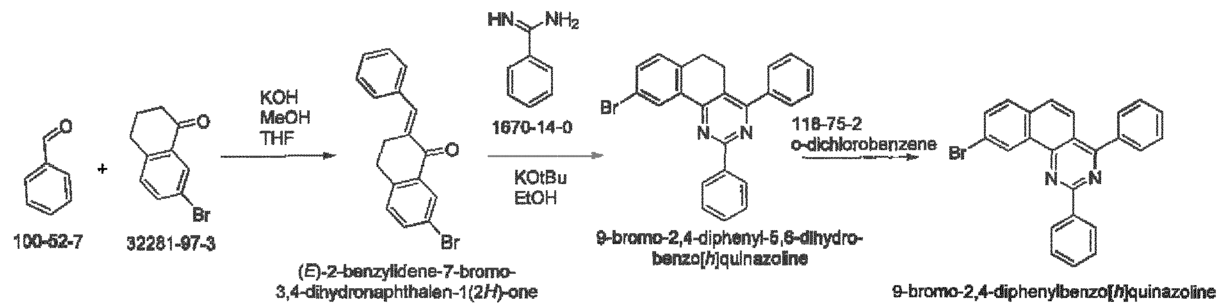


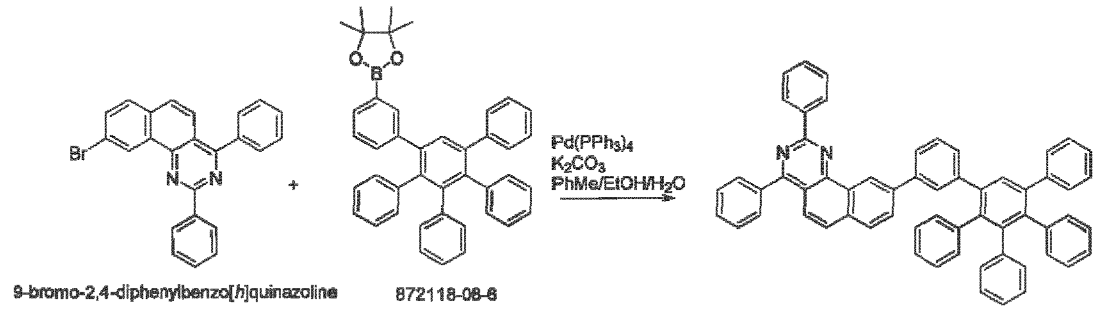


A flask was flushed with nitrogen and charged with 7-bromo-3,4-dihydronaphthalen-1(2H)-one (CAS

32281-97-3, 15,0 g, 66,6 mmol), benzaldehyde (CAS 100-52-7, 8,8 mL, 86,6 mmol) and 4% KOH solution in MeOH (CAS 1310-58-3, 112 mL, 218,0 mmol) and stirred for 1 h at room temperature. The product was filtered off and washed with MeOH. The product was isolated as a solid in 17,3 g (83%). GC-MS 100%, m/z = 313 $[M]^+$.

A flask was flushed with nitrogen and charged with (E)-2-benzylidene-7-bromo-3,4-dihydronaphthalen-1(2H)-one (17,1 g, 54,6 mmol), benzimidamide (CAS 1670-14-0, 8,6 g, 54,6 mmol) and potassium tert-butoxide (CAS 865-47-4, 12,3 g, 109,2 mmol) and diluted in EtOH. The reaction mixture was refluxed overnight. The precipitate was filtered, washed with water and EtOH and dried. The product was isolated with 16,4 g (73%). ESI-MS 100%, m/z = 415 ($[M]^+$).

A flask was flushed with nitrogen and charged with 9-bromo-2,4-diphenyl-5,6-dihydrobenzo[h]quinazoline (16,2 g, 39,2 mmol) and 2,3,5,6-tetrachlorocyclohexa-2,5-diene-1,4-dione (CAS 118-75-2, 19,3 g, 78,4 mmol). 1,2-dichlorobenze was added and the reaction mixture was refluxed under a nitrogen atmosphere overnight. MeOH was added to the reaction mixture to induce precipitation. The crude solid was then filtered and washed with MeOH. The product was obtained with 12,1 g (75%) after drying as a white solid. HPLC/ESI-MS 100%, m/z = 411 ($[M]^+$).

A flask was flushed with nitrogen and charged with 9-bromo-2,4-diphenylbenzo[h]quinazoline (9,0 g, 21,9 mmol), 4,4',5,5'-tetramethyl-2-2(3',4',5'-triphenyl-[1,1',2',1'-terphenyl]-3-yl)-1,3,2-dioxaborolan-2-yl)phenyl)pyrimidine (CAS 872118-08-6, 12,4 g, 21,2 mmol), tetrakis(triphenylphosphine)palladium(o) (CAS 14221-01-3, 0.76 g, 0.7 mmol) and $K_2CO_3$ (6,0 g, 43,8 mmol). A mixture of deaerated toluene/EtOH/water (2:1:1) was added and the reaction mixture was heated to 85 °C under a nitrogen atmosphere overnight. The crude solid was filtrated, washed with water and MeOH. The product was dissolved in $CHCl_3$ and filtered through a Florisil pad and rinsed with an additional 100 mL $CHCl_3$. After evaporating the solvent the solid was washed with n-hexane. The product was obtained with 13,7 g (82%) after drying, m/z = 789 ($[M+H]^+$). The final purification was realized through sublimation.

## Compound A-11

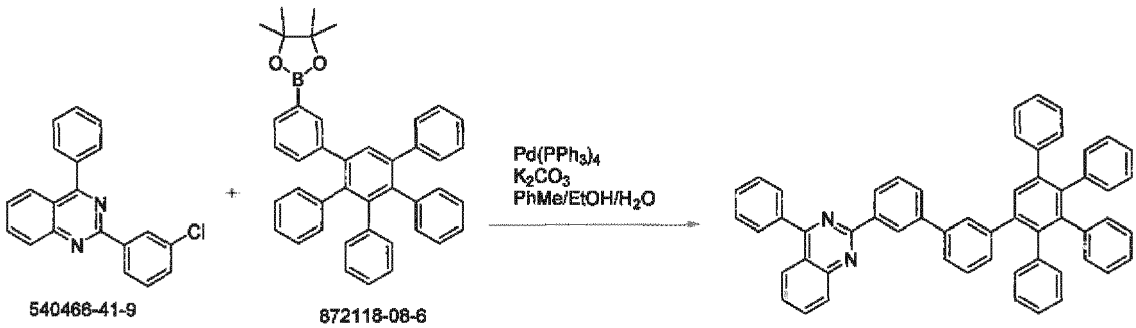


540466-41-9

872118-08-6

A flask was flushed with nitrogen and charged with 2-(3-chlorophenyl)-4-phenylquinazoline (CAS 540466-41-9, 7,5 g, 23,7 mmol), 4,4',5,5'-tetramethyl-2-(3',4',5'-triphenyl-[1,1',2',1"-terphenyl]-3-yl)-1,3,2-dioxaborolane (CAS 872118-08-6, 13,4 g, 23,0 mmol), tetrakis(triphenylphosphine)palladium(o) (CAS 14221-01-3, 2,2 g, 1,9 mmol) and $K_2CO_3$ (17,5 g, 126,6 mmol). A mixture of deaerated toluene/EtOH/water (4:1:1) was added and the reaction mixture was heated to 85 °C under a nitrogen atmosphere overnight. The crude solid was filtrated, washed with water. The product was dissolved in $CHCl_3$ and filtered through a Florisil pad and rinsed with an additional 200 mL $CHCl_3$. After partially evaporating the solvent, n-hexane was added to induce precipitation. The solid product was recrystallized from ethyl acetate. The off white solid was washed with a mixture of ethyl acetate and hexane (1:1) to obtained the product with 9,3 g (55%) after drying. m/z = 739 ($[M+H]^+$).

## Compound A-1

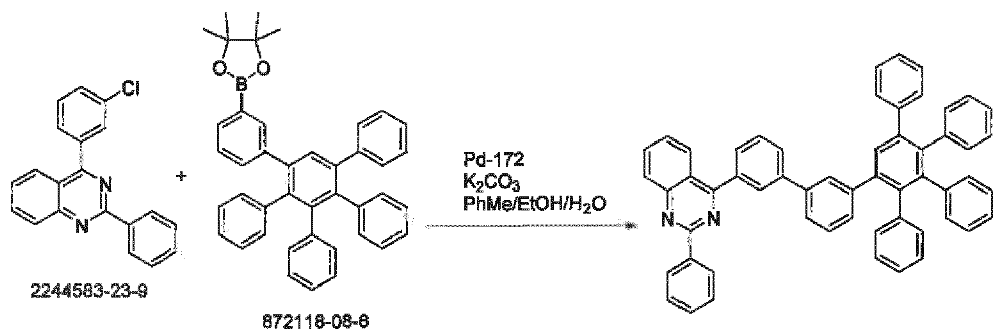


A flask was flushed with nitrogen and charged with 4-(3-chlorophenyl)-2-phenylquinazoline (CAS 2244583-23-9, 20,2 g, 63,8 mmol), 4,4',5,5'-tetramethyl-2-(3',4',5'-triphenyl-[1,1',2',1"-terphenyl]-3-yl)-1,3,2-dioxaborolane (CAS 872118-08-6, 36,6 g, 62,5 mmol), Pd-172 (CAS 1798781-99-3. 0,8 g, 1,2 mmol) and $K_2CO_3$ (27,1 g, 127,6 mmol). A mixture of deaerated THF/water (4:1) was added and the reaction mixture was heated to 45 °C under a nitrogen atmosphere overnight. THF was evaporated from the mixture and the crude product was dissolved in DCM and extracted twice with water. The organic phase was dried over $Na_2SO_4$ and filtered through a pad of Florisil and silica, washed with water. After removing the solvent in vacuo the solid was dissolved in cyclo-hexane and n-heptane was added to induce precipitation. The solid product was recrystallized twice from isopropyl acetate. The off white solid was washed with n-hexane to obtained the product with 28,4 g (60%) after drying. The final purification was through sublimation. m/z = 739 ($[M+H]^+$).

## Compound A-4

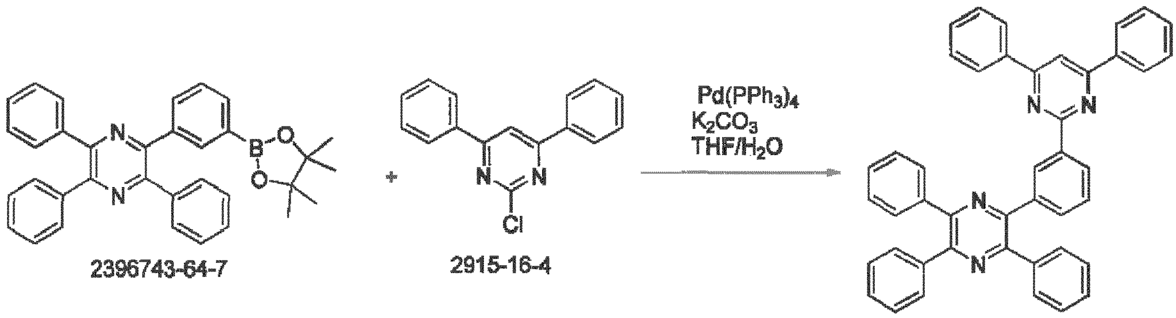


3 Neck round-bottom flask was flushed with nitrogen and charged with 2,3,5-triphenyl-6-(3-(4,4,5,5-tetramethyl-1,3,2-dioxaborolan-2-yl)phenyl)pyrazine (CAS 2396743-64-7, 34,8 g, 68,2 mmol), 2-chloro-4,6-diphenylpyrimidine (CAS 2915-16-4, 20,0 g, 75,0 mmol), tetrakis(triphenylphosphine)palladium(o) (CAS 14221-01-3, 2,4 g, 2,0 mmol), potassium carbonate (CAS 584-08-7, 18,8 g, 138,2 mmol). Deaerated mixture of THF/water (4:1) was added. Reaction was running at reflux under a nitrogen atmosphere overnight. Next day the reaction was cooled down and thick yellowish suspension was formed, precipitate was filtrated and washed with water. Grey solid was dissolved in 1,2 L of chloroform, washed with water 2x200 ml, dried over magnesium sulfate, filtrated through silica pad. Further purification was done by washing with hot THF and recrystallization from chlorobenzene. Final purification was done by sublimation. White crystalline powder. Yield: 32,3 g (77%). m/z = 637 ($[M+Na]^+$).

## Compound A-12

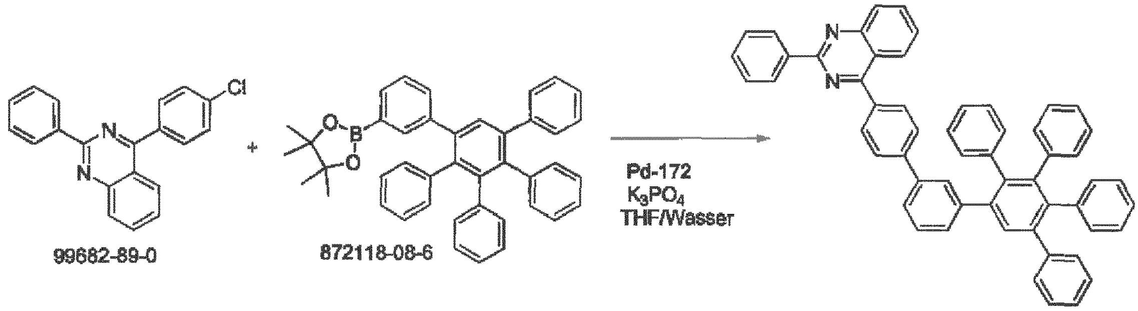


3 Neck round-bottom flask was flushed with nitrogen and charged with 4,4,5,5-tetramethyl-2-(3',4',5'-triphenyl-[1,1':2',1"-terphenyl]-3-yl)-1,3,2-dioxaborolane (CAS 872118-08-6, 32,5 g, 55,6 mmol), 4-(4-chlorophenyl)-2-phenylquinazoline (CAS 99682-89-0, 17,6 g, 55,6 mmol), chloro(crotyl)(2-dicyclohexylphosphino-2',6'-dimethoxybiphenyl)palladium(II) (CAS 1798781-99-3, 0,7 g, 1,1 mmol), potassium phosphate (CAS 7778-53-2, 23,6 g, 111,2 mmol). Deaerated mixture of THF/water (5:1) was added. Reaction was running at 55 °C under a nitrogen atmosphere overnight. Next day reaction was cooled down; solvent was evaporated at reduced pressure. Residue was dissolved in 700 ml of chloroform, washed with water, dried over magnesium sulfate, filtrated through silica pad; solvent was evaporated at reduced pressure. Resulted white powder was recrystallized first from toluene and second time from toluene/acetonitrile 1:1 mixture. Final purification was done by sublimation. White crystalline powder. Yield: 27,1 g (66%), m/z = 739 ($[M+H]^+$).

General procedure for fabrication of OLEDs

For the top emission OLED devices a substrate with dimensions of 150 mm x 150 mm x 0.7 mm was ultrasonically cleaned with a 2% aqueous solution of Deconex FPD 211 for 7 minutes and then with pure water for 5 minutes, and dried for 15 minutes in a spin rinse dryer. Subsequently, Ag was deposited as anode at a pressure of 10-5 to 10-7 mbar.

Then, HT-1 and D-1 were vacuum co-deposited on the anode to form a HIL. Then, HT-1 was vacuum deposited on the HIL to form an HTL. Then, HT-2 was vacuum deposited on the HTL to form an electron blocking layer (EBL).

Afterwards the first emission layer was formed on the EBL by co-deposition of HOST-1 and EMITIER-1.

Then, the compound of Formula (I) (compounds A-1, A-2 and A-4) respectively the comparative compound T-2, T-13 and T-15 was vacuum deposited onto the emission layer to form the first electron transport layer. Then, the second electron transport layer was formed on the first electron transport layer by depositing a pre-mix compound of Formula (II) and compound (III).

Then, the n-type CGL was formed on the second electron transport layer by co-depositing compound E and lithium.

Then, HT-1 and D-1 were vacuum co-deposited on the n-type CGL to form a p-type CGL

Then, HT-1 was vacuum deposited on the p-type CGL to form an HTL. Then, HT-2 was vacuum deposited on the HTL to form an EBL.

Afterwards the second emission layer was formed on the EBL by co-deposition of HOST-1 and EMITIER-1.

Then, the compound of Formula (I) (compounds A-1, A-2 and A-4) respectively the comparative compound T-2, T-13 and T-15was vacuum deposited onto the emission layer to form the first electron transport layer. Then, the second electron transport layer was formed on the first electron transport layer by depositing a pre-mix compound of Formula (II) and compound (III).

Then, the n-CGL was formed on the second electron transport layer by co-depositing compound E and Lithium.

Then, HT-1 and D-1 were vacuum co-deposited on the n-type CGL to form a p-type CGL

Then, HT-1 was vacuum deposited on the HIL to form an HTL. Then, HT-2 was vacuum deposited on the HTL to form an electron blocking layer (EBL).

Afterwards the third emission layer was formed on the EBL by co-deposition of HOST-1 and EMITIER-1.

Then, the compound of Formula (I) (compounds A-1, A-2 and A-4) respectively the comparative compound T-2, T-13 and T-15 was vacuum deposited onto the emission layer to form the first electron transport layer. Then, the second electron transport layer was formed on the first electron transport layer by depositing a pre-mix compound of Formula (II) and compound (III).

For examples OLED-5 to OLED-15 the second electron transport layer was formed on the first electron transport layer by depositing a pre-mix compound of Formula (II) and compound (III).

Then, the electron injection layer is formed as a double layer on the electron transport layer by depositing first

LiQ and subsequently Yb.

Ag:Mg is then evaporated at a rate of 0.01 to 1 Å/s at 10-7 mbar to form a cathode.

A cap layer of HT-3 is formed on the cathode.

The details of the layer stack in the top emission OLED devices are given below. A slash "/" separates individual layers. Layer thicknesses are given in squared brackets [...], mixing ratios in wt% given in round brackets (...):

*Layer stack details used in the OLED device examples of Table 5*:

Ag [100nm] / HT-1:D-1 (wt% 92:8) [10nm] / HT-1 [24nm] / HT-2 [5nm] / H09:BD200 (wt% 97:3) [20 nm] / Compound of Formula (I) [5nm] / Compound of Formula (II): Compound of Formula (III) (wt% 20:80) [25nm] / E:Li (wt% 99:1) [15nm] / HT-1:D-1 (wt% 90:10) [10nm] /HT-1 [36nm] / HT-2 [5nm] / H09:BD200 (wt% 97:3) [20 nm] / Compound of Formula (I) [5nm] / Compound of Formula (II): Compound of Formula (III) (wt% 20:80) [25nm] / E:Li (wt%99:1) [15nm] / HT-1:D-1 (wt% 90:10) [10nm] /HT-1 [57nm] / HT-2 [5nm] / H09:BD200 (wt% 97:3) [20 nm] / Compound of Formula (I) [5nm] / Compound of Formula (II): Compound of Formula (III) (wt% 20:80) [30nm] / LiQ [1nm] / Yb [2nm] / Ag:Mg (wt% 90:10) [13 nm] / HT-3 [65nm]

Table 4: List of compounds used

Table 4:

| | IUPAC name | Reference |
|---|---|---|
| HT-1 | N-([1,1'-biphenyl]-2-yl)-N-(9,9-dimethyl-9H-fluoren-2-yl)-9,9'-spirobi[fluoren]-2-amine [CAS 1364603-07-5] | WO2012034627 |
| HT-2 | N-(1,1'-biphenyl]-4-yl)-9,9-diphenyl-N-(4-(triphenylsilyl) phenyl)-9H-fluoren-2-amine [CAS 1613079-70-1] | WO2014088047 |
| D-1 | 4,4',4"-((1E,1'E,1"E)-cyclopropane-1,2,3-triylidenetris (cyanomethanylylidene))tris(2,3,5,6-tetrafluorobenzonitrile) [CAS 1224447-88-4] | US2008265216 |
| HOST-1 | H09 (Fluorescent-blue host material) | Commercially available from Sun Fine Chemicals, Inc, S. Korea |
| EMITTER-1 | BD200 (Fluorescent-blue emitter material) | Commercially available from Sun Fine Chemicals, Inc, S. Korea |
| LiQ | 8-Hydroxyquinolinolato-lithium [CAS 850918-68-2] | WO2013079217 |
| HT-3 | N-([1,1'-biphenyl]-4-yl)-9,9-dimethyl-N-(4-(9-phenyl-9H-carbazol-3-yl)phenyl)-9H-fluoren-2-amine [CAS 1242056-42-3] | US2016322581 |
| T-2 | 2,4-diphenyl-6-(4',5',6'-triphenyl-[1,1':2',1":3",1"':3"',1""-quinquephenyl]-3""-yl)-]1,3,5-triazine 2032364-64-8 | n/a |
| T-13 | 2-([1,1'-biphenyl]-3-yl)-4-phenyl-6-(3-(9-phenyl-9H-fluoren-9-yl)phenyl)-1,3,5-triazine 2396647-22-4 | WO2019235871 |
| T-15 | 2,4-diphenyl-6-(3"-(3,5,6-triphenylpyrazin-2-yl)-[1,1':3',1"-terphenyl]-3-yl)-1,3,5-triazine 2396743-80-7 | n/a |
| B-17 | (3-(4-(4-(4,6-diphenyl-1,3,5-triazin-2-yl)phenyl)naphthalen-1-yl)phenyl)dimethylphosphine oxide 2253724-56-8 | EP3407401 |

(continued)

| | IUPAC name | Reference |
|---|---|---|
| C-3 | 2-(11,1'-biphenyl]-3-yl)-4-phenyl-6-(3-(10-phenylanthracen-9-yl)phenyl)-1,3,5-triazine<br>2437303-42-7 | n/a |
| E | 1,3-bis(9-phenyl-1,10-phenanthrolin-2-yl)benzene<br>[CAS 721969-94-4] | JP 2004281390 |

Table 5: Performance of an organic electroluminescent device comprising the compound of Formula (I) in the first electron transport layer and a mixture of compound of Formula (II) and compound of Formula (III) in the second electron transport layer.

Table 5:

| | First electron transport layer | Second electron transport layer | EIL | Operating voltage at 10 mA/cm$^2$ | cd/A efficiency at 10 mA/cm$^2$ | Relative Operating voltage at 10 mA/cm$^2$ (% vs Comparative-1) | Relative cd/A efficiency at 10 mA/cm$^2$ (% vs Comparative-1) |
|---|---|---|---|---|---|---|---|
| Comparative-1 | T-2 | B-17+C-3 (20:80) | LiQ | 9,86 | 27,2 | 100 | 100 |
| Comparative-2 | T-13 | B-17+C-3 (20:80) | LiQ | 9,77 | 27,7 | 99 | 102 |
| Comparative-3 | T-15 | B-17+C-3 (20:80) | LiQ | 9,83 | 27 | 100 | 99 |
| | | | | | | | |
| OLED-1 | A-1 | B-17+C-3 (20:80) | LiQ | 9,88 | 29,53 | 100 | 109 |
| OLED-2 | A-2 | B-17+C-3 (20:80) | LiQ | 9,65 | 31,21 | 98 | 115 |
| OLED-3 | A-4 | B-17+C-3 (20:80) | LiQ | 9,84 | 28,7 | 100 | 106 |

As it can be seen from comparison of the inventive examples (OLEDs 1 to 3) with the comparative example in which a 1,3,5-triazine-containing compound was used as the compound of Formula (I) respectively, the use of the inventive 1,3,5-triazine-free compounds as the compound of Formula (I) in the first electron transport layer results in improved efficiency at comparable voltage.

The features disclosed in the foregoing description and in the dependent claims may, both separately and in any combination thereof, be material for realizing the aspects of the disclosure made in the independent claims, in diverse forms thereof.

## Claims

**1.** Organic light emitting diode comprising an anode, a cathode, a first emission layer, a second emission layer, a first charge generation layer and a first electron transport layer stack; wherein

- the first charge generation layer is arranged between the first emission layer and the second emission layer;
- the first electron transport layer stack is arranged between the first emission layer and the second emission layer;
- the first electron transport layer stack comprises a first electron transport layer and a second electron transport layer;
- the first electron transport layer comprises a compound of Formula (I)

$$(Ar^1\text{-}A_c)_a\text{-}X_b \qquad (I);$$

- a and b are independently 1 or 2;
- c is independently 0 or 1;
- $Ar^1$ is independently selected from $C_6$ to $C_{60}$ aryl or $C_2$ to $C_{42}$ heteroaryl,

  - wherein each $Ar^1$ may be substituted with one to three substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^1$ and each $C_3$ to $C_{11}$ heteroaryl substituent on $Ar^1$ may be substituted with $C_1$ to $C_4$ alkyl or halogen;

- A is independently selected from $C_6$ to $C_{30}$ aryl,

  - wherein each A may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on A may be substituted with $C_1$ to $C_4$ alkyl or halogen;

- X is independently selected from the group consisting of $C_2$ to $C_{42}$ heteroaryl,

  - wherein each X may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or

perdeuterated $C_1$ to $C_6$ alkyl, partially or perdeuterated $C_1$ to $C_6$ alkoxy;
- wherein each $C_6$ to $C_{12}$ aryl substituent on X and each $C_3$ to $C_{11}$ heteroaryl substituent on X may be substituted with $C_1$ to $C_4$ alkyl or halogen;

- the molecular dipole moment of the compound of formula (I) is ≥ 0 D and ≤ 4 D;
- the second electron transport layer comprises a compound of Formula (II)

$$(Ar^2)_m\text{-}(Z_k\text{-}G)_n \qquad (II);$$

- m and n are independently 1 or 2;
- k is independently 0, 1 or 2;
- $Ar^2$ is independently selected from the group consisting of $C_2$ to $C_{42}$ heteroaryl and $C_6$ to $C_{60}$ aryl,

  - wherein each $Ar^2$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;

- Z is independently selected from $C_6$ to $C_{30}$ aryl,

  - wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on Z may be substituted with $C_1$ to $C_4$ alkyl or halogen;

- G is chosen so that the dipole moment of a compound G-phenyl is ≥ 1 D and ≤ 7 D;
- the first electron transport layer and the second electron transport layer are free of an electrical dopant; and
- the compound of Formula (I) is free of 1,3,5-triazine;

wherein it is provided that the following compounds are excluded from Formula (I)

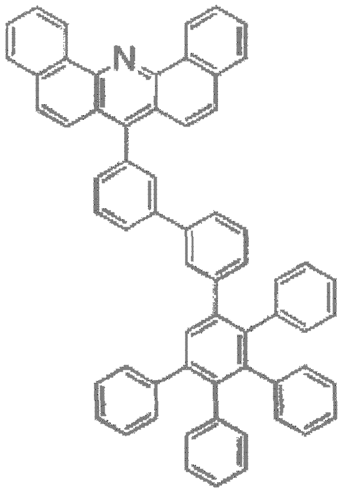
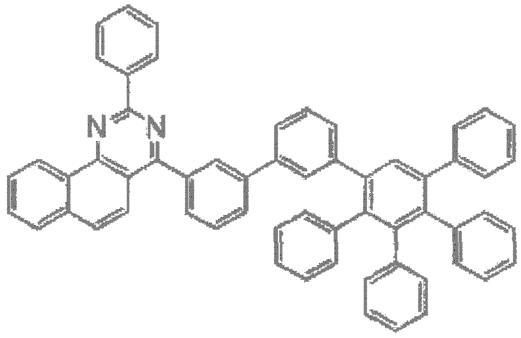

2. Organic light emitting diode according to claim 1, wherein $Ar^1$ is independently selected from the group consisting of phenyl, naphthyl, anthracenyl, fluoranthenyl, xanthenyl, dibenzofuranyl, pyrimidinyl pyrazinyl -, spiro-xanthenyl, fluorenyl, spiro-fluorenyl, triphenylsilyl, tetraphenylsilyl, or a group having the formula (IIa) or (IIb)

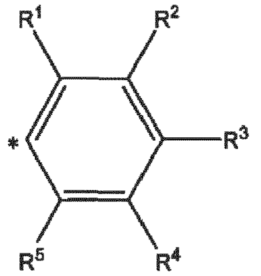
(IIa)

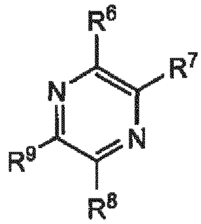
(IIb)

wherein

- the asterisk symbol "*" represents the binding position for binding the group of formula (IIa) to A; and
- $R^1$ to $R^9$ are independently selected from the group consisting of H, $C_6$ to $C_{12}$ aryl and $C_4$ to $C_{10}$ heterorayl.

3. Organic light emitting diode according to claim 1, wherein $Ar^1$ is independently selected from the group consisting of fluoranthenyl, dibenzofuranyl, pyrimidinyl pyrazinyl, 9,9-dimethylfluorenyl, a group having the formula (IIa), a group having the formula (IIb),

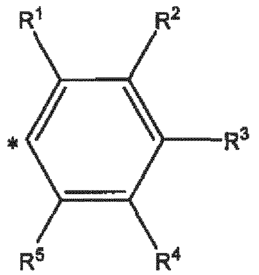
(IIa)

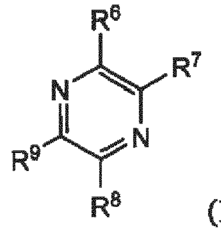
(IIb)

wherein

- the asterisk symbol "*" represents the binding position for binding the group of formula (IIa) to A; and
- $R^1$ is H and $R^2$ to $R^5$ are independently phenyl; or
- $R^1$ and $R^3$ are phenyl and $R^2$, $R^4$ and $R^5$ are H or

- $R^6$ to $R^9$ are phenyl.

4. Organic light emitting diode according to any of the preceding claims, wherein A is selected from the group consisting of phenylene, naphthylene, biphenylene and terphenylene, which may be substituted or unsubstituted, respectively.

5. Organic light emitting diode according to any of the preceding claims, wherein X is independently selected from the group consisting of pyridazinyl, pyrimidinyl, pyrazinyl, quinazolinyl, benzoquinazolinyl, benzimidazolyl, quinolinyl, benzoacridinyl, dibenzoacridinyl, phenathrolinyl, and dinaphthofuranyl which may be substituted or unsubstituted, respectively.

6. Organic light emitting diode according to any of the preceding claims, wherein X is independently selected from the group consisting of pyrimidinyl, pyrazinyl, quinazolinyl, benzoquinazolinyl, benzoacridinyl, dibenzoacridinyl, which may be substituted or unsubstituted, respectively.

7. Organic light emitting diode according to any of the preceding claims, wherein the compound of Formula (I) has a LUMO of ≥ -1.81 eV in the absolute scale taking vacuum energy level as zero, computed by the TURBOMOLE V6.5 program package using hybrid functional B3LYP and Gaussian 6-31G* basis set

8. Organic light emitting diode according to any of the preceding claims, wherein the compound of Formula (I) is selected from A-1 to A-12

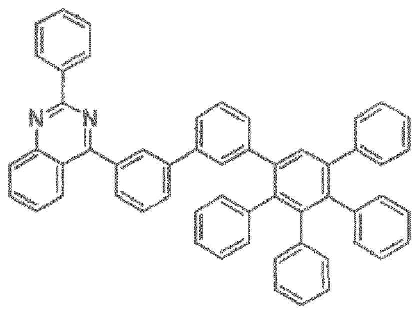

A-1

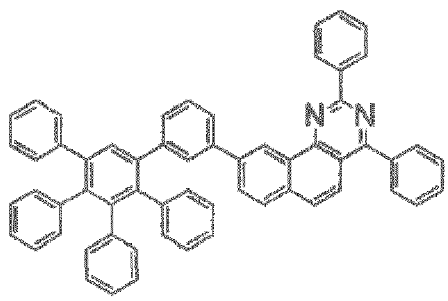

A-2

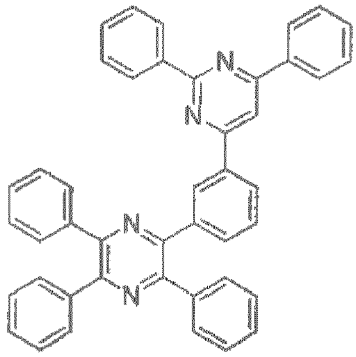

A-3

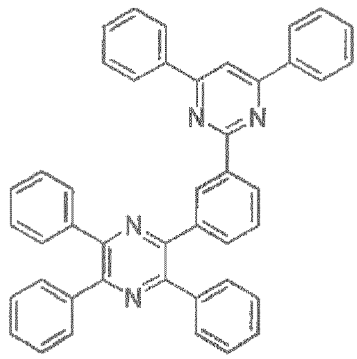

A-4

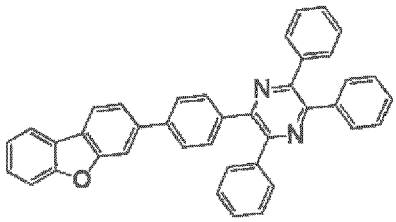

A-5

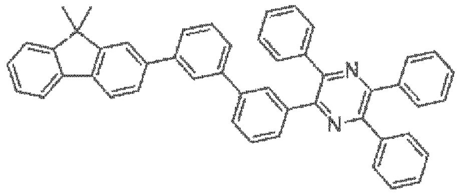

A-6

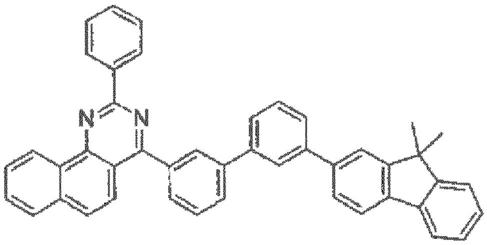

A-7

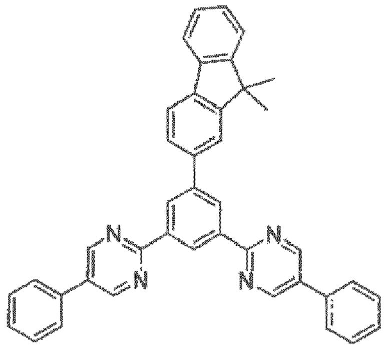

A-8

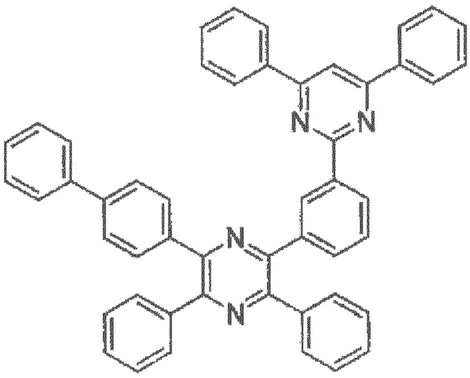

A-9

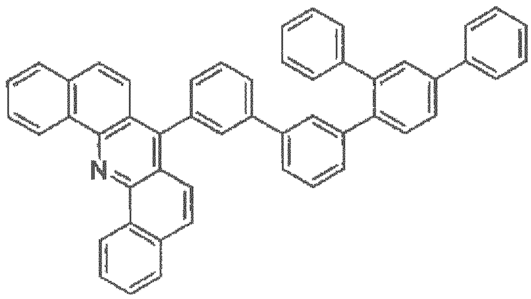

A-10

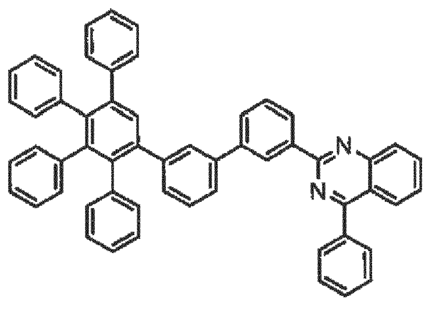

A-11

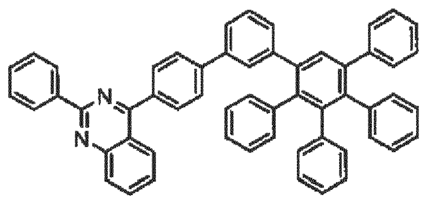

A-12

**9.** Organic light emitting diode according to any of the preceding claims, wherein $Ar^2$ is independently selected from the group consisting of pyridinyl, triazinyl, 1,2-diazinyl, 1,3-diazinyl, 1,4-diazinyl, quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl which may be substituted or unsubstituted, respectively.

**10.** Organic light emitting diode according to any of the preceding claims, wherein G independently selected from the group consisting of dimethylphosphinyl, diphenylphosphinyl, 2-phenyl-1H-benzo[d]imidazolyl, 2-ethyl-1H-benzo[d]imidazolyl, 2-phenylbenzo[h]quinolinyl, pyridinyl, 2,2'-bipyridinyl, 5-phenylbenzo[4,5]imidazo[1,2-a]quinolinyl, 9-phenyl-1,10-phenanthrolinyl and (pyridine-2-yl)imidazo[1,5-a]pyridinyl.

**11.** Organic light emitting diode according to any of the preceding claims, wherein G is selected such that the compound G-phenyl is represented by one of the following structures

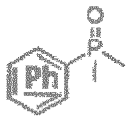 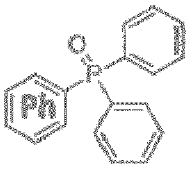 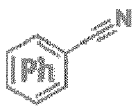 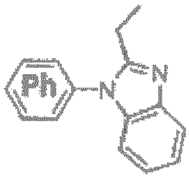 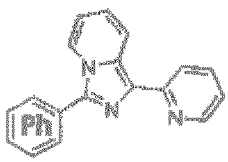 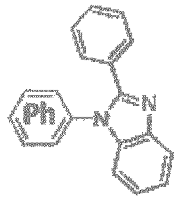

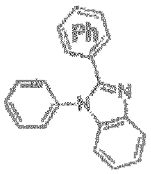 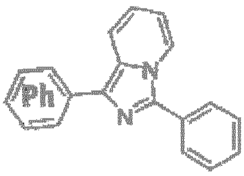 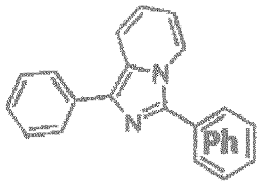 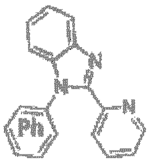 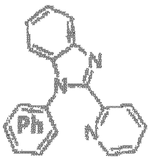 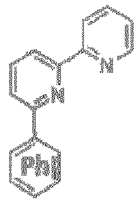

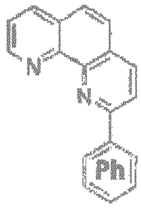 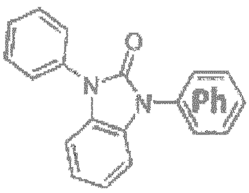 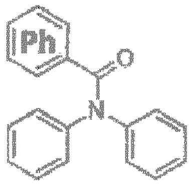 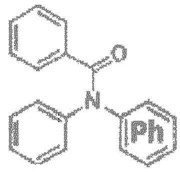 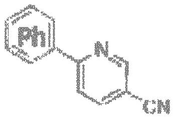 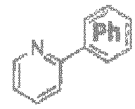

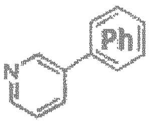 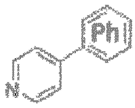 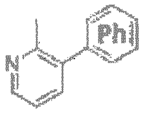 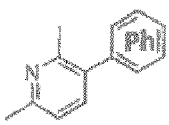 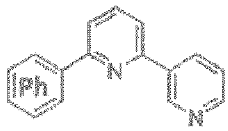 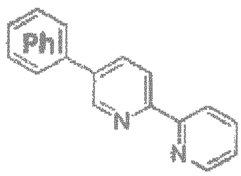

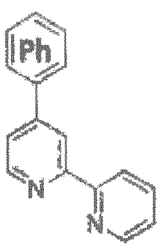 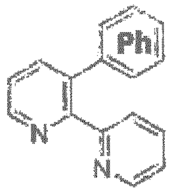 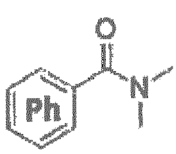 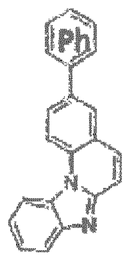 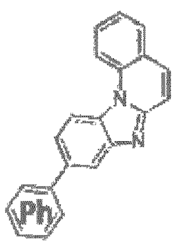 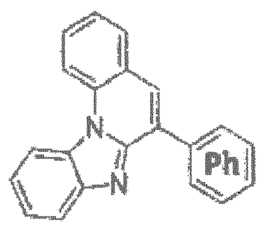

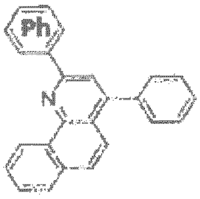
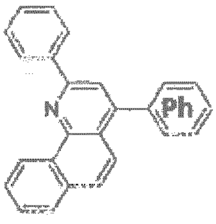
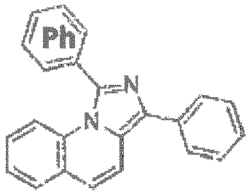
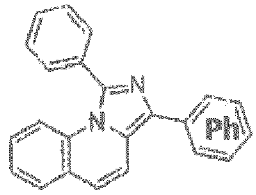
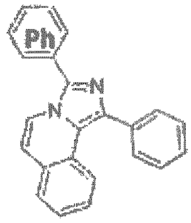
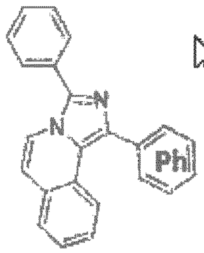
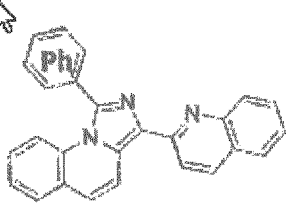
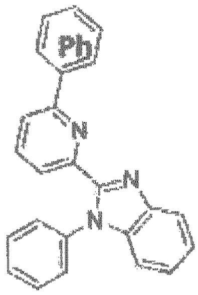
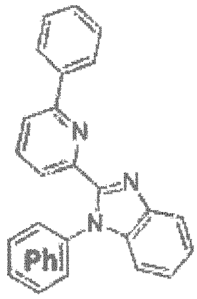
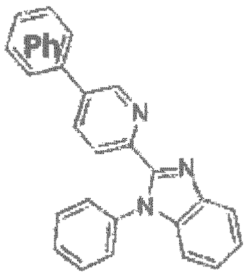
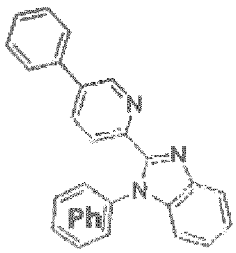
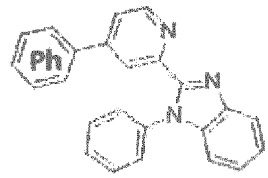
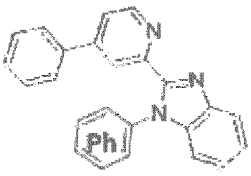
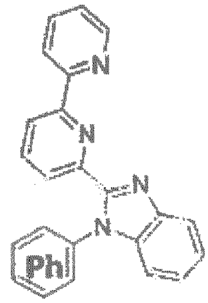
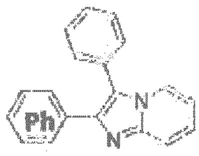
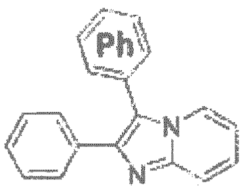
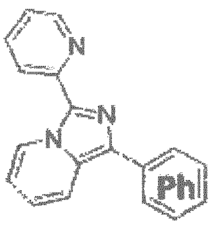
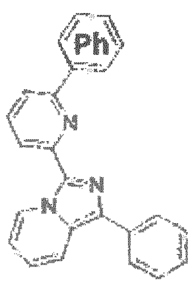
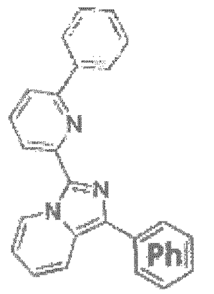
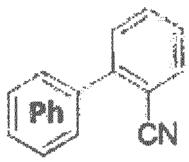
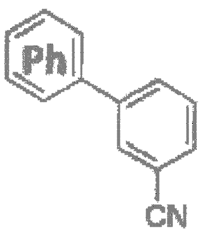
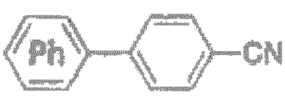
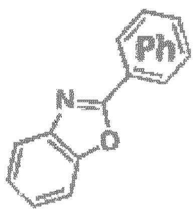
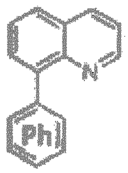
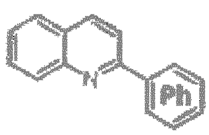
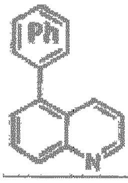
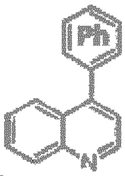
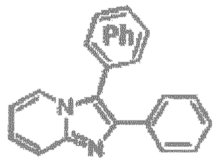
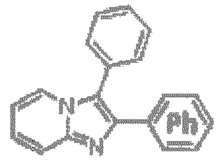

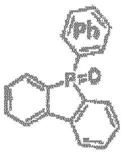 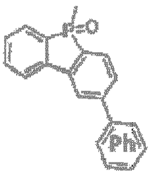 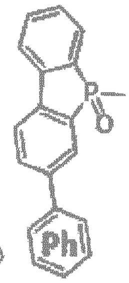 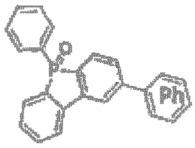 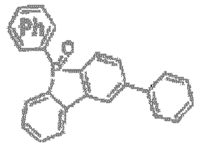 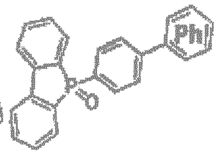

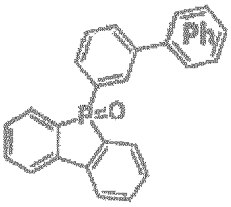 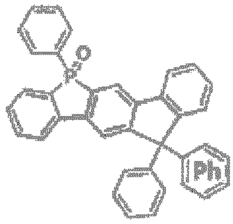 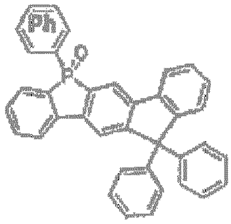 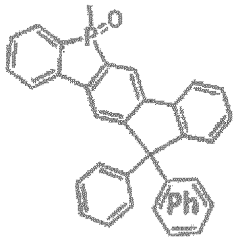

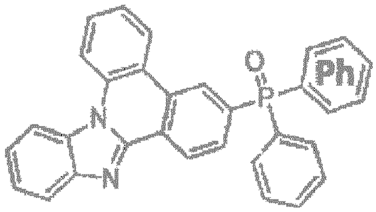 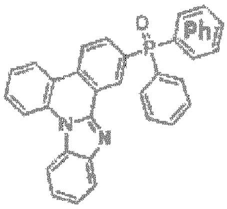 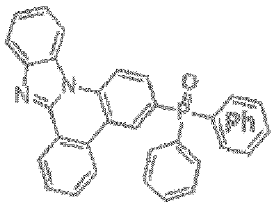

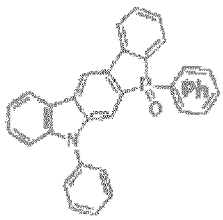 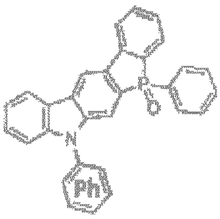 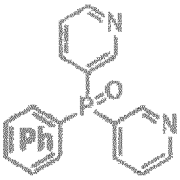 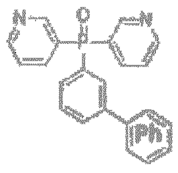 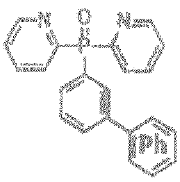 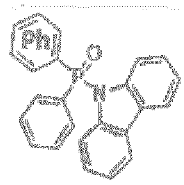

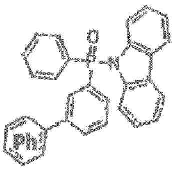 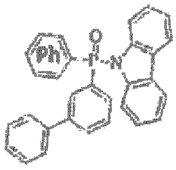 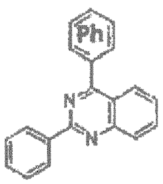 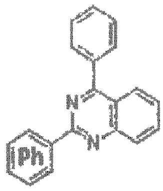 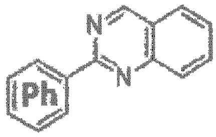

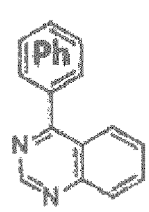 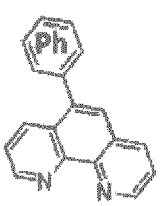 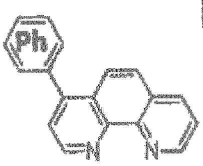 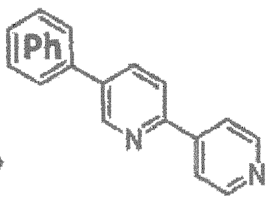

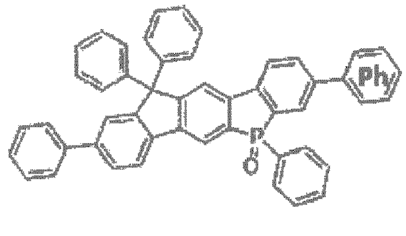
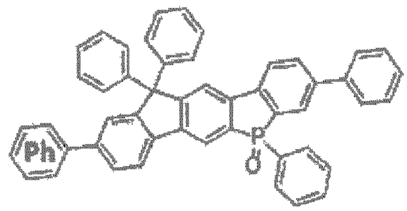

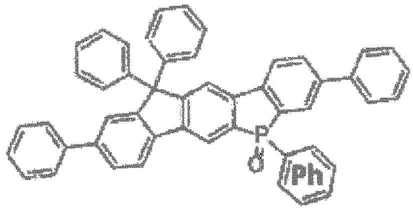
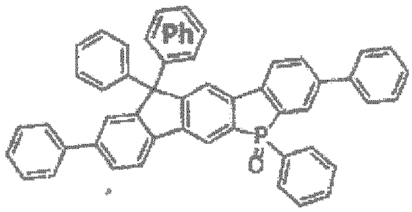

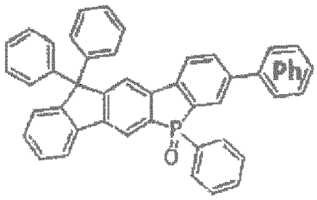
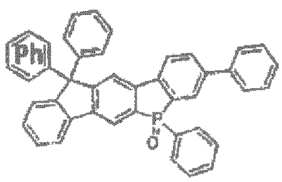
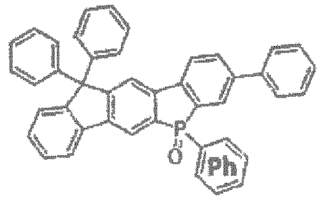

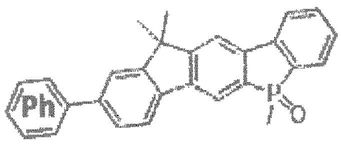
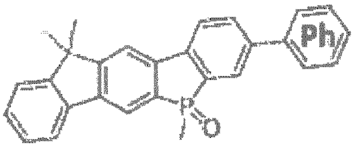
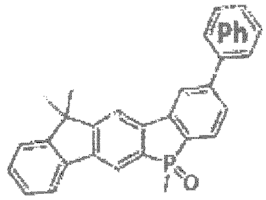

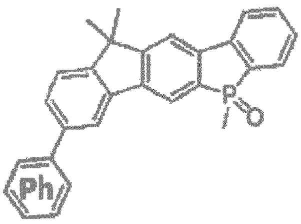
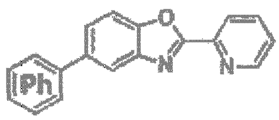
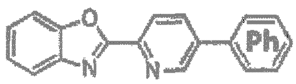
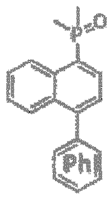

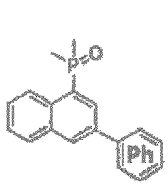
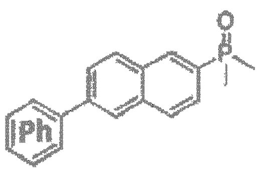
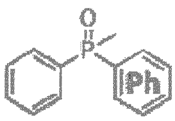
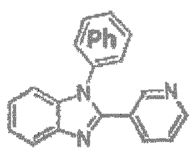
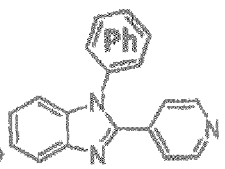

**12.** Organic light emitting diode according to any of the preceding claims, wherein the second electron transport layer further comprises a compound (III), wherein the compound (III) is different from the compound of formula (II) and comprises 8 to 13 aromatic or heteroaromatic rings.

**13.** Organic light emitting diode according to any of the preceding claims, wherein the first electron transport layer and the second electron transport layer are in direct contact with each other.

**14.** Organic light emitting diode according to any of the preceding claims, wherein the second electron transport layer is in direct contact with the charge generation layer.

**15.** Compound of the following formula (IV)

$$(Ar'^{1}\text{-}A'_{d})_{e}\text{-}X'_{f} \qquad (IV)$$

wherein

- e and f are independently 1 or 2;
- d is independently o or 1;
- Ar'[1] is independently selected from the group consisting of a group having the formula (IVa) and a group having the formula (IVb)

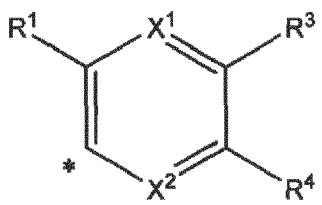

(IVa)

wherein in (IVa)

- the asterisk symbol "*" represents the binding position for binding the group of formula (IVa) to A' or, in case that d = o to X';
- $X^1$ and $X^2$ are both N; or $X^1$ is $CR^2$ and $X^2$ is CR5; and
- at least one of $R^1$ and $R^5$ is phenyl; and/or $R^1$ and $R^2$ are both phenyl; and/or $R^2$ and $R^3$ are both phenyl; and/or $R^3$ and $R^4$ are both phenyl; and/or $R^4$ and $R^5$ are both phenyl;

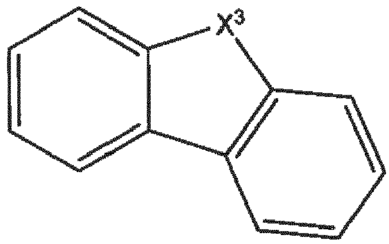

(IVb)

wherein in (IVb)

- the asterisk symbol "*" represents the binding position for binding the group of formula (IVb) to A' or, in case that d = o to X'; and
- $X^3$ is selected from $C(CH_3)_2$, O or S;
- wherein each $Ar^1$ may be substituted with one or two phenyl, preferably one phenyl

- A' is independently selected from phenylene or biphenylene;
- X' is independently selected from pyrimidinyl, pyrazinyl, quinazolinyl, benzoquinazolinyl, benzoacridinyl and dibenzoacridinyl;

wherein each pyrimidinyl, pyrazinyl, quinazolinyl, and benzoquinazolinyl, may be independently be substituted with one or more phenyl, preferably one or two phenyl;
wherein it is provided that the following compounds are excluded from Formula (IV)

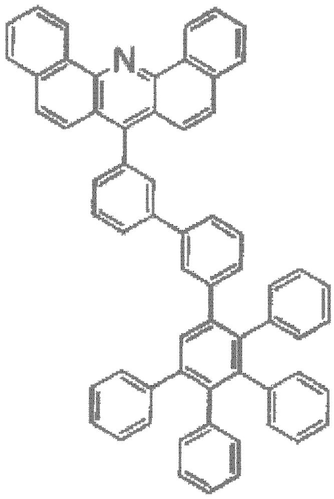

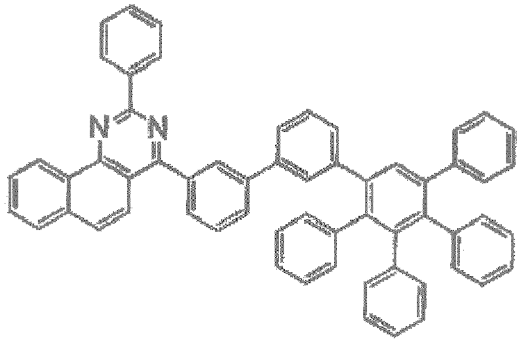

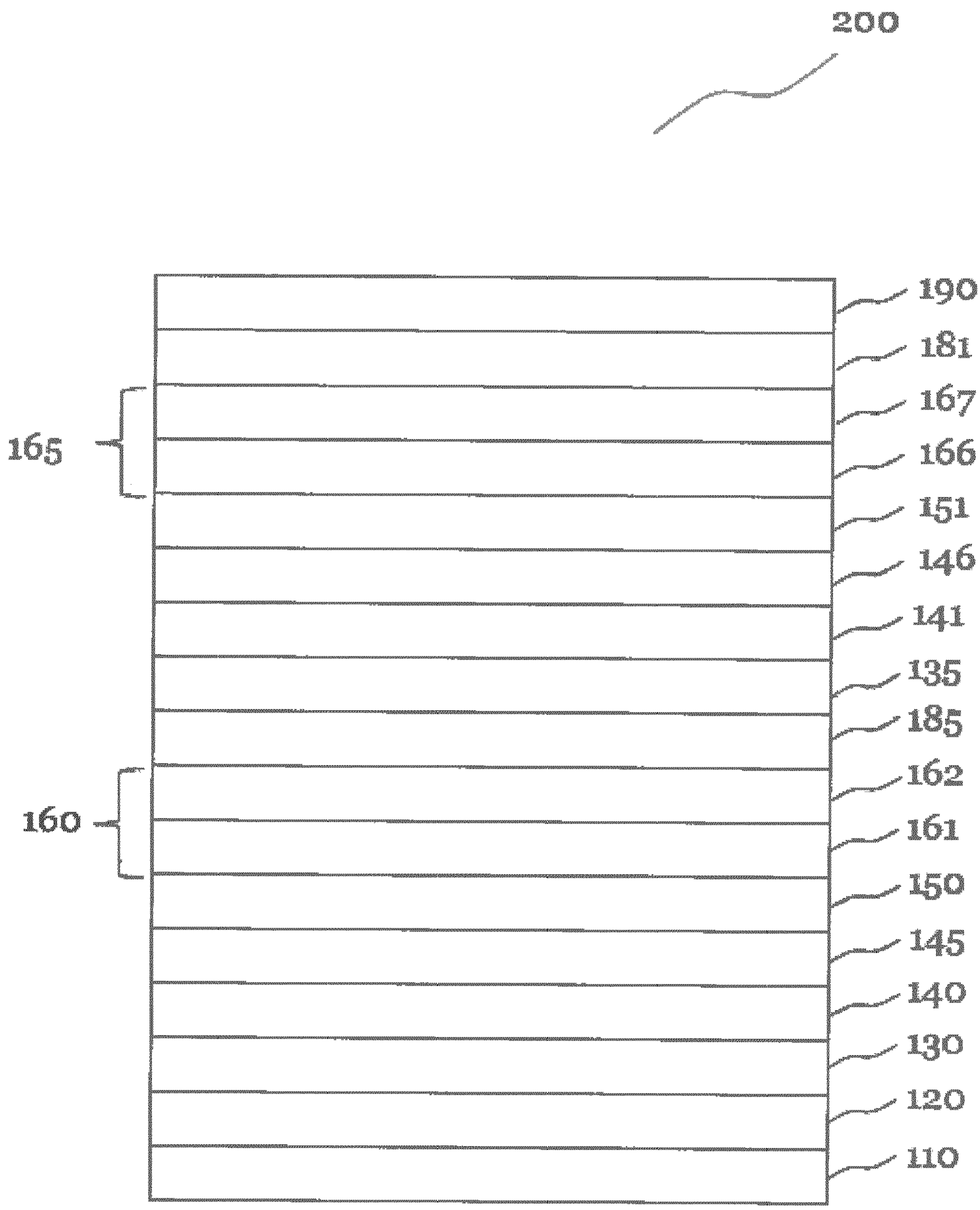
200
190
181
167
166
151
146
141
135
185
162
161
150
145
140
130
120
110
165
160

Fig. 1

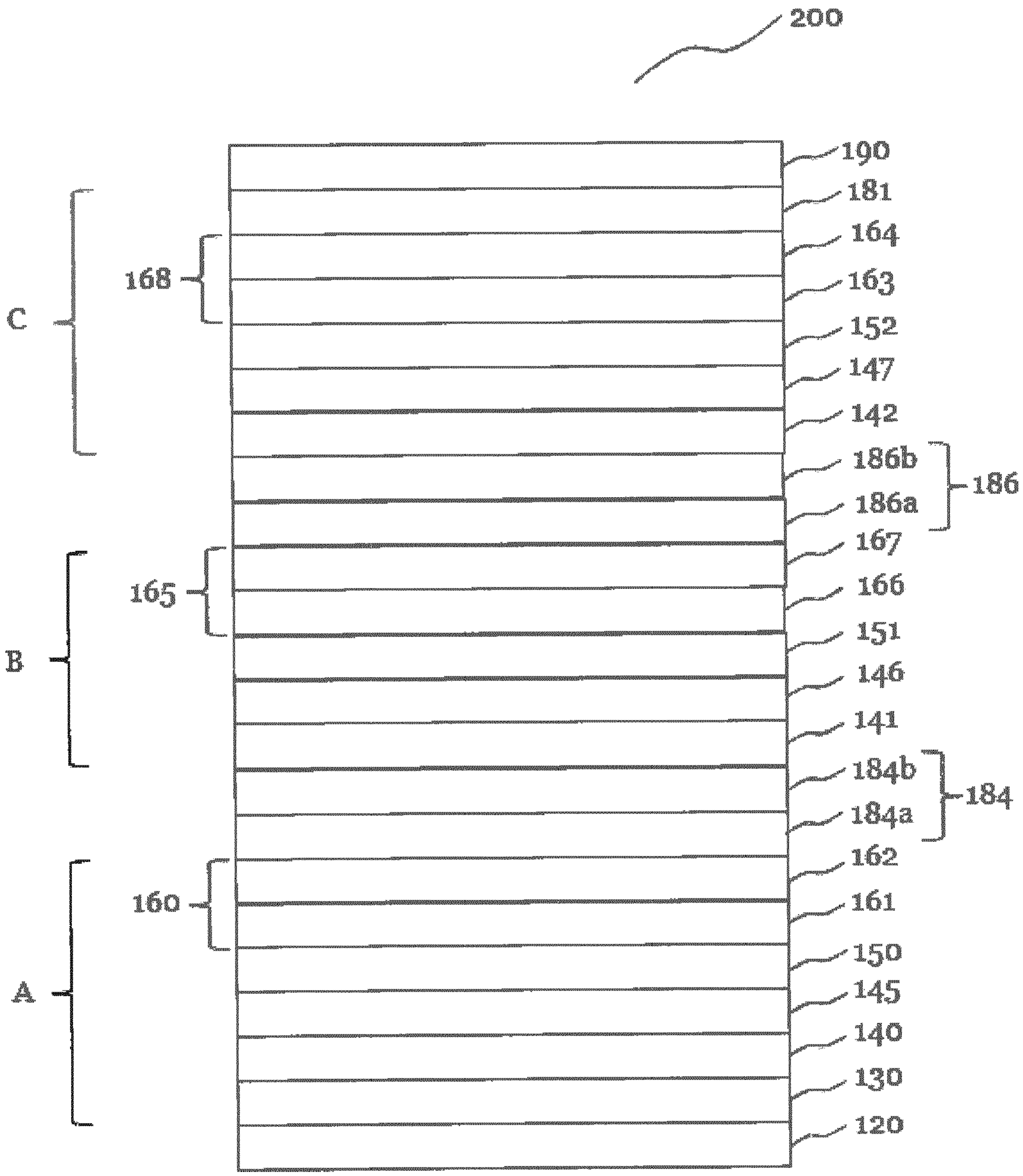
200
190
181
164
163
152
147
142
186b
186a
186
167
166
151
146
141
184b
184a
184
162
161
150
145
140
130
120
C
168
B
165
A
160

Fig. 2

Europäisches Patentamt
European Patent Office
Office européen des brevets

# EUROPEAN SEARCH REPORT

Application Number
EP 21 19 2830

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 208 861 A1 (NOVALED GMBH [DE]) 23 August 2017 (2017-08-23) * paragraphs [0319], [0322] - [0328], [0332], [0191], [0264], [0265], [0371]; figure 7; example 3; table 7; compounds ETM1-15, A18 * | 1-14 | INV. H01L51/54 |
| X | EP 3 182 478 A1 (NOVALED GMBH [DE]) 21 June 2017 (2017-06-21) * paragraphs [0095], [0224], [0225], [0246], [0262], [0265] - [0274]; figure 5; example 12; tables 5,9; compounds ETM-15, Vr * | 1-14 | |
| X | US 2017/309830 A1 (KIM HYUNG SUN [KR] ET AL) 26 October 2017 (2017-10-26) * pages 14,18; compounds 26,59,60 * | 15 | |
| X | WO 2015/186882 A1 (SAMSUNG SDI CO LTD [KR]) 10 December 2015 (2015-12-10) * pages 14,16; compounds 34,88 * | 15 | TECHNICAL FIELDS SEARCHED (IPC) |
| X | EP 3 766 875 A1 (NOVALED GMBH [DE]) 20 January 2021 (2021-01-20) * page 40; compound 148 * | 15 | H01L |

1 The present search report has been drawn up for all claims

| Place of search | Date of completion of the search | Examiner |
|---|---|---|
| The Hague | 3 February 2022 | Fratiloiu, Silvia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 19 2830

This annex lists the patent family members relating to the patent documents cited in the above-mentioned European search report. The members are as contained in the European Patent Office EDP file on
The European Patent Office is in no way liable for these particulars which are merely given for the purpose of information.

03-02-2022

| Patent document cited in search report | | Publication date | Patent family member(s) | | | Publication date |
|---|---|---|---|---|---|---|
| EP 3208861 | A1 | 23-08-2017 | CN | 108701773 | A | 23-10-2018 |
| | | | EP | 3208861 | A1 | 23-08-2017 |
| | | | EP | 3417495 | A1 | 26-12-2018 |
| | | | KR | 20180110681 | A | 10-10-2018 |
| | | | US | 2021210708 | A1 | 08-07-2021 |
| | | | US | 2021376259 | A1 | 02-12-2021 |
| | | | WO | 2017140780 | A1 | 24-08-2017 |
| EP 3182478 | A1 | 21-06-2017 | CN | 108463898 | A | 28-08-2018 |
| | | | EP | 3182478 | A1 | 21-06-2017 |
| | | | JP | 2019500752 | A | 10-01-2019 |
| | | | KR | 20180097653 | A | 31-08-2018 |
| | | | US | 2019006589 | A1 | 03-01-2019 |
| | | | WO | 2017102822 | A1 | 22-06-2017 |
| US 2017309830 | A1 | 26-10-2017 | CN | 107001292 | A | 01-08-2017 |
| | | | EP | 3287505 | A1 | 28-02-2018 |
| | | | JP | 6714002 | B2 | 24-06-2020 |
| | | | JP | 2018518036 | A | 05-07-2018 |
| | | | KR | 20160126698 | A | 02-11-2016 |
| | | | TW | 201710243 | A | 16-03-2017 |
| | | | US | 2017309830 | A1 | 26-10-2017 |
| | | | WO | 2016171358 | A1 | 27-10-2016 |
| WO 2015186882 | A1 | 10-12-2015 | KR | 20150140127 | A | 15-12-2015 |
| | | | WO | 2015186882 | A1 | 10-12-2015 |
| EP 3766875 | A1 | 20-01-2021 | EP | 3766875 | A1 | 20-01-2021 |
| | | | WO | 2021009206 | A1 | 21-01-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1837926 A1
- WO 07107306 A1
- WO 07107356 A1
- EP 2722908 A1
- WO 2012034627 A
- WO 2014088047 A
- US 2008265216 A
- WO 2013079217 A
- US 2016322581 A
- WO 2019235871 A
- EP 3407401 A
- JP 2004281390 B


### Non-patent literature cited in the description


- **YASUHIKO SHIROTA ; HIROSHI KAGEYAMA.** *Chem. Rev.,* 2007, vol. 107, 953-1010
- *CHEMICAL ABSTRACTS,* 1502825-34-4
- *CHEMICAL ABSTRACTS,* 1005771-03-8
- *CHEMICAL ABSTRACTS,* 14221-01-3
- *CHEMICAL ABSTRACTS,* 243472-78-8
- *CHEMICAL ABSTRACTS,* 1342892-16-3
- *CHEMICAL ABSTRACTS,* 67714-55-0
- *CHEMICAL ABSTRACTS,* 1381862-91-4
- *CHEMICAL ABSTRACTS,* 5122,94-1
- *CHEMICAL ABSTRACTS,* 32281-97-3
- *CHEMICAL ABSTRACTS,* 100-52-7
- *CHEMICAL ABSTRACTS,* 1310-58-3
- *CHEMICAL ABSTRACTS,* 1670-14-0
- *CHEMICAL ABSTRACTS,* 865-47-4
- *CHEMICAL ABSTRACTS,* 118-75-2
- *CHEMICAL ABSTRACTS,* 872118-08-6
- *CHEMICAL ABSTRACTS,* 540466-41-9
- *CHEMICAL ABSTRACTS,* 2244583-23-9
- *CHEMICAL ABSTRACTS,* 1798781-99-3
- *CHEMICAL ABSTRACTS,* 2396743-64-7
- *CHEMICAL ABSTRACTS,* 2915-16-4
- *CHEMICAL ABSTRACTS,* 584-08-7
- *CHEMICAL ABSTRACTS,* 99682-89-0
- *CHEMICAL ABSTRACTS,* 7778-53-2
- *CHEMICAL ABSTRACTS,* 1364603-07-5
- *CHEMICAL ABSTRACTS,* 1613079-70-1
- *CHEMICAL ABSTRACTS,* 1224447-88-4
- *CHEMICAL ABSTRACTS,* 850918-68-2
- *CHEMICAL ABSTRACTS,* 1242056-42-3
- *CHEMICAL ABSTRACTS,* 721969-94-4